(19) Europäisches Patentamt
European Patent Office
Office européen des brevets

(11) EP 2 700 400 A1

(12) **EUROPEAN PATENT APPLICATION**

(43) Date of publication:
**26.02.2014 Bulletin 2014/09**

(21) Application number: **13191878.1**

(22) Date of filing: **26.01.2010**

(51) Int Cl.:
***A61K 9/22*** *(2006.01)*
***A61K 9/36*** *(2006.01)*
***A61K 9/20*** *(2006.01)*
***A61K 9/28*** *(2006.01)*
***A61K 31/485*** *(2006.01)*

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **26.01.2009 DK 200900127**
**26.01.2009 US 147151 P**
**24.06.2009 DK 200900782**

(62) Document number(s) of the earlier application(s) in accordance with Art. 76 EPC:
**10701803.8 / 2 389 169**

(71) Applicant: **Egalet Ltd.**
**London**
**W1S 2LQ (GB)**

(72) Inventors:
- **Andersen, Christine**
  **2950 Vedbæk (DK)**
- **Jespersen, Lillian**
  **2730 Herlev (DK)**
- **Lindhardt, Karsten**
  **4690 Haslev (DK)**
- **Øvergård, Jan Martin**
  **3600 Frederikssund (DK)**
- **Lyhne-Iversen, Louise Inoka**
  **2820 Gentofte (DK)**
- **Olsen, Martin Rex**
  **3400 Holbæk (DK)**
- **Christensen, Lars Hedevang**
  **2870 Dyssegård (DK)**
- **Høilund-Jensen, Jacob Aas**
  **3600 Frederikssund (DK)**

(74) Representative: **Høiberg A/S**
**St. Kongensgade 59 A**
**1264 Copenhagen K (DK)**

Remarks:
This application was filed on 07-11-2013 as a divisional application to the application mentioned under INID code 62.

(54) **Controlled release formulation with continuous efficacy**

(57) The present invention relates to pharmaceutical compositions, which provide controlled release of a drug. The compositions are suitable for continuous administration as they remain effective throughout the treatment regimen. The present invention also relates to the use of the compositions for preparation of a medicament for continuous treatment of an individual.

EP 2 700 400 A1

Printed by Jouve, 75001 PARIS (FR)

## Description

All patent and non-patent references cited in the application are hereby incorporated by reference in their entirety.

## Field of invention

The present invention relates to the field of controlled release formulations, and in particular to formulations useful for once daily administration.

## Background of invention

Steady state concentrations are an important aspect for a controlled release formulation, which cannot be determined based on single dosage studies. Efficacy may be dependent on the steady state Cmin and a small difference in steady state Cmax and steady state Cmin may be advantageous, to provide maximal possible time in the therapeutic window, (higher than minimal effective concentration and lower than a level giving rise to side effects). In relation to analgesics, the minimal effective concentration is referred to as "minima effective analgesic concentration (MEAC). Accordingly, a given Cmin for a given active drug substance may be desirable. However, for many drug substances a preferred Cmin is not easy to achieve.

For opioids a concern is that the mu receptor (m OR) can show tolerance. This kind of tolerance development always carries a risk that repeated dose studies provide unexpected results in efficacy (i.e. tachyphylaxis). As it appears from the section from Raehhal and Bohn (Mu Opioid Receptor Regulation and Opiate Responsiveness The AAPS Journal 2005;7(3):Article 60) below m OR can be differently regulated in different cellular environment, and any regulations on receptor level give rise to a risk of unpredictable results.

*"Further, chronic morphine has been shown to induce desensitization of the m OR as measured by adenylyl cyclase inhibition in thalamus and periaqueductal gray brain regions but not in caudate putamen or nucleus accumbens. These observations suggest that while the m OR is expressed in these brain regions, it is not desensitized to the same extent following chronic morphine treatment and demonstrates that the m OR can be differentially regulated in different cellular environments."* (Raehal and Bohn, 2005).

Due to tolerance development / tachyphylaxia on a receptor level repeated dose efficacy cannot be predicted from a single dose PK profile.

The International Association for the Study of Pain gives this definition of pain: PAIN is *"an unpleasant sensory and emotional experience associated with actual or potential tissue damage, or described in terms of such damage".* Pain and degree of pain may be determined using questionnaires asking afflicted individuals to evaluate their perception of pain.

Even though Morphine has been known as an analgesic for a very long time, a MEAC, a concentration-effect relationship, or a therapeutic window have not been established for Morphine.

However, the pharmacokinetic profile of Morphine is known to be critical as described by Camu and Vanlersberghe (Pharmacology of systemic analgesics. Best Pract Res Clin Anaesthesiol 2002;16(4):475-88): *"...But in clinical practise, their* [i.e. opioids, applicant's note] *very steep concentration analgesic effect relationship remains a critical aspect of opioid therapy. Thus, small fluctuations in plasma concentrations of opioids may lead to profound fluctuations in analgesic effect when their plasma concentration and effect-site concentrations are near the minimum effective analgesic concentration"* (Camu and Vanlersberghe 2002).

Attempts have been made to estimate the MEAC for morphine. Dahlström et al (1982) (Patient-controlled analgesic therapy, part IV: Pharmacokinetics and analgesic plasma concentrations of morphine. Clin Pharmacokinet;7: 266-79) reported in a group of 10 postoperative patients a calculated mean (SD) MEAC of 16 (9) ng/mL while Graves et al (1985, Relationship between plasma morphine concentrations and pharmacologic effects in postoperative patients using patient-controlled analgesia. Clin Pharm 1985;4:41-7) estimated this limit to be in the range of 20-40 ng/mL. These limits have, however, not proved useful in clinical practice.

Commercially available controlled release formulations of Morphine includes MST Continus and Dolcontin, both which are for administration twice daily. Thus, there is a need for controlled release formulations for less frequent administration, which however are continuously effective.

WO2003/024430 and WO2004/084868 describes Morphine polymer release systems, which are suggested for once or twice daily administration. The documents describe administration of single dosages of the systems, but do not relate to continuous administration of these systems and no information regarding efficacy is given. Example 3 in WO2003/024430, which is identical to Example 3 in WO2004/084868,mentions that therapeutic effect was achieved up to 5 hours after administration of a single dosage, but no information of efficacy over a longer period is revealed.

**Summary of invention**

There is thus a need for controlled release formulations, which are suitable for continuous administration once daily, and which remain effective throughout the treatment regimen. In particular, in relation to analgesics, it is important that the treatment remains efficient for the entire period between two administrations, i.e. for 24 hours for each administration.

The present invention provides such pharmaceutical compositions. Accordingly, it is one objective of the present invention to provide pharmaceutical compositions comprising

a) a matrix composition comprising

i) an active drug substance which is an analgesic; and
ii) at least one polyglycol

said matrix composition having a cylindrical shape optionally with tapered end(s), said matrix being surrounded by
b) a coating having at least one opening exposing one surface of said matrix, said coating being substantially impermeable to an aqueous medium;

for continuous treatment of pain in an individual in need thereof, wherein the medicament is prepared for continuous administration once daily and wherein steady state C24 in respect of the active drug substance is at least 20%, preferably at least 25%, more preferably at least 30%, for example in the range of 30 to 90%, such as in the range of 30 to 80%, for example in the range of 30 to 70%, such as in the range of 30 to 60% of steady state Cmax in respect of the active drug substance. By the term "substantially impermeable" as used herein is meant that the coating is impermeable to an aqueous medium for at least 24 hours, more preferred for at least 48 hours.

Thus, the present invention relates to pharmaceutical compositions comprising

a) a matrix composition comprising

i) an active drug substance which may be any of the active drug substances described herein below in the section "Active drug substance"; and
ii) at least one polyglycol, which may be any of the polyglycols described herein below in the section "Polyglycol"

said matrix composition having a cylindrical shape optionally with tapered end(s), wherein said shape may be any of the shapes described herein below in the section "Geometry", said matrix being surrounded by
b) a coating having at least one opening exposing one surface of said matrix, said coating being substantially insoluble in an aqueous medium and impermeable to water, wherein the coating may be any of the coatings described herein below in the section "Coating".

The pharmaceutical compositions may be prepared for continuous treatment of pain in an individual in need thereof. Said continuous treatment of pain is preferably a once daily administration and may for example be any of the administrations described herein below in the section "Administration" and said individual in need thereof may be any of the individuals described herein below in the section "Individual in need of treatment".

In addition, Cmax, Cmin, Tmax, $1^{st}$ and $2^{nd}$ time to 50% Cmax, and Protraction index are preferably as described herein below in the section "Steady state".

In addition the present invention relates to use of above mentioned pharmaceutical composition for preparation of a medicament for treatment of pain in an individual in need thereof. Said continuous treatment of pain is preferably a once daily administration and may for example be any of the administrations described herein below in the section "Administration" and said individual in need thereof may be any of the individuals described herein below in the section "Individual in need of treatment".

Furthermore the invention relates to methods for continuously treating pain in an individual in need thereof, said method comprising continuously administering to said individual once daily, a pharmaceutical composition comprising

a) a matrix composition comprising

i) an active drug substance which may be any of the active drug substances described herein below in the section "Active drug substance"; and
ii) at least one polyglycol, which may be any of the polyglycols described herein below in the section "Polyglycol"

said matrix composition having a cylindrical shape optionally with tapered end(s), wherein said shape may be any of the shapes described herein below in the section "Geometry", said matrix being surrounded by
b) a coating having at least one opening exposing one surface of said matrix, said coating being substantially insoluble in an aqueous medium and impermeable to water, wherein the coating may be any of the coatings described herein below in the section "Coating";

wherein steady state C24 is at least 20%, preferably at least 25%, more preferably at least 30% of steady state Cmax in respect of the active drug substance. In addition, Cmax, Cmin, Tmax, 1$^{st}$ and 2$^{nd}$ time to 50% Cmax, and Protraction index are preferably as described herein below in the section "Steady state".

**Description of Drawings**

Figure 1 shows geometric mean concentration (nmol/L) versus time curve (0-24h), which have been dose-normalised (TDD 100 mg/day) in steady state individuals having received Egalet® Morphine Formulation A (n=10) or MST Continus (n=11). The individuals had received either Egalet® Morphine Formulation A (once daily) or MST Continus (twice daily) for 14 days prior to time point 0.

Figure 2 shows mean steady state morphine plasma concentration (nmol/L) versus time curve (0-24h). The data were obtained as described in Example 2.

Figure 3 shows in vitro dissolution results (drug release (%) versus time (minutes)) of pharmaceutical compositions A (30 mg morphine), B1 (30, 60, 100, and 200 mg morphine) and B2 (100 mg morphine) according to the present invention.

Figure 4 shows the mean morphine plasma concentration (nmol/L) versus time curve by dose group (0 48h). The data were obtained as described in Example 3.

**Detailed description of the invention**

**Definitions**

The term "cylindrical shape" as used herein refers to any geometrical shape having the same cross section area throughout the length of the geometrical shape. The cross section of a cylinder within the meaning of the present invention may have any two dimensional shape, for example the cross section may be circular, oval, rectangular, triangular, angular or star shaped. The pharmaceutical compositions according to the invention preferably have a cylindrical shape, wherein the end(s) may be tapered.

The term "steady state" refers to the state when the plasma concentration level following one dosing is the same within the standard deviation as the plasma concentration level following the following dosing. Thus, for pharmaceutical compositions for once daily administration then at steady state $AUC_{(0-24h)d}=AUC_{(0-24h)d+1}$ +/- the standard deviation, and $Cmax_{(0-24h)d}=Cmax_{(0-24h)d+1}$ +/- the standard deviation where d is day.

The term "steady state Cmin" is defined by the average lowest plasma concentration at steady state observed over the dosing interval. Thus, for pharmaceutical compositions prepared for once daily administration Cmin is defined by the average lowest plasma concentration at steady state observed over a 24 hour dosing-interval. Preferably, said average lowest plasma concentration is the average of the lowest plasma concentration observed in at least 10, preferably at least 15, more preferably at least 18 steady state individuals.

The term "Trough" is defined as the average plasma concentration in a steady state individual just prior to the following dose. Thus, for pharmaceutical compositions prepared for once daily administration then trough is the average plasma concentration in a steady state individual 24 hours after dosing and just prior to the following dose. Preferably, said average plasma concentration is the average of the plasma concentration observed in at least 10, preferably at least 15, more preferably at least 18 steady state individuals. For pharmaceutical compositions having a very flat profile analytical variation may provide a different result for Cmin and trough, but for most practical matters they are the same.

The term "steady state C24" is defined as the average plasma concentration of an active drug substance in a steady state individual observed 24 hours after last administration of said active drug substance. Preferably, said average plasma concentration is the average of the plasma concentration of said active drug substance after 24 hours after last administration observed in at least 10, preferably at least 15, more preferably at least 18 steady state individuals. For pharmaceutical compositions prepared for once daily administration C24 and trough will be the same.

The term "steady state Cmax" is the average highest plasma concentration at steady state observed over the dosing interval. Thus, for pharmaceutical compositions prepared for once daily administration max is defined by the average highest plasma concentration at steady state observed over a 24 hour dosing-interval. Preferably, said average highest plasma concentration is the average of the highest plasma concentration observed in at least 10, preferably at least 15, more preferably at least 18 steady state individuals.

The term "steady state individual" refers to an individual to whom the pharmaceutical compositions according to the present invention have been administered for a sufficient number of times in order to have arrived at steady state. Thus, for pharmaceutical compositions prepared for administration once daily, then a steady state individual is an individual to whom the pharmaceutical compositions according to the present invention has been administered once daily for a sufficient number of days in order to have arrived at steady state. Steady state is reached when the plasma concentration level after one dosing is the same within the standard deviation as the plasma concentration level after the following dosing, meaning for once daily dosing that $AUC_{(024h)d}$=$AUC_{(0-24h)d+1}$, and $Cmax_{(0-24h)d}$=$Cmax_{(0-24h)d+1}$ where d is day. Preferably, a steady state individual, is an individual to whom who the pharmaceutical compositions according to the present invention has been administered once daily for at least 3 days, preferably for at least 4 days, for example for at least 7 days.

The term "steady state Tmax" refers to the average time lapsing between administration and arrival at Cmax in a steady state individual. Preferably, said average time is the average of the time observed in at least 10, preferably at least 18 steady state individuals.

Steady state $AUC_{0-24h}$ is defined by the average area under the curve of a steady state plasma concentration profile of an active drug substance from 0-24h after administration of said active drug substance. This is obtained from sum of steady state AUCs (I.e. $\Sigma(AUC_{0-1h}, AUC_{1-2h}...... AUC_{t-24})$) between measurements from each sample point. The AUCs are calculated by the linear trapezoidal method. If the last blood sample is taken less than 24h after drug administration, the 24h value will be extrapolated using the terminal elimination rate constant as described below. Single missing values will remain missing, i.e. corresponding to interpolation between the neighbouring points when calculating AUC. $AUC_{0-24h}$ is preferably calculated as an average of $AUC_{0-24h}$ observed in at least 10, preferably at least 15, more preferably at least 18 steady state individuals.

The term "Protraction index" as used herein illustrates the flatness of the steady state plasma concentration profile and is defined as the average concentration in the 24 hour dosing interval divided by the maximum concentration, i.e. $((AUC_{0-24h} / 24\ h) / C_{max})$. In the theoretical case where the profile is completely flat the average concentration will be identical to the maximum concentration and the Protraction index will be equal to 1. Hence, due to the fact that the average concentration cannot take a value higher than the maximum concentration, the Protraction index can never be higher than 1. In cases where the profile is substantially flat, the difference between the maximum concentration and the average concentration is small and the Protraction index will take a value close to 1. In other cases where the maximum concentration for instance is 5 times higher than the average concentration the Protraction index wil take the value 0.2.

**Polyglycol**

The pharmaceutical compositions according to the invention comprise a matrix composition comprising at least one polyglycol.

The matrix composition may comprise more than one different kind of polyglycol, such as 2, for example 3, such as 4, for example 5, such as more than 5 different polyglycols. Preferably, the matrix composition comprises in the range of 1 to 4, even more preferably in the range of 1 to 3, yet more preferably 2 different polyglycols.

The polyglycol may e.g. be in the form of a homopolymer and/or a copolymer. If the matrix composition comprises more than one polyglycol they may all be different homopolymer, or different copolymers or a mixture of homopolymers and copolymers. In one preferred embodiment of the invention, the matrix composition comprises at least one polyglycol, which is a homopolymer and at least one polyglycol, which is a copolymer. In another preferred embodiment of the invention, the matrix composition comprises at least one polyglycol, which is a homopolymer.

In a preferred embodiment the polyglycols are substantially water soluble, thermoplastic, crystalline, semi-crystalline or amorphous or a mixture of substantially water soluble, crystalline, semi-crystalline or amorphous polymers. In particular it is preferred that the polyglycol is at least thermoplastic. Suitable polyglycols for use in a matrix composition according to the invention are polyethylene glycols, as well as derivatives of polyethylene glycol such as mono or dimethoxypolyethylene glycols (mPEGs), polyethylene oxides and/or block copolymers of ethylene oxide and propylene oxide.

Polyethylene glycols (PEGs) are linear polydisperse polymers composed of repeating units of ethylene glycol. Their chemical formula is $HOCH_2[CH_2OCH_2]_mCH_2OH$ where m represents the average number of repeating units. Alternatively, the general formula $H[OCH_2CH_2]_nOH$ may be used to represent polyethylene glycol, where n is as number m in the previous formula + 1. See the structural presentations of polyethylene glycol below, n is the average number

of oxyethylene groups, n equals m + 1.

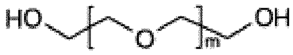

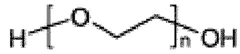

In a preferred embodiment, the matrix composition comprises at least one polyglycol which is a polyethylene oxide.

Polyethylene oxides (PEOs) are linear polydisperse nonionic polymers composed of repeating units of ethylene oxide. Their chemical formula is $HO[CH_2CH_2O]_nH$ where n represents the average number of oxyethylene groups. See the structural presentation of polyethylene oxide below, n is the average number of oxyethylene groups. Depending on preparation method high molecular weigh PEO may have one terminal methyl group.

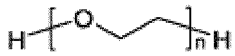

In general PEG refers to polymers chains with molecular weights below 20,000, while PEO refers to higher molecular weights polymers. However, because of the similarities between PEO and PEG, the terms are often used interchangeably for the same compound.

Polyethylene glycols and/or polyethylene oxides, which are suitable for use in the matrix composition are those having an average molecular weight of at least 20,000 daltons, such as an average molecular weight of in the range of 20,000 to 700,000 daltons, for example in the range of 20,000 to 600,000 daltons, such as in the range of 35,000 to 500,000 daltons, for example in the range of 35,000 to 400,000 daltons, such as in the range of 35,000 to 350,000 daltons, for example in the range of 50,000 to 350,000 daltons, such as in the range of 100.000 to 300.000 daltons, for example in the range of 150.000 to 350.000, such as in the range of 200.000 to 300.000, such as approximately 35,000 daltons, for example approximately 50,000 daltons, such as approximately 75,000 daltons, for example approximately 100,000 daltons, such as approximately 150,000 daltons, for example approximately 200,000 daltons, such as approximately 250,000 daltons, for example approximately 300,000 daltons, such as approximately 400,000 daltons, such as 150,000 daltons, for example 200,000 daltons, such as 250,000 daltons, for example 300,000 daltons, such as 400,000 daltons. In the present context "approximately" preferably means +/- 30%.

In a specific embodiment at least one polyglycol is a polyethylene oxide or a polyethylene glycol that has a molecular weight of approximately 20,000 daltons, approximately 35,000 daltons, approximately 50,000 daltons, approximately 100,000 daltons, approximately 200,000 daltons, approximately 300,000 daltons and approximately 400,000 daltons. In the present context "approximately" preferably means +/- 30%. PEG is commercially available with average molecular weights up to 35,000. PEO is commercially available with average molecular weights up to 8,000,000. In specific embodiment, the polymer is a PEO having an average molecular weight of at least 100,000, such as in the range of 100,000 to 8,000,000, for example in the range of 100,000 to 7,000,000, such as in the range of 100,000 to 5,000,000, for example in the range of 100,000 to 4,000,000, such as in the range of 100,000 to 2,000,000, for example in the range of 100,000 to 1,000,000, such as in the range of 100,000 to 900,000. When PEO is employed with a molecular weight in the lower end, the PEO typically has a molecular weight as mentioned in the preceding paragraph. Commercially available PEOs with a molecular weight in the higher end have typically the following molecular weights: approximately 900,000, approximately 1 ,000,000, approximately 2,000,000, approximately 4,000,000, approximately 5,000,000, approximately 7,000,000, approximately 8,000,000.

The matrix composition according to the invention may also comprise at least one polyglycol which is a copolymer.

In preferred embodiments of the invention the matrix composition comprise at least one polyglycol which is a poloxamer. Poloxamers are copolymers or block copolymers and are a range of non-ionic surfactants of polyethylene glycol (PEG) and polypropylene glycol (PPG).

The poloxamer may be Diol EO/PO block copolymers, which for example in chemical abstracts are described under the scientific name -hydroxy-hydroxypoly(oxyethylene)poly(oxypropylene)-poly(oxyethylene)-block copolymer in combination with the CAS register number. In specific embodiments a suitable poloxamer for use in a composition of the invention has a HLB value of at least about 18 such as, e.g., at least approximately 20, preferably at least 24. The average molecular weight of a suitable poloxamer is typically at least about 2,000.

Typical block copolymers of ethylene oxide and propylene oxide to be comprised in the matrix composition according to the invention have a molecular weight of at least 2,000 daltons, typically in the range of 3,000 to 30,000 daltons, such as in the range of 4,000 to 15,000 daltons.

Preferred poloxamers have the formula $HO(C_2H_4O)_a(C_3H_6O)_b(C_2H_4O)_aH$, and preferably a is an integer from 10 to 150, such as from 30 to 140, for example from 50 to 100, such as from 65 to 90, for example from 70 to 90 and preferably b is an integer from 10 to 80, such as from 15 to 80, for example from 20 to 60, such as from 25 to 55.

The matrix composition may comprise mixtures of PEO with different average molecular weights for example in order to obtain a PEO with a desirable average molecular weight. The same applies to PEG.

It should be noted that in this context Vitamin E polyethylene glycol succinate (TPGS) is not considered a polyglycol.

The polyglycol should preferably have a melting point higher than the body temperature of the human in which the composition is to be used. Thus, the polyglycol(s) employed in the matrix composition will suitably have a melting point of in the range of 38-120°C such as in the range of 38 to 100°C, for example in the range of 40 to 80°C.

In a very preferred embodiment of the invention the matrix composition comprises at least one polyethylene oxide and at least one copolymer.

In addition to a polymer of a polyglycol type, the matrix composition may comprise an additional polymer, for example at least one polymer selected from the group consisting of: modified or unmodified water soluble natural polymers such as glucomannan, galactan, glucan, polygalacturonic acid, polyxylane, polygalactomannans, rhanogalacturonan, polyxyloglycan, arabinogalactan, and starch, cellulose, chitosan, alginate, fibrin, collagen, gelatin, hyaluronic acid, amylopectin, pectin including low methylated or methoxylated pectins, dextran and fatty acids and alcohols; synthetic polymers such as polyvinylpyrrolidone (PVP), PVA, PVB, Eudragit L methyl ester, Eudragit L, Eudragit RL, Eudragit RS, Eudragit E, Eudragit S, PHPV, PHA, PCL, PLGA and PLA; and hydrogels made from the polymers or combined polymers mentioned above and or from polymers originated from: HEMA, HEEMA, MEMA, MEEMA, EDGMA, NVP, VAc, AA, acrylamide, MAA, HPMA, PEGA, PEGMA, PEGDMA, PEGDA, and PEGDMA.

One or more polymers are typically present in a matrix composition of the invention in a concentration amount of from 5 % to 99.9% w/w, such as from 5 to 95% w/w, such as from 5 % to 80% w/w, such as from 10 % to 80% w/w, such as from 20 % to 80% w/w, for example from 30 % to 80% w/w, such as from 40 % to 80% w/w, for example from 45 % to 75% w/w calculated as w/w % of the composition.

The total concentration of the polyglycols (notably the sum of homo- and copolymers of the polyglycol type) in the matrix composition is preferably from 5 % to 99% w/w such as from 15 % to 95% w/w, for example from 30 % to 90% w/w, such as from 30 % to 85% w/w, for example from 30 % to 80% w/w, such as from 40 % to 80% w/w, for example from 45 % to 75% w/w, such as from 40 % to 50% w/w, for example from 45 % to 50% w/w, such as from 60 % to 85% w/w, for example from 70 % to 80% w/w, for example from 70 % to 75% w/w, such as from 71 % to 75% w/w.

The concentration of the polyglycol homopolymer in the matrix composition is preferably from 5 % to 80% w/w and in those cases where the homopolymer is the only thermoplastic polymer present in the matrix composition, then the concentration is preferably from 20 % to 80% w/w, such as from 40 % to about 80% w/w, such as for example from 70 % to 80% w/w, such as from 70 % to 75% w/w, for example from about 71 % to about 75% w/w.

In preferred embodiments of the invention, then the concentration of the homopolymers in the matrix composition is in the range of 5 % to 90% w/w, such as in the range of 20 % to 85% w/w, for example in the range of 20 % to 75% w/w, such as in the range of 20 % to 70% w/w, for example in the range of 20 % to 40% w/w, such as in the range of 30% to 85% w/w, for example in the range of about 30 % to 75% w/w, such as in the range of 30 % to 50% w/w, for example in the range of 30 % to 40% w/w, such as in the range of 30 % to 35% w/w, such as in the range of 31 % to about 33% w/w, such as in the range of 50 % to 85% w/w, from 60 % to 80% w/w, for example in the range of 70 % to 80% w/w, for example in the range of 70 % to 75% w/w, such as in the range of 71 % to about 73% w/w.

The concentration of the polyglycol copolymer in the matrix composition, if present in combination with a polyglycol homopolymer, is preferably in the range of 0 % to 60% w/w, such as for example 0 % to 30% w/w. If the copolymer is the sole thermoplastic polymer in the matrix composition the concentration may be from about 5 % to about 99.5% w/w such as those ranges described above and described for the homopolymer.

In preferred embodiments, the concentration of polyglycols which are co-polymers in the matrix composition is in the range of 0 % to 30% w/w, such as in the range of 1 % to 20 % w/w, for example in the range of 2 % to 10% w/w, such as in the range of 2 % to 5%, w/w, such as in the range of 5 % to 30% w/w, for example in the range of 10 % to 30% w/w, such as in the range of 10 % to 20% w/w, for example in the range of 10 % to 15% w/w, such as less than 10% w/w, for example less than 5% w/w, such as less than 1% w/w, for example 0% w/w.

## Active drug substance

An active drug substance in a composition for use according to the invention is a therapeutically, prophylactically and/or diagnostically active drug substance (herein also abbreviated "active drug substance").

Examples of specific active drug substances suitable for use in a composition of the invention are:

Antiinflammatory and antirheumatic active substances; Butylpyrazolidines, Phenylbutazone, Mofebutazone, Oxyphenbutazone, Clofezone, Kebuzone, Acetic acid derivatives and related substances, Indometacin, Sulindac, Tolmetin, Zomepirac, Diclofenac, Alclofenac, Bumadizone, Etodolac, Lonazolac, Fentiazac, Acemetacin, Difenpiramide, Oxametacin, Proglumetacin, Ketorolac, Aceclofenac, Bufexamac, Oxicams, Piroxicam, Tenoxicam, Droxicam,

Lornoxicam, Meloxicam, Propionic acid derivatives, Ibuprofen, Naproxen, Ketoprofen, Fenoprofen, Fenbufen, Benoxaprofen, Suprofen, Pirprofen, Flurbiprofen, Indoprofen, Tiaprofenic acid, Oxaprozin, Ibuproxam, Dexibuprofen, Flunoxaprofen, Alminoprofen, Dexketoprofen, Fenamates, Mefenamic acid, Tolfenamic acid, Flufenamic acid, Meclofenamic acid, Coxibs, Celecoxib, Rofecoxib, Valdecoxib, Parecoxib, Etoricoxib, Lumiracoxib, Nabumetone, Niflumic acid, Azapropazone, Glucosamine, Benzydamine, Glucosaminoglycan polysulfate, Proquazone, Orgotein, Nimesulide, Feprazone, Diacerein, Morniflumate, Tenidap, Oxaceprol, Chondroitin sulfate, Feprazone, Dipyrocetyl, Acetylsalicylic acid, Quinolines, Oxycinchophen, Gold preparations, Sodium aurothiomalate, Sodium aurotiosulfate, Auranofin, Aurothioglucose, Aurotioprol, Penicillamine and similar agents, Bucillamine.

Analgesics; Opioids, Natural opium alkaloids, Morphine, Opium, Hydromorphone, Nicomorphine, Oxycodone, Dihydrocodeine, Diamorphine, Papaveretum, Codeine, Phenylpiperidine derivatives, Ketobemidone, Pethidine, Fentanyl, Diphenylpropylamine derivatives, Dextromoramide, Piritramide, Dextropropoxyphene, Bezitramide, Methadone, Benzomorphan derivatives, Pentazocine, Phenazocine, Oripavine derivatives, Buprenorphine, Morphinan derivatives, Butorphanol, Nalbuphine, Tilidine, Tramadol, Dezocine, Salicylic acid and derivatives, Acetylsalicylic acid, Aloxiprin, Choline salicylate, Sodium salicylate, Salicylamide, Salsalate, Ethenzamide, Morpholine salicylate, Dipyrocetyl, Benorilate, Diflunisal, Potassium salicylate, Guacetisal, Carbasalate calcium, Imidazole salicylate, Pyrazolones, Phenazone, Metamizole sodium, Aminophenazone, Propyphenazone, Nifenazone, Anilides, Paracetamol, Phenacetin, Bucetin, Propacetamol, Other analgesics and antipyretics, Rimazolium, Glafenine, Floctafenine, Viminol, Nefopam, Flupirtine, Ziconotide.

Anesthetics; Ethers, Diethyl ether, Vinyl ether, Halogenated hydrocarbons, Halothane, Chloroform, Methoxyflurane, Enflurane, Trichloroethylene, Isoflurane, Desflurane, Sevoflurane, Barbiturates, Methohexital, Hexobarbital, Thiopental, Narcobarbital, Opioid anesthetics, Fentanyl, Alfentanil, Sufentanil, Phenoperidine, Anileridine, Remifentanil, Other general anesthetics, Droperidol, Ketamine, Propanidid, Alfaxalone, Etomidate, Propofol, Hydroxybutyric acid, Nitrous oxide, Esketamine, Xenon, Esters of aminobenzoic acid, Metabutethamine, Procaine, Tetracaine, Chloroprocaine, Benzocaine, Amides, Bupivacaine, Lidocaine, Mepivacaine, Prilocaine, Butanilicaine, Cinchocaine, Etidocaine, Articaine, Ropivacaine, Levobupivacaine, Esters of benzoic acid, Cocaine, Other local anesthetics, Ethyl chloride, Dyclonine, Phenol, Capsaicin.

Antimigraine active substances; Ergot alkaloids, Dihydroergotamine, Ergotamine, Methysergide, Lisuride, Corticosteroid derivatives, Flumedroxone, Selective serotonin (5HT1) agonists, Sumatriptan, Naratriptan, Zolmitriptan, Rizatriptan, Almotriptan, Eletriptan, Frovatriptan, Other antimigraine preparations, Pizotifen, Clonidine, Iprazochrome, Dimetotiazine, Oxetorone.

Antiepileptic active substances; Barbiturates and derivatives, Methylphenobarbital, Phenobarbital, Primidone, Barbexaclone, Metharbital, Hydantoin derivatives, Ethotoin, Phenytoin, Amino(diphenylhydantoin) valeric acid, Mephenytoin, Fosphenytoin, Oxazolidine derivatives, Paramethadione, Trimethadione, Ethadione, Succinimide derivatives, Ethosuximide, Phensuximide, Mesuximide, Benzodiazepine derivatives, Clonazepam, Carboxamide derivatives, Carbamazepine, Oxcarbazepine, Rufinamide, Fatty acid derivatives, Valproic acid, Valpromide, Aminobutyric acid, Vigabatrin, Progabide, Tiagabine, Other antiepileptics, Sultiame, Phenacemide, Lamotrigine, Felbamate, Topiramate, Gabapentin, Pheneturide, Levetiracetam, Zonisamide, Pregabalin, Stiripentol, Lacosamide, Beclamide.

Anticholinergic active substances; Tertiary amines, Trihexyphenidyl, Biperiden, Metixene, Procyclidine, Profenamine, Dexetimide, Phenglutarimide, Mazaticol, Bornaprine, Tropatepine, Ethers chemically close to antihistamines, Etanautine, Orphenadrine (chloride), Ethers of tropine or tropine derivatives, Benzatropine, Etybenzatropine.

Dopaminergic active substances; Dopa and dopa derivatives, Levodopa, Melevodopa, Etilevodopa, Adamantane derivatives, Amantadine, Dopamine agonists, Bromocriptine, Pergolide, Dihydroergocryptine mesylate, Ropinirole, Pramipexole, Cabergoline, Apomorphine, Piribedil, Rotigotine, Monoamine, oxidase B inhibitors, Selegiline, Rasagiline, Other dopaminergic agents, Tolcapone, Entacapone, Budipine.

Antipsychotic active substances; Phenothiazines with aliphatic side-chain, Chlorpromazine, Levomepromazine, Promazine, Acepromazine, Triflupromazine, Cyamemazine, Chlorproethazine, Phenothiazines with piperazine structure, Dixyrazine, Fluphenazine, Perphenazine, Prochlorperazine, Thiopropazate, Trifluoperazine, Acetophenazine, Thioproperazine, Butaperazine, Perazine, Phenothiazines with piperidine structure, Periciazine, Thioridazine, Mesoridazine, Pipotiazine, Butyrophenone derivatives, Haloperidol, Trifluperidol, Melperone, Moperone, Pipamperone, Bromperidol, Benperidol, Droperidol, Fluanisone, Indole derivatives, Oxypertine, Molindone, Sertindole, Ziprasidone, Thioxanthene derivatives, Flupentixol, Clopenthixol, Chlorprothixene, Tiotixene, Zuclopenthixol, Diphenylbutylpiperidine derivatives, Fluspirilene, Pimozide, Penfluridol, Diazepines, oxazepines and thiazepines, Loxapine, Clozapine, Olanzapine, Quetiapine, Neuroleptics, in tardive dyskinesia, Tetrabenazine, Benzamides, Sulpiride, Sultopride, Tiapride, Remoxipride, Amisulpride, Veralipride, Levosulpiride, Lithium, Other antipsychotics, Prothipendyl, Risperidone, Clotiapine, Mosapramine, Zotepine, Aripiprazole, Paliperidone.

Anxiolytic active substances; Benzodiazepine derivatives, Diazepam, Chlordiazepoxide, Medazepam, Oxazepam, Potassium clorazepate, Lorazepam, Adinazolam, Bromazepam, Clobazam, Ketazolam, Prazepam, Alprazolam, Halazepam, Pinazepam, Camazepam, Nordazepam, Fludiazepam, Ethyl loflazepate, Etizolam, Clotiazepam, Cloxazolam, Tofisopam, Diphenylmethane derivatives, Hydroxyzine, Captodiame, Carbamates, Meprobamate, Emylcamate, Mebutamate, Dibenzo-bicyclo-octadiene derivatives, Benzoctamine, Azaspirodecanedione derivatives, Buspirone, Other anxiolytics, Mephenoxalone, Gedocarnil, Etifoxine.

Hypnotic and sedative active substances; Barbiturates, Pentobarbital, Amobarbital, Butobarbital, Barbital, Aprobarbital, Secobarbital, Talbutal, Vinylbital, Vinbarbital, Cyclobarbital, Heptabarbital, Reposal, Methohexital, Hexobarbital, Thiopental, Etallobarbital, Allobarbital, Proxibarbal, Aldehydes and derivatives, Chloral hydrate, Chloralodol, Acetylglycinamide chloral hydrate, Dichloralphenazone, Paraldehyde, Benzodiazepineemepronium derivatives, Flurazepam, Nitrazepam, Flunitrazepam, Estazolam, Triazolam, Lormetazepam, Temazepam, Midazolam, Brotizolam, Quazepam, Loprazolam, Doxefazepam, Cinolazepam, Piperidinedione derivatives, Glutethimide, Methyprylon, Pyrithyldione, Benzodiazepine related drugs, Zopiclone, Zolpidem, Zaleplon, Ramelteon, Other hypnotics and sedatives, Methaqualone, Clomethiazole, Bromisoval, Carbromal, Scopolamine, Propiomazine, Triclofos, Ethchlorvynol, Valerian, Hexapropymate, Bromides, Apronal, Valnoctamide, Methylpentynol, Niaprazine, Melatonin, Dexmedetomidine, Dipiperonylaminoethanol.

Antidepressant active substances; Non-selective monoamine reuptake inhibitors, Desipramine, Imipramine, Imipramine oxide, Clomipramine, Opipramol, Trimipramine, Lofepramine, Dibenzepin, Amitriptyline, Nortriptyline, Protriptyline, Doxepin, Iprindole, Melitracen, Butriptyline, Dosulepin, Amoxapine, Dimetacrine, Amineptine, Maprotiline, Quinupramine, Selective serotonin reuptake inhibitors, Zimeldine, Fluoxetine, Citalopram, Paroxetine, Sertraline, Alaproclate, Fluvoxamine, Etoperidone, Escitalopram, Monoamine oxidase inhibitors, non-selective, Isocarboxazid, Nialamide, Phenelzine, Tranylcypromine, Iproniazide, Iproclozide, Monoamine oxidase A inhibitors, Moclobemide, Toloxatone, Other antidepressants, Oxitriptan, Tryptophan, Mianserin, Nomifensine, Trazodone, Nefazodone, Minaprine, Bifemelane, Viloxazine, Oxaflozane, Mirtazapine, Medifoxamine, Tianeptine, Pivagabine, Venlafaxine, Milnacipran, Reboxetine, Gepirone, Duloxetine, Agomelatine, Desvenlafaxine, Centrally acting sympathomimetics, Amfetamine, Dexamfetamine, Lisdexamfetamine, Metamfetamine, Methylphenidate, Dexmethylphenidate, Pemoline, Fencamfamin, Modafinil, Fenozolone, Atomoxetine, Fenetylline, Xanthine derivatives, Caffeine, Propentofylline, Other psychostimulants and nootropics, Meclofenoxate, Pyritinol, Piracetam, Deanol, Fipexide, Citicoline, Oxiracetam, Pirisudanol, Linopirdine, Nizofenone, Aniracetam, Acetylcarnitine, Idebenone, Prolintane, Pipradrol, Pramiracetam, Adrafinil, Vinpocetine.

Anti-dementia active substances; Anticholinesterases, Tacrine, Donepezil, Rivastigmine, Galantamine, Other antidementia drugs, Memantine, Ginkgo biloba.

Other nervous system active substances; Parasympathomimetics, Anticholinesterases, Neostigmine, Pyridostigmine, Distigmine, Ambenonium, Choline esters, Carbachol, Bethanechol, Other parasympathomimetics, Pilocarpine, Choline alfoscerate.

Active substances used in addictive disorders; Drugs used in nicotine dependence, Nicotine, Bupropion, Varenicline, Drugs used in alcohol dependence, Disulfiram, Calcium carbimide, Acamprosate, Naltrexone, Drugs used in opioid dependence, Buprenorphine, Methadone, Levacetylmethadol, Lofexidine. Antivertigo active substances; Betahistine, Cinnarizine, Flunarizine, Acetylleucine, other nervous system drugs, Gangliosides and ganglioside derivatives, Tirilazad, Riluzole, Xaliproden, Hydroxybutyric acid, Amifampridine.

Opium alkaloids and derivatives, Ethylmorphine, Hydrocodone, Codeine, Opium alkaloids with morphine, Normethadone, Noscapine, Pholcodine, Dextromethorphan, Thebacon, Dimemorfan, Acetyldihydrocodeine, Benzonatate, Benproperine, Clobutinol, Isoaminile, Pentoxyverine, Oxolamine, Oxeladin, Clofedanol, Pipazetate, Bibenzonium bromide, Butamirate, Fedrilate, Zipeprol, Dibunate, Droxypropine, Prenoxdiazine, Dropropizine, Cloperastine, Meprotixol, Piperidine, Tipepidine, Morclofone, Nepinalone, Levodropropizine, Dimethoxanate.

The active drug substance may for example be an active drug substance with abuse potential or safety risk suitable. Such active drug substance may for example be selected from the group consisting of:

1-(1-Phenylcyclohexyl)pyrrolidine, 1-(2-Phenylethyl)-4-phenyl-4-acetoxypiperidine, 1-[1-(2-Thienyl)- cyclohexyljpiperidine, 1-[1-(2-Thienyl)cyclohexyl]pyrrolidine, 1-Methyl-4-phenyl-4-propionoxy-piperidine, 1-Phenylcyclohexylamine, 1-Piperidinocyclohexanecarbonitrile, 2,5-Dimethoxy-4-ethylamphetamine, 2,5-Dimethoxyamphetamine, 2C-B-(4-bromo-2,5-dimethoxypenethylamine), 2C-D (2,5-dimethoxy-4-methylphenethylamine), 2C-I (4-iodo-2,5-dimethoxy-phenethylamine), 2C-T-2 (2,5-dimethoxy-4-ethylthiophenethylamine), 2C-T-4 (2,5-dimethoxy-4-isopropyl thiophenethylamine), 2C-T-7 (2,5-dimethoxy-4-(n)-propylthiopenethylamine), 3,4-Methylene-dioxymethamphetamine, 3,4,5-Trimethoxyamphetamine, 3,4-Methylenedioxyamphetamine, 3,4-Methylenedioxy-N-ethylamphetamine, 3-Methylfentanyl, 3-Methylthiofentanyl, 4-Brorno-2,5-dimethoxyamphetamine, 4-Bromo-2,5-dimethoxyphenethylamine, 4-Methoxyamphetamine, 4-Methyl-2,5-dimethoxyamphetamine, 4-Methylaminorex (cis isomer), 5-MeO-DIPT (5-Methoxy-N,N-diisopropyltryptamine), 5-MeO-DMT (5-Methoxy-N,N-dimethyltryptamine), 5-Methoxy-3,4-methylenedioxyamphetamine, Acetorphin, Acetorphine, Acetyl-alpha-methylfentanyl, Acetyl-alpha-methylfentanyl, Acetyldihydrocodeine, Acetylmethadol, Acetylmethadol, Alfentanil, Allobarbital, Allylprodin, Allylprodine, Alphacetylmethadol except levo-alphacetylmethadol, Alpha-ethyltryptamine, Alphameprodine, Alphamethadol, Alphamethadol, Alpha-Methylfentanyl, Alpha-Methylthiofentanyl, Alphaprodine, Alprazolam, Amfepramon, Amfetaminil, Amineptin, Aminorex, Amobarbital, Amphetamine, Amylnitrit (all isomers of the amyl group), Anabolic steroids, Anileridine, Aprobarbital, Barbital, Barbituric acid derivative, BDB (3,4-methylenedioxyphenyl)-2-butanamine), Benzethidin, Benzethidine, Benzoylecgonine, Benzphetamine, Benzphetamine, Benzylmethylketon, Benzylmorphine, Betacetylmethadol, Beta-Hydroxy-3-methylfentanyl, Beta-Hydroxyfentanyl, Betameprodine, Betameprodine, Betamethadol, Betaprodine, Bezitramide, Bezitramide, Boldenone, Brolamfetamin, Bromazepam, Brotizolam, Bufotenine, Buprenorphine, Butabarbital, Butalbital, Butobarbital, Butorphanol, BZP (A 2)(1-benzylpiperazin), Camazepam, Cannabis, Carfentanil, Catha edulis, Cathine, Cathinone, Chloral betaine, Chloral hydrate, Chlordiazepoxide, Chlorhexadol, Chlorotestosterone (same as clostebol), Chlorphentermine, Clobazam, Clonazepam, Clonitazene, Clonitazene, Clorazepate, Clortermine, Clostebol, Clotiazepam, Cloxazolam, Coca Leaves, Cocaine, Codeine, Codeine & isoquinoline alkaloid, Codeine methylbromide, Codeine-N-oxide, Codoxim, Cyclobarbital (Hexemal NFN), Cyprenorphine, Dehydrochlormethyltestosterone, Delorazepam, Desomorphine, Dexamfetamine, Dexfenfluramine, Dexmethylphenidate, Dextromoramide, Dextropropoxyphene, Diacetylmorphine, Diampromide, Diazepam, Dichloralphenazone, Diethylpropion, Diethylthiambutene, Diethyltryptamine, Difenoxin, Dihydrocodeine, Dihydroetorphine, Dihydromorphine, Dihydrotestosterone, Dimenoxadol, Dimepheptanol, Dimethylthiambutene, Dimethyltryptamine, Dioxaphetyl butyrate, Diphenoxylate, Dipipanone, Diprenorphine, Dronabinol, Drostanolone, Drotebanol, Ecgonine, Estazolam, Ethchlorvynol, Ethinamate, Ethyl loflazepate, Ethylestrenol, Ethylmethylthiambutene, Ethylmorphine, Ethylmorphine, Eticyclidin, Etilamfetamine, Etonitazene, Etorphine, Etoxeridine, Etryptamine, Fencamfamin, Fenethylline, Fenetylline, Fenfluramine, Fenproporex, Fentanyl, Fludiazepam, Flunitrazepam, Fluoxymesterone, Flurazepam, Formebolone, Fungi and Spores of the sepcies Psilocype Semilanceata, Furethidine, Gammahydroxybutanic acid, Glutethimide, Halazepam, Haloxazolam, Heroine, Hydrocodone, Hydrocodone & isoquinoline alkaloid, Hydromorphinol, Hydromorphone, Hydroxypethidine, Ibogaine, Isobutylnitrit, Isomethadone, Ketamine, Ketazolam, Ketobemidone, Levamfetamine, Levo- alphacetylmethadol, Levo-methamphetamine, Levomethorphan, Levomoramide, Levophenacylmorphan, Levorphanol, Lisdexamfetamin, Loprazolam, Lorazepam, Lormetazepam, Lysergic acid, Lysergic acid amide, Lysergic acid diethylamide, Marijuana, Mazindol, MBDN (N-methyl-1-(3,4-methylenedioxyphenyl)-2- butanamine), mCPP (1-(3- chlorphenyl)piperazine), Mebutamate, Mecloqualone, Medazepam, Mefenorex, MeOPP (1-(4-methoxyphenyl)piperazine), Meperidine, Meperidine intermediate, Meprobamate, Mescaline, Mesocarb, Mesterolone, Metamfetamine, Metazocine, Methadone, Methadone intermediate, Methamphetamine, Methandienone, Methandranone, Methandriol, Methandrostenolone, Methaqualone, Methcathinone, Methenolone, Methohexital, Methyldesorphine, Methyldihydromorphine, Methylphenidate, Methylphenobarbital (mephobarbital), Methyltestosterone, Methyprylone, Metopone, Mibolerone, Midazolam, Modafinil, Moramide-intermediate, Morpheridine, Morphine, Morphine methylbromide, Morphine methylsulfonate, Morphine-N-oxide, Myrophine, N,N-Dimethylamphetamine, Nabilone, Nalorphine, Nandrolone, N-Ethyl-1-phenylcyclohexylamine, N-Ethyl-3-piperidyl benzilate, N-Ethylamphetamine, N-Hydroxy-3,4-methylenedioxyamphetamine, Nicocodeine, Nicocodine, Nicodicodine, Nicomorphine, Nimetazepam, Nitrazepam, N-Methyl-3-piperidyl benzilate, Noracymethadol, Norcodeine, Nordiazepam, Norethandrolone, Norlevorphanol, Normethadone, Normorphine, Norpipanone, Norpipanone, Opium, Oxandrolone, Oxazepam, Oxazolam, Oxycodone, Oxymesterone, Oxymetholone, Oxymorphone, Para-Fluorofentanyl, Parahexyl, Paraldehyde, Pemoline, Pentazocine, Pentobarbital, Petrichloral, Peyote, Phenadoxone, Phenampromide, Phenazocine, Phencyclidine, Phendimetrazine, Phenmetrazine, Phenobarbital, Phenomorphan, Phenoperidine, Phentermine, Phenylacetone, Pholcodine, Piminodine, Pinazepam, Pipradrole, Piritramide, PMMA (paramethyxymethyl amphetamine), Prazepam, Proheptazine, Properidine, Propiram, Psilocybine, Psilocyn, Pyrovalerone, Quazepam, Racemethorphane, Racemoramide, Racemorphane, Remifentanil, Salvia divinorum, Salvinorin A, Secobarbital, Secobarbital, Sibutramine, SPA, Stanolone, Stanozolol, Sufentanil, Sulfondiethylmethane, Sulfonethylmethane, Sulfonmethane, Talbutal, Temazepam, Tenamfetamin, Testolactone, Testosterone, Tetrahydrocannabinols, Tetrazepam, TFMPP (1-(3-triflourmethylphenyl)piperazine), Thebacon, Thebaine, Thiamylal, Thiofentanyl, Thiopental, Tiletamine & Zolazepam in Combination, Tilidine, Trenbolone, Triazolam, Trimeperidine, Vinbarbital, Zaleplon, Zipeprol, Zolpidem and Zopiclon.

Other suitable examples of a useful active drug substance include alfentanil, allylprodine, alphaprodine, anileridine, benzylmorphine, bezitramide, buprenorphine, butophanol, clonitazene, codeine, cyclazocine, desomorphine, dextromoramide, dezocine, diapromide, dihydrocodeine, dihydromorphine, dimenoxadol, dimephetanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, dextropropoxyphene, ketobemidone, levallorphan, levorphanol, levophenacylmorphan, lofentanil, meperidine, meptazinol, metazocine, methadone, metopon, morphine, morphine 6- glucuronide, morphine 3-glucuronide, myrophine, nalbuphine, narccine, nicomorphine, norlevorphanol, normethadone, nalorphine, normorphine, norpipanone, opium, oxycodone, oxycodeine, oxymorphone, papaveretum, pentazocine, phenadoxone, phenomorphan, phenazocine, phenoperidine, piminodine, piritramide, propheptazine, promedol, properidine, propiram, propoxyphene, sufentanil, tilidine, tramadol, thebaine, levo- alphacetylmethadol (LAAM), remifentanil, carfentanyl, ohmefentanyl, MPPP, prodine, PEPAP, levomethorphan, etorphine, lefetamine, loperamide, diphenoxylate or pethidine.

Other suitable examples also include Anabolic steroids, cannabis, cocaine and diazepam.

In preferred embodiments, the active substance is selected from the group consisting of the therapeutic classes including non-steroids anti-inflammatory and antirheumatic active substances.

In preferred embodiments, the active substance is selected from the group consisting of the therapeutic classes including analgesics, opioids, antipyretics, anesthetics, antimigraine agents, antiepileptics, anti-parkinson agents, dopaminergic agents, antipsychotics, anxiolytics, sedatives, antidepressants, psychostimulants agents, dopamine, noradrenaline, nicotinic, alfa-andrenergic, serotonin, $H_3$ antagonist used for ADHD and nootropics agents used in addictive disorders.

In preferred embodiments, the active substance is selected from the group consisting of the therapeutic classes including anesthetics, centrally-acting analgesics, sedative-hypnotics, anxiolytics; appetite suppressants, decongestants, antitussives, antihistamines, antiemetics, antidiarrheals, and drugs used to treat narcolepsy and attention deficit hyperactivity disorder.

In preferred embodiments, the active drug substance is associated with abuse syndromes and the active drug substance may thus for example be selected from the group consisting of opioids, CNS depressants, CNS stimulants, cannabinoids, nicotine-like compounds, glutamate antagonists and N-methyl-D-aspartate (NMDA) antagonists.

Preferably, the active drug substance is an analgesic. Examples of preferred analgesics according to the present invention includes for example Opioids, Natural opium alkaloids, Morphine, Opium, Hydromorphone, Nicomorphine, Oxycodone, Hydrocodone, Dihydrocodeine, Diamorphine, Papaveretum, Codeine, Phenylpiperidine derivatives, Ketobemidone, Pethidine, Fentanyl, Diphenylpropylamine derivatives, Dextromoramide, Piritramide, Dextropropoxyphene, Bezitramide, Methadone, Benzomorphan derivatives, Pentazocine, Phenazocine, Oripavine derivatives, Buprenorphine, Morphinan derivatives, Butorphanol, Nalbuphine, Tilidine, Tramadol, Dezocine, Salicylic acid and derivatives, Acetylsalicylic acid, Aloxiprin, Choline salicylate, Sodium salicylate, Salicylamide, Salsalate, Ethenzamide, Morpholine salicylate, Dipyrocetyl, Benorilate, Diflunisal, Potassium salicylate, Guacetisal, Carbasalate calcium, Imidazole salicylate, Pyrazolones, Phenazone, Metamizole sodium, Aminophenazone, Propyphenazone, Nifenazone, Anilides, Paracetamol, Phenacetin, Bucetin, Propacetamol, Other analgesics and antipyretics, Rimazolium, Glafenine, Floctafenine, Viminol, Nefopam, Flupirtine, Ziconotide.

Very preferred active drug substances, which are analgesics to be included in the pharmaceutical compositions according to the present invention, are opioids. Said opioids may be selected from the group consisting of naturally occurring opioids, synthetic opioids and semisynthetic opioids.

In another preferred embodiment the active drug substance is selected from the group consisting of Amfetamine, Dexamfetamine, Lisdexamfetamine, Metamfetamine, Methylphenidate, Dexmethylphenidate and combinations thereof.

In preferred embodiments of the invention the pharmaceutical compositions contain an opioid selected from the group consisting of buprenorphine, codeine, dextromoramide, dihydrocodeine, fentanyl, hydrocodone, hydromorphone, morphine, pentazocine, oxycodeine, oxycodone, oxymorphone, norhydrocodone, noroxycodone, morphine-6-glucuronide, tramadol and dihydromorphine.

Furthermore, the opioid such as morphine, hydrocodone, oxycodone or hydromorphone may be in any of its crystalline, polymorphous or amorphous forms or combinations thereof.

In a very preferred embodiment of the invention the active drug substance is selected from the group consisting of morphine, oxycodone, hydrocodone, hydromorphone, norhydrocordone, oxymorphone, noroxycodone, morphine-6-glucuronide and pharmaceutically acceptable salt thereof, such as morphine sulphate, morphine sulphate pentahydrate, oxycodone hydrochloride and hydrocodone bitartrate.

In a particular preferred embodiment of the invention the active drug substance is morphine or a pharmaceutically acceptable salt thereof, such as morphine sulphate or morphine sulphate pentahydrate.

All of the above mentioned active drug substances may also be in the form of pharmaceutically acceptable

salts, uncharged or charged molecules, molecular complexes, solvates or anhydrates thereof, and, if relevant, isomers, enantiomers, racemic mixtures, and mixtures thereof.

In particular, the pharmaceutical compositions according to the invention may comprise pharmaceutically acceptable salts of any of the above mentioned active drug substances.

The term "pharmaceutically acceptable salts" of an active drug substance includes alkali metal salts such as, e.g., sodium or potassium salts, alkaline earth metal salts such as, e.g., calcium and magnesium salts, and salts with organic or inorganic acid like e.g. hydrochloric acid, hydrobromic acid, nitric acid, sulfuric acid, phosphoric acid, citric acid, formic acid, maleic acid, succinic acid, tartaric acid, methansulphonic acid, toluenesulphonic acid etc.

The term "pharmaceutically acceptable salts" of an opioid includes alkali metal salts such as, e.g., sodium or potassium salts, alkaline earth metal salts such as, e. g., calcium and magnesium salts, and salts with organic or inorganic acids like e. g. hydrochloric acid, hydrobromic acid, nitric acid, sulfuric acid, phosphoric acid, citric acid, formic acid, maleic acid, succinic acid, tartaric acid, methansulphonic acid, toluenesulphonic acid etc or tartrate acid. Preferred salts may be selected from the group consisting of sulphate salt, hydrochloride salts and bitartrate salts.

The term "solvates" includes hydrates or solvates wherein other solvates than water are involved such as, e.g., organic solvents like chloroform and the like.

Furthermore, the active drug substance may be in any of its crystalline, polymorphous, semi-crystalline, amorphous or polyamorphous forms and mixtures thereof.

The concentration of the active drug substance in a composition for use according to the invention depends on the specific active drug substance, the disease to be treated, the condition of the patient, the age and gender of the patient etc. The above-mentioned active drug substances are well-known active drug substances and a person skilled in the art will be able to find information as to the dosage of each active drug substance and, accordingly, he will know how to determine the amount of each active drug substance in a composition. The active drug substance is typically present in a matrix composition of the invention in a concentration amount of from 0.01 % - 99 % w/w such as, e.g., from about 0.01 % to about 90 % w/w, from about 0.01 % to about 80 % w/w, from about 0.01 % to about 70 % w/w, from about 0.01 % to about 60 % w/w, from about 0.01 % to about 55% w/w, from about 0.01 % to about 50% w/w, from about 0.01 % to about 45% w/w ,from about 0.01 % to about 40% w/w, from about 0.01 % to about 35% w/w, from about 0.01 % to about 30% w/w, from about 0.01 % to about 25% w/w, from about 0.01 % to about 20% w/w, from about 0.01 % to about 15% w/w or from about 0.01 % to about 10% w/w.

When the active drug substance is an opioid, such as morphine or salts thereof, then said opioid is typically present in the matrix compositions in a concentration of in the range of 1% to 70% w/w, for example in the range of 1% to 60% w/w, such as in the range of 1% to 55% w/w, for example in the range of 1% to 50% w/w, such as in the range of 1% to 40% w/w, for example in the range of 1% to 35% w/w, such as in the range of 1% to 30% w/w, for example in the range of 1% to 20% w/w, such as in the range of 1% to 17% w/w, or the opoid, such as morphine, may be present in the matrix in the range of 5% to 60% w/w, for example in the range of 20% to 60% w/w, such as in the range of 30% to 60 % w/w, for example in the range of 30% to 55% w/w, such as in the range of 35% to 55% w/w.

In one preferred embodiment of the invention, the matrix composition comprises in the range of 1% to 17% w/w, such as 10% to 17% w/w, for example 15% to 17% w/w, such as 16% w/w of the opioid, such as morphine or salts thereof. In other embodiments of the invention, the matrix composition comprises more than 17% w/w, such as in the range of 20% to 60% w/w of the opioid, such as morphine or salts thereof.

In another preferred embodiment, the matrix composition comprises in the range of 1% to 70% w/w, for example in the range of 1% to 60% w/w, such as in the range of 1% to 50% w/w, for example in the range of 1% to 45% w/w, such as in the range of 1% to 40% w/w, for example in the range of 1% to 35% w/w, such as in the range of 1% to 30% w/w, for example in the range of 5% to 20% w/w, such as in the range of 10% to 20% w/w, for example in the range of 12% to 15% w/w of said opioid, such as hydrocodone bitartrate, or the the matrix composition may comprise in the range of 5% to 50% w/w, for example in the range of 10% to 50% w/w, such as in the range of 20% to 50% w/w, for example in the range of 30% to 50% w/w, such as in the range of 35% to 50% w/w, for example in the range of 35% to 45% w/w of said opoid, such as hydrocodone bitartrate.

In another preferred embodiment, the matrix composition comprises a high load of said opioid, wherein a high load preferably is at least 15% w/w, preferably in the range of 15% to 70% w/w, for example in the range of 15% to 60% w/w, such as in the range of 15% to 50% w/w, for example in the range of 15% to 40% w/w, such as in the range of 15% to 30% w/w, for example in the range of 20% to 30% w/w, such as in the range of 24% to 28% w/w of said opioid, such as hydrocodone bitartrate.

In yet another preferred embodiment the matrix composition comprises in the range of 1% to 70% w/w, for example in the range of 1% to 60% w/w, such as in the range of 1% to 50% w/w, for example in the range of 1% to 45% w/w, such as in the range of 1% to 40% w/w, for example in the range of 1% to 35% w/w, such as in the range of 1% to 30% w/w, for example at least 15% w/w, preferably in the range of 15% to 70% w/w, for example in the range of 15% to 60% w/w, such as in the range of 15% to 50% w/w, for example in the range of 15% to 40% w/w, such as in the range of 15% to 30% w/w, for example in the range of 20% to 30% w/w, such as in the range of 24% to 28% w/w of said opioid,

such as in the range of 20% to 50% w/w, for example in the range of 30% to 50% w/w, such as in the range of 35% to 50% w/w, for example in the range of 35% to 45% w/w, such as oxycodone hydrochloride.

In certain embodiments of the invention it is preferred that the matrix compositions comprise a low load of the active drug substance, such as an opioid. A low load is generally less then 55% w/w, preferably less than 50% w/w, more preferably even less then 45% w/w even more preferably less than 40% w/w of said active drug substance.

A pharmaceutical composition according to the invention containing an active drug substance as described herein above is typically for oral administration. In one preferred embodiment of the invention, the matrix composition provides for administration only once or twice daily.

A composition according to the invention may comprise one active drug substance or more than one different active drug substances. Typically, the amount of the active substance corresponds to a daily or part of a daily therapeutic dose.

A composition according to the invention is suitable for use for both water soluble as well as slightly soluble or substantially insoluble active substances.

## Pharmaceutically acceptable excipients

The matrix composition may also contain other excipients as well, e.g. in order to improve the technical properties of the matrix composition so that it may be easier to produce or in order to improve the properties of the composition such as release rate of the active drug substance, stability of the active drug substance or of the composition itself.

A suitable pharmaceutically acceptable excipient for use in a matrix composition of the invention may be selected from the group consisting of fillers, diluents, disintegrants, glidants, pH-adjusting agents, viscosity adjusting agents, solubility increasing or decreasing agents, osmotically active agents and solvents.

Suitable excipients include conventional tablet or capsule excipients. These excipients may be, for example, diluents such as dicalcium phosphate, calcium sulfate, lactose or sucrose or other disaccharides, cellulose, cellulose derivatives, kaolin, mannitol, dry starch, glucose or other monosaccharides, dextrin or other polysaccharides, sorbitol, inositol or mixtures thereof; binders such as alginic acid, calcium alginate, sodium alginate, starch, gelatin, saccharides (including glucose, sucrose, dextrose and lactose), molasses, panwar gum, ghatti gum, mucilage of isapol husk, carboxymethylcellulose, methylcellulose, veegum, larch arabolactan, polyethylene glycols, ethylcellulose, water, alcohols, waxes, polyvinylpyrrolidone such as PVP K90 or mixtures thereof; lubricants such as talc, silicium dioxide, magnesium stearate, calcium stearate, stearic acid, hydrogenated vegetable oils, sodium benzoate, sodium chloride, leucine, carbowax 4000, magnesium lauryl sulfate, Sodium laurilsulfate, Stearyl alcohol, Polysorbate 20, Polysorbate 60, Polysorbate 80, Macrogol stearate, Macrogol lauryl ether, Stearoyl macrogolglycerides, Sorbitan stearate, Sorbitan laurate, Macrogol glycerol hydroxystearat, colloidal silicon dioxide and mixtures thereof, disintegrants such as starches, clays, cellulose derivatives including crosscarmellose, gums, aligns, various combinations of hydrogencarbonates with weak acids (e.g. sodium hydrogencarbonate/tartaric acid or citric acid) crosprovidone, sodium starch glycolate, agar, cation exchange resins, citrus pulp, veegum, glycollate, natural sponge, bentonite, sucralfate, calcium hydroxyl-apatite or mixtures thereof.

The composition such as the matrix composition may comprise one or more agents selected from the group consisting of gelling agents. By the term "gelling agent" as used herein is meant any substance, which is capable of providing the texture of a gel, when added to a liquid solution. Examples are polymers selected from the group consisting of modified or unmodified water soluble natural polymers such as glucomannan, galactan, glucan, polygalacturonic acid, polyxylane, polygalactomannans, polyxyloglycan, arabinogalactan, starch, cellulose, chitosan, alginate, fibrin, collagen, gelatin, amylopectin, pectin including low methylated or methoxylated pectins, dextran; synthetic polymers such as PVA and PVB; and hydrogels made from the polymers or combined polymers mentioned above and or from polymers originated from: HEMA, HEEMA, MEMA, MEEMA, EDGMA, NVP, VAc, AA, acrylamide, MAA, HPMA, PEGA, PEGMA, PEGDMA, PEGDA, and/or PEGDMA, hydroxypropyl methylcellulose, hydroxypropyl cellulose, methylcellulose, hydroxyethyl ncellulose, ethylcellulose, hydroxypropyl methylcellulose phthalate, hydroxypropyl methylcellulose Acetate Succinate or other cellulose derivates, carboxymethylcellulose sodium, carboxymethylcellulose calcium, carrageenans, guar gum, gellan gum, xanthan gum, tragacanth and Arabic gum.

Furthermore, the composition may comprise one or more agents selected from the group consisting of sweetening agents, flavouring agents and colouring agents, in order to provide an elegant and palatable preparation. Examples are maltol, citric acid, water soluble FD&C dyes and mixtures thereof with corresponding lakes and direct compression sugars such as Di-Pac from Amstar. In addition, coloured dye migration inhibitors such as tragacanth, acacia or attapulgite talc may be added. Specific examples include Calcium carbonate, 1,3,5-trihydroxybenzene, Chromium-cobalt-aluminium oxide, ferric ferrocyanide, Ferric oxide, Iron ammonium citrate, Iron (III) oxide hydrated, Iron oxides, Carmine red, Magnesium carbonate and Titanium dioxide.

Plasticizers may be incorporated in the composition. A suitable plasticizer may be selected from the group consisting of mono- and di-acetylated monoglycerides, diacetylated monoglycerides, acetylated hydrogenated cottonseed glyceride, glyceryl cocoate, Polyethylene glycols or polyethylene oxides (e.g. with a molecular weight of about 1 ,

000-500,000 daltons), dipropylene glycol salicylate glycerin, fatty acids and esters, phthalate esters, phosphate esters, amides, diocyl phthalate, phthalyl glycolate, mineral oils, hydrogenated vegetable oils, vegetable oils, acetylated hydrogenated soybean oil glycerides, Castor oil, acetyl tributyl citrate, acetyl triethyl citrate, methyl abietate, nitrobenzene, carbon disulfide, [beta]-naphtyl salicylate, sorbitol, sorbitol glyceryl tricitrate, fatty alcohols, cetostearyl alcohol, cetyl alcohol, stearyl alcohol, oleyl alcohol, myristyl alcohol, sucrose octaacetate, alfa<~>-tocopheryl polyethylene glycol succinate (TPGS), tocopheryl derivative, diacetylated monoglycerides, diethylene glycol monostearate, ethylene glycol monostearate, glyceryl monooleate, glyceryl monostearate, propylene glycol monostearate, macrogol esters, macrogol stearate 400, macrogol stearate 2000, polyoxyethylene 50 stearate, macrogol ethers, cetomacrogol 1000, lauromacrogols, nonoxinols, octocinols, tyloxapol, poloxamers, polyvinyl alcohols, polysorbate 20, polysorbate 40, polysorbate 60, polysorbate 65, polysorbate 80, polysorbate 85, sorbitan monolaurate, sorbitan monooleate, sorbitan monopalmitate, sorbitan monostearate, sorbitan sesquioleate, sorbitan trioleate, sorbitan tristearate and sucrose esters, amyl oleate, butyl oleate, butyl stearate, diethylene glycol monolaurate, glycerol tributyrate, Cumar W-1 , Cumar MH-1 , Cumar V-1 , Flexol B-400, monomeric polyethylene ester, Piccolastic A-5, Piccalastic A-25, Beckolin, Clorafin 40, acetyl tributyl citrate, acetyl triethyl citrate, benzyl benzoate, butoxyethyl stearate, butyl and glycol esters of fatty acids, butyl diglycol carbonate, butyl ricinoleate, butyl phthalyl butyl glycolate, camphor, dibutyl sebacate, dibutyl tartrate, diphenyl oxide, glycerine, HB-40, hydrogenated methyl ester of rosin, methoxyethyl oleate, monoamylphthalate, Nevillac 10, Paracril 26, technical hydroabietyl alcohol, Methylene glycol dipelargonate, solid aliphatic alcohols and mixtures thereof.

Preferred stabilizers (chemical) include TPG preferably in the form of TPGS (Vitamin E Polyehtylene glycol succinate) due to surfactant properties and BHT, BHA, t-butyl hydroquinone, calcium ascorbate, gallic acid, hydroquinone, maltol, octyl gallate, sodium bisulfite, sodium metabisulfite.tocopherol and derivates thereof, citric acid, tartaric acid, and ascorbic acid. Thus, in one preferred embodiment, the matrix composition comprises TPGS and/or BHT. Other stabilisers include trivalent phosphorous like e.g phosphite, phenolic antioxidants, hydroxylamines, lactones such as substituted benzofuranones. Hindered phenols, thiosynergists and/or hindered amines, acids (ascorbic acid, erythorbic acid, etidronic acid, hypophosphorous acid, nordihydroguaiaretic acid, propionic acid etc.), phenols, dodecyl gallate, octyl gallate, 1,3,5-trihydroxybenzene, organic and inorganic salts (calcium ascorbate, sodium ascorbate, sodium bisulphite, sodium metabisulfite, sodium sulfite, potassium bisulphite, potassium metabisulphite), esters (calcium ascorbate, dilauryl thiodipropionate, dimyristyl thiodipropionate, distearyl thiodipropionate), pyranon (maltol), and vitamin E (tocopherol, D-[alpha]-tocopherol, DL-[alpha]-tocopherol, tocopheryl acetate, d-[alpha]-tocopheryl acetate, dl-[alpha]-tocopheryl acetate. However, other anti- oxidative agents known in the art may be used according to the present invention. Other suitable stabilizer is selected from such as e.g. sorbitol glyceryl tricitrate, sucrose octaacetate.

In one preferred embodiment the matrix comprises one or more stabilizers selected from above mentioned group of stabilizers, preferably butylhydoxytoluene (BHT).

In another preferred embodiment the matrix comprises one or more stabilizers selected from above mentioned group of stabilizers, preferably TPGS.

A release modifier may be incorporated in the composition. A suitable release modifier is selected from the group consisting of fatty acids and esters, fatty alcohols, cetyl alcohol, stearyl alcohol, mineral oils, hydrogenated vegetable oils, vegetable oils, acetylated hydrogenated soybean oil glycerides, Castor oil, phosphate esters, amides, phthalate esters, glyceryl cocoate oleyl alcohol, myristyl alcohol, sucrose octaacetate, diacetylated monoglycerides, diethylene glycol monostearate, ethylene glycol monostearate, glyceryl monooleate, glyceryl monostearate, propylene glycol monostearate, macrogol esters, macrogol stearate 400, macrogol stearate 2000, polyoxyethylene 50 stearate, macrogol ethers, cetomacrogol 1000, lauromacrogols, poloxamers, polyvinyl alcohols, sorbitan monolaurate, sorbitan monooleate, sorbitan monopalmitate, sorbitan monostearate, sorbitan sesquioleate, sorbitan trioleate, sorbitan tristearate, ethylcellulose, cellulose acetate, cellulose propionate, cellulose nitrate, cellulose derivative selected from the group consisting of methylcellulose, carboxymethylcellulose and salts thereof, cellulose acetate phthalate, microcrystalline cellulose, ethylhydroxyethylcellulose, ethylmethylcellulose, hydroxyethylcellulose, hydroxyethylmethylcellulose, hydroxypropylcellulose, hydroxypropylmethylcellulose, hydroxymethylcellulose and hydroxymethylpropylcellulose, cellulose acetate, polylactic acid or polyglycolic acid and copolymers thereof, methacrylates, a co-polymer of methacrylate-galactomannan etc., Polyvinyl alcohols, glycerinated gelatine and cocoa butter.

Other suitable release modifiers may be selected from the group consisting of inorganic acids, inorganic bases, inorganic salts, organic acids or bases and pharmaceutically acceptable salts thereof, saccharides, oligosaccharides, polysaccharides, polyethylene glycol derivatives and cellulose and cellulose derivatives.

Alternatively or additionally, a suitable pharmaceutically acceptable excipient is a mono-, di-, oligo, polycarboxylic acid or amino acids such as, e.g. acetic acid, succinic acid, citric acid, tartaric acid, acrylic acid, benzoic acid, malic acid, maleic acid, sorbic acid etc., aspartic acid or glutamic acid etc.

Examples of suitable organic acids include for example acetic acid/ ethanoic acid, adipic acid, angelic acid, ascorbic acid/vitamin C, carbamic acid, cinnamic acid, citramalic acid, formic acid, fumaric acid, gallic acid, gentisic acid, glutaconic acid, glutaric acid, glyceric acid, glycolic acid, glyoxylic acid, lactic acid, levulinic acid, malonic acid, mandelic acid, oxalic acid, oxamic acid, pimelic acid, or pyruvic acid.

Examples of suitable inorganic acids include for example pyrophosphoric, glycerophosphoric, phosphoric such as ortho and meta phosphoric, boric acid, hydrochloric acid, or sulfuric acid.

Examples of suitable inorganic compounds include for example aluminium.

Examples of organic bases include for example p-nitrophenol, succinimide, benzenesulfonamide, 2- hydroxy-2cyclohexenone, imidazole, pyrrole, diethanolamine, ethyleneamine.tris (hydroxymethyl) aminomethane, hydroxylamine and derivates of amines, sodium citrate, aniline or hydrazine. Examples of inorganic bases include for example aluminium oxide such as, e.g., aluminium oxide trihydrate, alumina, sodium hydroxide, potassium hydroxide, calcium carbonate, ammonium carbonate, ammonium hydroxide or KOH.

Suitable pharmaceutically acceptable salts of an organic acid is e.g. an alkali metal salt or an alkaline earth metal salt such as, e.g. sodium phosphate, sodium dihydrogenphosphate, disodium hydrogenphosphate etc., potassium phosphate, potassium dihydrogenphosphate, potassium hydrogenphosphate etc., calcium phosphate, dicalcium phosphate etc., sodium sulfate, potassium sulfate, calcium sulfate, sodium carbonate, sodium hydrogencarbonate, potassium carbonate, potassium hydrogencarbonate, calcium carbonate, magnesium carbonate etc., sodium acetate, potassium acetate, calcium acetate, sodium succinate, potassium succinate, calcium succinate, sodium citrate, potassium citrate, calcium citrate, sodium tartrate, potassium tartrate or calcium tartrate.

A suitable inorganic salt for use in a matrix composition of the invention is for example sodium chloride, potassium chloride, calcium chloride or magnesium chloride.

The matrix composition may preferably comprise at least one saccharide, such as glucose, ribose, arabinose, xylose, lyxose, xylol, allose, altrose, inosito, glucose, sorbitol, mannose, gulose, Glycerol, idose, galactose, talose, mannitol, erythritol, ribitol, xylitol, maltitol, isomalt, lactitol, sucrose, fructose, lactose, dextrin, dextran, amylase or xylan. In a preferred embodiment the matrix composition comprises mannitol.

The matrix composition may also comprise polyethylene glycol derivatives such as e.g. polyethylene glycol di (2-ethyl hexoate), polyethylene glycols (200 - 600 daltons) or polyethylene oxides, e.g. with an average molecular weight of about 800-500,000 daltons, typically about 1,000-100,000 daltons, more typically 1,000-50,000 daltons, especially about 1,000-10,000 daltons, in particular about 1,500-5,000 daltons, or mixtures thereof.

The matrix composition may also comprise cellulose and/or cellulose derivatives selected from the group consisting of methylcellulose, carboxymethylcellulose and salts thereof, microcrystalline cellulose, ethylhydroxyethylcellulose, ethylcellulose, cellulose acetate, cellulose proprionate, cellulose nitrate, cellulose acetate phthalate, ethylmethylcellulose, hydroxyethylcellulose, hydroxyethylmethylcellulose, hydroxypropylcellulose, hydroxypropylmethylcellulose, hydroxymethylcellulose and hydroxymethylpropylcellulose.

**Preparation**

The pharmaceutical composition as well as the matrix composition of the invention may be produced by various methods which are either known per se in the pharmaceutical industry or which, for example, are used in the production of polymer-based materials, depending upon the desired embodiment and the materials employed in the composition in question. One advantage of the composition according to the invention is that it may be produced by methods, which are relatively simple and inexpensive. Suitable preparation methods for compositions according to the invention include extrusion, injection moulding, moulding, tabletting, capsule filling, melt-processed, thermoforming, spray coating, micro encapsulation and other methods of preparing controlled release compositions. Also a combination of one or more of the aforementioned may be employed.

The controlled release composition may be prepared by several different methods. Many systems for controlled release are marketed and it is currently an aim for the industry to reduce the risk of dose dumping, drug abuse or alcohol induced dose dumping in each of the systems.

In other words, in addition to a less frequent administration, one challenge in controlled release delivery may be expressed by the goal of decreasing the incidence of adverse effects and at the same time increasing the effect of the treatment. This may be obtained by an interaction between the specific pharmacological properties of the active drug substance and the matrix composition.

High concentrations or a fast rise in the concentration of for example morphine is one important factor resulting in side effects including the risk of getting addicted to morphine. The fear of addiction is often a major obstacle for initiation of the otherwise effective pain treatment with morphine both in the view of the clinical personnel as well as in the view of the patients themselves.

Compositions for controlled release according to the invention may be prepared in numerous ways giving rise to different release mechanisms. Particularly the composition may be prepared by 1, 2 or multiple component injection mouldings, by conventional tablet compression, by micro encapsulation, by 1, 2 or multiple component extrusions, by capsule filling, melt-processed or by thermoforming. In cases where a preparation is needed in order to make the controlled release properties before/after the above mentions preparation steps, the preparation may also comprise separate steps as for example wet granulation, dry granulation, melt granulation, pelletizing, spray coating, electrostatic

coating or other forms of controlled release forming preparation methods.

In a particular example the composition is prepared by two component injection moulding of a matrix composition and a coating (which may be any of the coatings described herein below in the section "Coating") surrounding the matrix and exposing at least one surface of the matrix, preferably the two ends of the matrix composition for erosion governed release.

A composition may also be produced by, for example, injection moulding, melt-processing, co-extrusion of the coating with the matrix composition and the active drug substance, extrusion and dip coating, injection moulding and dip coating, or by extrusion or injection moulding and solvent coating by spraying or dipping. Multiple component injection moulding, or a combination of these methods.

**Geometry**

The release mechanisms described above depends on the geometry of the composition. For example erosion based release from a matrix depends on the exposed area of the matrix. In this case the area may be manipulated by employment of a coat that is not subject to erosion and thus covering the areas of the matrix that hence will not be a releasing site.

Preferably, the pharmaceutical compositions of the invention are cylindrical compositions optionally with tapered end(s). It follows that the matrix composition also preferably is of a cylindrical shape (optionally with tapered end(s)), which preferably is surrounded by a coating having at least one opening exposing one surface of said matrix.

The cylindrical shape may be any geometrical shape having the same cross section area throughout the length of the geometrical shape. Within the present context, cross sections are perpendicular to the axis of the cylinder. By way of example, if the cylindrical shape is elongated then the cross sections are perpendicular to the longitudinal axis. Preferably, the cylindrical shape is elongated. The cross section of a cylinder within the meaning of the present invention may have any two dimensional shape, for example the cross section may be circular, oval, parabola, hyperbola, rectangular, triangular, otherwise angular, star shaped or an irregular shape. The pharmaceutical compositions according to the invention preferably have a cylindrical shape, wherein the end(s) may be tapered.

Accordingly, the cylindrical shape may for example be an elliptic cylinder, a parabolic cylinder, a hyperbolic cylinder or a prism. A prism within the present context is a cylinder whose cross-section is a polygon.

The pharmaceutical composition as well as the matrix composition according to the invention may be a cylindrical shape with one tapered end or two tapered ends.

Preferably, the matrix composition is being surrounded by a coating having at least one opening, for example one opening, such as two openings each exposing one surface of said matrix. Preferably, said at least one opening is exposing one end of the cylindrical shape, more preferably the coating has two openings each exposing an end of the cylindrical shape.

Thus, the pharmaceutical composition may be a cylindrical shape with the two ends exposing the eroding matrix composition. Such a shape will give rise to zero order release because the releasing area is constant.

The geometric form of the composition is very important for the obtainment of the above-mentioned controlled release. Thus, in one embodiment of the invention, the pharmaceutical composition has a geometric shape, which enables a substantially constant surface area to become exposed during erosion of the matrix.

In a specific example, the compositions employed are coated in such a manner that the surface has a substantially constant or controlled surface area during release or erosion. In the present context controlled surface area relates to a predetermined surface area typically predicted from the shape of the coat of the unit dosage system. It may have a simple uniform cylindrical shape or the cylindrical form can have one or more tapered ends in order to decrease (or increase) the initial release period. As another example, in diffusion based systems the release will furthermore depend on the thickness of the diffusion layer and in this case the release will depend both on the diffusion area and thickness of the diffusion system.

As yet another example the release mechanism of dissolving/solubilization also depend on the releasing area and the release rate may be controlled by covering parts of the releasing matrix by a coat. Controlling the coverage of the matrix by the coat hence preferably refers to covering from 0 to 99% of the matrix by a coat.

In a preferred embodiment of the invention the pharmaceutical composition is prepared for oral intake, preferably for oral intake by swallowing. Accordingly, the size of the pharmaceutical composition should be in a range that allows oral intake by swallowing.

**Coating**

The composition may be partly or fully covered by a coat with specific properties in such a way that the exposed area of the matrix may be controlled by the use of a coat.

For the present purpose, it is important to ensure that the coating is impermeable to an aqueous medium, such

as water. This ensures that the matrix only is in contact with surrounding aqueous media via the openings in the coatings. In addition it is preferred that the coating also is substantially insoluble in an aqueous medium, preferably the coating is insoluble in an aqueous medium.

In a specific example the coating is substantially insoluble, non-erodable and impermeable to water leaving only the exposed areas of the matrix for release. Within the present context, the coating is considered substantially insoluble in an aqueous medium if the coating dissolves so much slower in an aqueous medium than the matrix composition so that the coating remains intact until the matrix has eroded and/or released the active drug substance.

Preferably, the coating is considered substantially insoluble in water, when it has a solubility in water of at least 100, for example at least 1000, wherein solubility is determined as parts of water needed to dissolve 1 part of solute at ambient temperature. Preferably, the coating is considered insoluble in water, when it has a solubility in water of at least 10.000, wherein solubility is determined as parts of water needed to dissolve 1 part of solute at ambient temperature.

In an embodiment of the invention, the coating is one, which biodegrades, disintegrates crumbles or dissolves after erosion of the matrix and/or during the release of the active drug substance. A coating applied for an erosion matrix will remain intact as long as it is supported by the matrix containing the active drug substance, but it lacks the ability to remain intact after erosion of the matrix, because it then biodegrades, disintegrates or crumbles, so that it will not remain in e.g. a human for any significant amount of time after the complete erosion of the matrix and the release of the active drug substance.

In a one embodiment of the invention, the coating may biodegrade, disintegrate, crumble or dissolve after erosion of the matrix composition and/or during the release of the active drug substance in the matrix composition.

The coating may in general comprise or even consist of one or more polymers. It is preferred that at least some, however more preferably all of these polymers are thermoplastic polymers.

Thus, in one embodiment of the invention all the polymers comprised in the coating are thermoplastic polymers. By thermoplastic polymers is meant that the polymer(s) is/are an elastic and flexible liquid when heated and freezes to a solid state when cooled (e.g. cooled to 20°C or to ambient temperature).

The coating may be made of a material comprising one or more of the polymers described herein in this section, e.g. a material comprising one or more starch based polymers, one or more cellulose based polymers, one or more synthetic polymers, one or more biodegradable polymers or a combination thereof, such as mixtures of starch and synthetic polymers or mixtures of starch and biodegradable polymers.

The coating may preferably be made of a material comprising one or more polymers selected from the group consisting of Ethyl cellulose grade 20 and 100, polylactic acid (PLA), Cornpack 200, polycaprolactone, PEO 7000000 and polyhydroxybuturate.

*Starch based polymers*

The coating may comprise one or more starch based polymers. The starch based polymer may be starch as such or a polymer having a high starch content, preferably more than 70%, such as more than 80%, for example more than 90%. Starch is a linear polysaccharide made up of repeating glucose groups with glycosidic linkages in the 1-4 carbon positions with chain lengths of 500 to 2,000 glucose units. There are two major polymer molecules in starch - amylose and amylopectin.

The starch based polymers to be used according to the present invention may preferably be thermoplastic starch biodegradable plastics (TPS). TPS have a starch (amylose) content greater than 70% and are in general based on gelatinised vegetable starch. Said vegetable starch may for example be selected from the group consisting of potato starch, rice starch, maize starch, tapioca starch, wheat starch, dextrin, carrageenan and chitosan. Said vegetable starch may also as such be suitable polymers used in the coating composition. The group of starch based polymer in general do not have a specified melting point, but changes phase within a temperature range of 90 °C to 260 °C typically depending upon the chain length of the starch based polymer, water content, and their branching and added side-groups as does the degree of crystallinity of the starch. Long chained-starches are usually completely amorphous, while shorter length starches may be semi-crystalline (20-80% crystalline). Long polymer chains are preferable because it contributes to the hardness, while not being too brittle.

Starch-based polymers are in general fully biodegradable as they are product of plant materials. The degradation rate varies and can be further induced by addition of other biodegradable polymers as listed herein.

One example of a preferred starch based polymer, which may be comprised in the coating or coating according to the present invention is maize starch. Maize starch is a linear polysaccharide made up of repeating glucose groups with glycosidic linkages in the 1-4 carbon positions with chain lengths of 500 to 2,000 glucose units. There are two major polymer molecules in starch - amylose and amylopectin. A preferred maize starch is cornpack. Cornpack is the maize starch used in some examples described herein below.

Starch is widely used in food and pharmaceutical industry as binder and dilluent. It is edible and essentially nontoxic. Starch is in general cheap and obtains a good hardness when moulded and thermoformed. Starch may in

general also be reheated several times without losing its thermodynamic properties. Accordingly, it is preferred that the coating comprises at least one starch based polymer, and more preferably a starch, because starch may be a great advantage when applying injection moulding or co-extrusion as a production process.

Starch based polymers are in general decomposable, and usually have a fast disintegration rate, especially in mixture with biodegradable polymers. These polymers are in generally recognized as stabile and inert in solid dosage forms.

*Cellulose based polymers*

The coating may also comprise one or more cellulose based polymers. In certain embodiments of the invention the coating may even consist of one or more cellulose based polymers (such as ethyl cellulose) and platizicers (such as any of the plastizicers described in this section below) and UV stabilisers (such as any of the UV stabilisers described in this section below).

Cellulose based polymers are useful in the coating composition because cellulose based polymers e.g. ethylcellulose (particularly grade 100-300) frequently have increased hardness and high ductility.

It is therefore preferred that the coating comprises a cellulose based polymer, preferably a cellulose based polymer, which is substantially insoluble in an aqueous medium, more preferably a cellulose based polymer, which is insoluble in an aqueous medium. The cellulose based polymer is preferably cellulose, wherein one or more of the free -OH groups have been substituted with an R-group to form a -O-R group. R may in this context for example be linear or branched lower alkyl, linear or branched lower alkyl-OH, linear or branched lower alkyl-COOH, -CO-(linear or branched lower alkyl), nitrate, aromatic rings or combinations of the aforementioned. Lower alkyl is preferably a $C_{1-10}$ alkyl, more preferably $C_{1-6}$ alkyl.

Accordingly, the cellulose based polymer may for example be one or more selected from the group consisting of ethylcellulose, cellulose acetate, cellulose propionate, cellulose nitrate, methylcellulose, carboxymethylcellulose and salts thereof, cellulose acetate phthalate, ethylhydroxyethylcellulose, ethylmethylcellulose, hydroxymethylcellulose, hydroxyethylmethylcellulose, hydroxypropylcellulose, hydroxypropylmethyl-cellulose, hydroxymethylcellulose and hydroxymethylpropylcellulose and cellulose acetate.

The coating may also comprise one or more cellulose based polymers selected from the group consisting of cellulose acetate, cellulose propionate, silicified microcrystalline cellulose, cellulose nitrate, methylcellulose, carboxymethylcellulose and salts thereof, cellulose acetate phthalate, microcrystalline cellulose, ethylhydroxyethylcellulose, ethylmethylcellulose, hydroxyethylcellulose, hydroxyethylmethylcellulose, hydroxyl-propylcellulose, hydroxypropylmethylcellulose, hydroxymethylcellulose phthalate, hydroxymethylcellulose and hydroxymethylpropylcellulose, cellulose acetate, ceratonia(high molecular-weight 310 000), Eudragit L methyl ester, Eudragit RL and Eudragit E.

Cellulose based polymers are in general fully biodegradable as they preferably are products of plant materials. The degradation rate is in general slower than for starch based polymers. This degradation rate can be induced by addition of other biodegradable polymers as listed herein. These other polymers may be polymers which can be attacked by microorganism which degrades the coating composition into smaller pieces giving rise to a bigger surface and thereby faster degradation.

It is very preferred that the coating comprises ethyl cellulose $C_{12}H_{23}O_6(C_{12}H_{22}O_5)_nC_{12}H_{23}O_5$ where n can vary to provide a wide variety of molecular weights. Ethylcellulose, an ethyl ether of cellulose, is a long-chain polymer of β-anhydroglucose units joined together by acetal linkages Ethyl cellulose comes in different grades which varies in molecular weight and number of ethoxy groups. Grades from 20 - 300 are preferred and these are also commercially available. Grades with high molecular weights are also preferred because they are optimal to give a hard coating. The coating may comprise one or more ethyl celluloses with different grades, for example one ethyl cellulose with a grade of in the range of 20 to 300, preferably in the range of 20 to 100, more preferably in the range of 20 to 40, such as 20 and another ethyl cellulose with a grade of in the range of 20 to 300, preferably in the range of 50 to 200, more preferably in the range of 80 to 120, such as 100. Ethyl cellulose generally has a glass transition temperature within 129-133°C. These polymers are widely used in food and pharmaceutical industry as coater, stabilizer, matrix former and taste masking and are regarded as non toxic substances.

Cellulose based polymers are in general derived from plant material and may subsequently be modified. Many cellulose based polymers are cheap and give a good hardness when moulded and thermoformed. As derivatives of plants, cellulose based polymers are in general easily decomposable when disposed. These polymers are stabile and inert in solid dosage.

*Synthetic polymers*

The coating according to the invention may also comprise one or more synthetic polymers. Suitable synthetic polymers for use in the coating composition may for example be one or more selected from the group consisting of

polyamide, polyethylene, polyethylene terephthalate, polypropylene, polyurethane, polyvinyl acetate, polyvinyl alcohol, polyvinyl butural, polyvinyl chloride, silicone rubber, latex, teflon, copolymers such as ethylene vinyl acetate (EVA), styrene-butadienestyrene (SBS) and styrene-isoprene-styrene (SIS), Polyethylene glycols, polyvinylpyrrolidone, polyethylene oxide (ranging in molecular weights 100,000 to 8,000,000), carboxymethylene (Carbomer) and sugars thereof (e.g. allylsucrose,) and co-polymers of ethylene and propylene oxide (PoloXamer).

*Biodegradable polymers*

Biodegradation is the process by which microorganisms (microbes such as bacteria, fungi or algae) convert materials into biomass, carbon dioxide and water. Biomass is a general term used to refer to the cells of the microorganisms that are using the material as a carbon source to grow on.

The coating may also comprise one or more biodegradable polymers. Said biodegradable polymer(s) may be one or more selected from the group consisting of starch based polymers as described herein above in this section and cellulose based polymers as described herein above in this section. However the biodegradable polymer may also one or more selected from the group consisting of polyhydroxybutyrate(PHB), polyhydroxyvalerate(PHV), polyhydroxyvalerate-co-hydroxy-valerate(PHV/VH), Polyhydroxyalkanoates(PHA), poly-3-hydroxy-5-phenylvalerate (PHPV), aliphatic polyesters, polycaprolactone(PCL), polylactic acid(PLA), polyglycolic acid(PGA), copolymers or block copolymers of polycaprolactone(PCL), polylactic acid(PLA) and/or polyglycolic acid(PGA), poly-propylene carbonate (PPC), polyester amide (PEA), polybutylene succinate adipate (PBSA), polybutylene adipate co-terephtalate (PBAT) and polybutylene succinate-adipate (PESA).

Copolymers or block copolymers of polycaprolactone(PCL), polylactic acid(PLA) and/or polyglycolic acid(PGA) may for example be selected from the group consisting of poly(lactic-co-glycolic acid)(PLGA), polylactic acid and epsilon-caprolactone copolymer (PLA/CL) and polylactic acid/glycolic acid polymers)(PLA/GA), which are all commercially available.

In a preferred embodiment the coating comprises one or more biodegradable polymers selected from the group consisting of polylactic acid(PLA), polycaprolactone(PCL) and polyhydroxybutyrate(PHB), preferably the coating comprises both polylactic acid(PLA), polycaprolactone(PCL) and polyhydroxybutyrate(PHB).

The use of polycaprolactone and other polymers in this group has been increased over the last decade, while the demand for environmental friendly plastics has grown. These polymers are regarded as nontoxic and are already used in parenteral pharmaceutical formulations. The advantages of these polymers are their ability to make a more flexible coating when moulded in mixture with starch derived polymers. The somewhat rigid structure of pure thermoplastic starch is improved. Furthermore the polymers are decomposable and disintegrate by microorganisms.

*Polylactic acid*

Polylactic acid or polylactide (PLA) is a biodegradable, thermoplastic, aliphatic polyester derived from renewable resources, such as corn starch. PLA belongs to the chemical family of polyesters, such as e.g. ε-caprolactone, PLA-caprolactone in different *ratios 15% PLA to 100% (25, 35, 50, 75, 85%),* polyglycolides, polyglycolic acids (PGA), poly (lactide-co-glycolide) in different *ratios 15 to 100% PLA (25, 35, 50, 75, 85%),* poly (lactide-co-glycolide)-OH in different *ratios 15% PLA to 100% (25, 35, 50, 75, 85%).* Each of the before mentioned polymers exist in L or D- form (making them optically active) and in equal amounts (1:1) of L- and D-forms results in an amorphous mixture, while the L- or D-form all possess a certain degree of crystallinity. The degree of crystallinity is highly related to the mechanical properties (incl. processability), physico-chemical properties related to particularly stability of the polymer. A high degree of crystallinity provides hardness, and possibly, more brittleness. This may affect processability as well as highly crystalline materials have a high melting temperature, hence process temperature, while amorphous esters have a lower melting temperature and thus a lower process temperature.

Moreover, an increased degree of crystallinity implies that the material is more thermodynamically stable, which leads to a longer shelf-life. A lower degree of crystallinity or amorphous materials are usually softer with a lower process temperature. The back-draw of amorphous materials or materials with a lower degree of crystallinity is that their physic-chemical stability is lower as it is in a thermodynamically unstable state.

Regarding PLA, it is necessary to find the optimal degree of crystallinity. Each degree of crystallinity has different mechanical properties, thus its adhesion to the matrix will vary depending on the degree of crystallinity of the given material (PLA).

The skeletal structure of PLA is shown below.

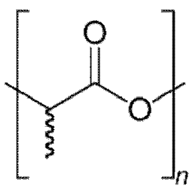

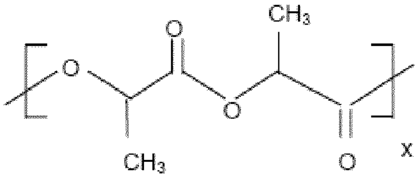

Due to the chiral nature of lactic acid, several distinct forms of polylactide exist: poly-L-lactide (PLA in its L-form) referred to as PLLA is the product resulting from polymerization of L,L-lactide (also known as L-lactide) and poly-D-lactide (PLA in its D-form) referred to as PDLA is the product resulting from polymerization of L,L-lactide (also known as L-lactide). Furthermore, PLLA and PDLA may be mixed with various ratios of the two stereo forms. As the L-form has stronger mechanical properties than the D-form and the L-form has been used in pharmaceutical products, it is attempted to optimize the blend by adding the D-form to the L-form i.e. in amounts of 5, 10, 20, 30, 40% w/w up to a ratio of 1:1, consequently making the material completely amorphous, however it may also form a highly regular stereo complex with increased crystallinity, since addition of PDLA increases the molecular energy of the mixture by forming a concentration gradient. Depending on the extent/magnitude of the temperature gradient, it may induce slow nucleation and hence crystallization. On the other hand, it may as well induce a nucleation with an incontrollable nucleation rate, which leads to an amorphous state.

PLA in its L-form has a crystallinity of around 35-45%, a glass transition temperature between 35-80 °C and a melting temperature between 173-178 °C.

Due to the structure of PLA, PLA may be exposed to hydrolysis during its path through the gastro-intenstinal tract, however PLA is impermeable and insoluble in aqueous media and in relation to applying PLA as shell material, it has been demonstrated that the shell at least macroscopically is intact within the first 48 hours of exposure. Furthermore, the possible degradation product of PLA is merely lactic acid.

*Polyglycols*

It is also comprised within the present invention that the coating may comprise any of the above-mentioned polyglycols in a form, which erodes at a substantially slower rate than the matrix composition. The coating may thus be one which is eroded in an aqueous medium at a substantially slower rate than the matrix composition comprising the active drug substance, whereby a substantially controlled area of the matrix composition comprising the active drug substance is exposed during erosion and/or release of the matrix composition, and whereby the coating is substantially eroded upon erosion and/or release of the matrix composition comprising the active drug substance. Such a coating will preferably be designed so that its longitudinal erosion rate is substantially the same as the longitudinal erosion and/or release rate of the matrix, whereby the matrix and the coating will erode longitudinally towards the centre of the composition at substantially the same rate. Thus, when the matrix composition has been completely eroded and/or released by the aqueous medium, the coating will also be substantially completely eroded. A matrix composition having such a coating has the obvious advantage of being completely biodegraded upon release of the active drug substance.

A preferred polyglycol to be comprised within the coating is high molecular weight PEO, preferably PEO with an average molecular weight which is significantly higher that the average molecular weight of any of the PEOs contained in the matrix composition. Thus, for any given pharmaceutical composition it is preferred that any PEO contained in the coating has a significantly higher average molecular weight than any PEO contained in the matrix. Accordingly, it is preferred that the coating comprises one or more PEO with an average molecular weight of at least 900,000, more preferably at least 2,000,000, yet more preferably at least 4,000,000, even more preferably at least 6,000,000, such as approximately 7,000,000, for example 7,000,000.

*Mixtures of polymers*

As noted herein above the coating may comprise one or more different polymers, and in particular one or more different polymers selected from the group consisting of starch based polymers, cellulose based polymers, synthetic

polymers and biodegradable polymers, in particular from the group consisting of any of the starch based polymers, cellulose based polymers, synthetic polymers and biodegradable polymers described herein above in this section.

In one embodiment of the invention it is preferred that the coating comprises polymers selected from or even that all polymers of the coating are selected from the group consisting of starch based polymer and biodegradable polymers, preferably from the group consisting of any of the starch based polymers and biodegradable polymers described herein above in this section. In particular, biodegradable polymers such as polycaprolactone, polyhydroxybuturate, polyhydroxyvalerate, polylactic acid, poly-hydroxyalkanoates and/or polypropylenecarbonate can be blended with various starches (such as any of the starches described herein above in this section) in different ratios. Suitable mixtures for use in the coating composition are e.g. polycaprolactone and sago and/or cassava starch, polycaprolactone or polyhydroxy-buturate and pre-dried, thermoplastic starch, polycaprolactone and gelatinized starch or thermoplastic starch. Other suitable mixtures are starch-based blends with biodegradable thermoplastic components like polyester amide, polyhydroxybuturate-co-valerate or polybutylene succinate-adipate. Polymers starches can be cross-linked with Maleic anhydride (MA) and dicumyl peroxide (DCP) giving harder items when moulded and thermoformed.

In another embodiment of the invention it is preferred that the coating comprises polymers selected from or even that all polymers of the coating are selected from the group consisting of starch based polymer and synthetic polymers, preferably from the group consisting of any of the starch based polymers and synthetic polymers described herein above in this section. In particular, suitable mixtures for use in the coating composition are e.g. native granular starch, modified starch, plasticized starch blended or grafted with many synthetic polymers such as polyethylene, polystyrene, Purified Terephthalic acid (PTA), optionally in mixture with aliphatic polyesters or polyvinyl alcohols in different ratios. Polybutylene succinate (PBS), polybutylene succinate adipate in blend with various starches in different ratios are also suitable such as e.g. Polybutylene succinate in mixture with thermoplastic starch, alkylene oxide modified starches in combination with hydrolyzed polyvinyl alcohol.

In yet another embodiment of the invention it is preferred that the coating comprises polymers selected from or even that all polymers of the coating are selected from the group consisting of cellulose based polymers and biodegradable polymers, preferably from the group consisting of any of the cellulose based polymers and biodegradable polymers described herein above in this section. Thus, the coating may for example comprise a mixture of PLA and ethylcellulose. In one embodiment the coating even consists of PLA, ethyl cellulose, one or more plasticizers (such as any of the plasticizers described herein below) and one or more UV stabilisers (such as any of the UV stabilisers described herein below).

*UV stabiliser*

Radiation from sunlight can accelerate the degradation of plastics, such as the coating according to the invention. The packaging material to protect the pharmaceutical compositions (e.g. tablets) from direct sunlight may not be enough protection.

Especially for a coating with high concentration of biodegradable polymers, it can be relevant to add UV-stabilizers to the compositions, due to many unsaturated functional groups (eg. carbonyl groups). UV-stabilizers could e.g. be titanium dioxide, metal complexes with sulfurcontaining groups, hindered amine light stabilisers (HALS), benzophenones, benzotriazoles.Titanium dioxide is already widely used in pharmaceutical preparations as pigment and is considered non toxic.

*Plasticizer*

In addition to above mentioned polymers, the coating may comprise one or more additional components. Thus, the coating may comprise at least one selected from the group consisting of

i) polymers which are soluble or dispersible in water,
ii) plasticizers, and
iii) fillers

In a preferred embodiment the polymers, which are soluble or dispersible in water, are cellulose derivatives, which are soluble or dispersible in water. Thus, the coating material may comprise one or more plasticizers, preferably, any of the plasticizers described herein above in the section "pharmaceutically acceptable excipients" and/or any of the plasticizers described below. Preferably, the coating material comprises one or more of the following plasticizers: Cetostearyl alcohol, castor oil, dibutyl sebacate, polyethylene oxides and/or Poloxamer; however other plasticizers may be contemplated to provide desired material properties.

Other suitable plasticizers may be selected from the group consisting of mono- and diacetylated monoglycerides, diacetylated monoglycerides, acetylated hydrogenated cottonseed glyceride, glyceryl cocoate, Polyethylene glycols or

polyethylene oxides (e.g. with a molecular weight of about 1,000-500,000 daltons), dipropylene glycol salicylate glycerin, fatty acids and esters, phthalate esters, phosphate esters, amides, diocyl phthalate, phthalyl glycolate, mineral oils, hydrogenated vegetable oils, vegetable oils, acetylated hydrogenated soybean oil glycerides, Castor oil, acetyl tributyl citrate, acetyl triethyl citrate, methyl abietate, nitrobenzene, carbon disulfide, β-naphtyl salicylate, sorbitol, sorbitol glyceryl tricitrate, fatty alcohols, cetostearyl alcohol, cetyl alcohol, stearyl alcohol, oleyl alcohol, myristyl alcohol, sucrose octaacetate, alfa-tocopheryl polyethylene glycol succinate (TPGS), tocopheryl derivative, diacetylated monoglycerides, diethylene glycol monostearate, ethylene glycol monostearate, glyceryl monooleate, glyceryl monostearate, propylene glycol monostearate, macrogol esters, macrogol stearate 400, macrogol stearate 2000, polyoxyethylene 50 stearate, macrogol ethers, cetomacrogol 1000, lauromacrogols, nonoxinols, octocinols, tyloxapol, poloxamers, polyvinyl alcohols, polysorbate 20, polysorbate 40, polysorbate 60, polysorbate 65, polysorbate 80, polysorbate 85, sorbitan monolaurate, sorbitan monooleate, sorbitan monopalmitate, sorbitan monostearate, sorbitan sesquioleate, sorbitan trioleate, sorbitan tristearate and sucrose esters, amyl oleate, butyl oleate, butyl stearate, diethylene glycol monolaurate, glycerol tributyrate, Flexol B-400, monomeric polyethylene ester, Piccolastic A-5, Piccalastic A-25, Clorafin 40, acetyl tributyl citrate, acetyl triethyl citrate, benzyl benzoate, butoxyethyl stearate, butyl and glycol esters of fatty acids, butyl diglycol carbonate, butyl ricinoleate, butyl phthalyl butyl glycolate, camphor, dibutyl sebacate, dibutyl tartrate, diphenyl oxide, glycerine, HB-40, hydrogenated methyl ester of rosin, methoxyethyl oleate, monoamylphthalate, Nevillac 10, Paracril 26, technical hydroabietyl alcohol, triethylene glycol dipelargonate, solid aliphatic alcohols and mixtures thereof.

In a preferred embodiment, the coating is made of a material, wherein the concentration of plasticizer is from 0 to 30% w/w.

Accordingly it is preferred that the coating comprises or even consists of one or more plasticizer(s) and one or more polymer(s).

Furthermore, the coating may comprise sweetening agents, flavouring agents and/or colouring agents, which may be any of the sweetening agents, flavouring agents and/or colouring agents described herein above in the section "pharmaceutically acceptable excipients".

The coating may be made of a material comprising one polymer, and wherein the concentration of the polymer is from 5 to *100%* w/w.

The coating may be made of a material comprising a mixture of polymers, and wherein the total concentration of polymers is from 70 to 100% w/w.

Preferably, the coating comprises at least 50% w/w, more preferably at least 60% w/w, yet more preferably at least 70% w/w, even more preferably at least 80% w/w in total of polymers substantially insoluble in water as described herein above.

Thus, in preferred embodiments, wherein the coating comprises cellulose derivatives (such as ethyl cellulose), then the coating preferably comprises at least 50% w/w, more preferably at least 60% w/w, yet more preferably at least 70% w/w, even more preferably at least 80% w/w, such as at least 85% w/w, for example 87% w/w cellulose derivative (such as ethyl cellulose).

In a preferred embodiment the coating comprises at the most 19% w/w, more preferably at the most 15% w/w, such as at the most 12% w/w, for example 12% w/w plasticizer (such as cetostearyl alcohol).

Thus, in preferred embodiments, wherein the coating comprises biodegradable polymers (such as polylactic acid), then the coating preferably comprises at least 50% w/w, more preferably at least 60% w/w, yet more preferably at least 70% w/w, even more preferably at least 80% w/w, such as at least 85% w/w, for example 86% w/w biodegradable polymers (such as polylactic acid).

In a preferred embodiment the coating comprises at the most 20% w/w, more preferably at the most 17% w/w, such as at the most 15% w/w, for example 14% w/w plasticizer (polyethylene oxides 200,000 daltons).

## Outer coat

In some cases the pharmaceutical composition of the present invention may also comprise an outer coat that fully covers the composition, i.e. the matrix and the coating. Said outer coat may be selected from the group consisting of task masking coats, coats with aqueous moisture barriers and/or oxidative barriers to improve the stability of the composition, and cosmetic coats e.g. a coat containing colouring agents, sweetening agents and/or flavouring agents in order to provide an elegant and palatable tablet and/or to easy distinguishable dose strengths. Especially, it is preferred to coat compositions having different strength with outer coats of different colours so that the different dose strengths are easily distinguished. Preferably, the outer coat is easily soluble in aqueous media in order to provide that the matrix becomes in contact with the surrounding aqueous media via the openings in the coating immediately after administration.

## Pharmaceutical compositions

Pharmaceutical compositions according to the present invention preferably comprises an active drug selected

from the group consisting of morphine, oxycodone, hydrocodone, hydromorphone, norhydrocordone, oxymorphone, noroxycodone, morphine-6-glucuronode and pharmaceutically acceptable salt thereof, such as morphine sulphate, morphine sulphate pentahydrate, oxycodone hydrochloride and hydrocodone bitartrate, at least one polyglycol selected from the group consisting of polyethyleneglycol and polyethylene oxide and any mixtures thereof, a coat material selected from the group consisting of ethyl cellulose, polylactic acid, polycaprolactone, polyhydroxy butyrate and polyethylene oxide and any mixtures thereof, a plasticizer selected from the group consisting of poloxamer, polyethylene oxide, cetostearyl alcohol, castor oil and dibutyl sebacate and any mixtures thereof, and a filler, which is titanium dioxide.

Pharmaceutical compositions according to the present invention preferably comprises an active drug selected from the group consisting of morphine, oxycodone, hydrocodone, hydromorphone, norhydrocordone, oxymorphone, noroxycodone, morphine-6-glucuronode and pharmaceutically acceptable salt thereof, such as morphine sulphate, morphine sulphate pentahydrate, oxycodone hydrochloride and hydrocodone bitartrate, at least one polyglycol selected from the group consisting of polyethyleneglycol and polyethylene oxide and any mixtures thereof, at least one plasticizer which is poloxamer, at least one stabilizer selected from the group consisting of mannitol, butylated hydroxytoluene and Vitamin E Polyethylene Glycol Succinate, Eudragit L, Eudragit RL, Eudragit RS, Eudragit E, Eudragit S, and at least one gelling agent selected from the group consisting of carrageenan and hydroxypropylmethylcellulose, a coat material selected from the group consisting of ethyl cellulose, polylactic acid, polycaprolactone and polyethylene oxide and any mixtures thereof, a plasticizer selected from the group consisting of polyethylene oxide and cetostearyl alcohol and any mixtures thereof and a filler, which is titanium dioxide.

In cases where the pharmaceutical composition also comprises an outer coat, the pharmaceutical compoisition according to the present invention comprises an active drug selected from the group consisting of morphine, oxycodone, hydrocodone, hydromorphone, norhydrocordone, oxymorphone, noroxycodone, morphine-6-glucuronode and pharmaceutically acceptable salt thereof, such as morphine sulphate, morphine sulphate pentahydrate, oxycodone hydrochloride and hydrocodone bitartrate, at least one polyglycol selected from the group consisting of polyethyleneglycol and polyethylene oxide and any mixtures thereof, a coat material selected from the group consisting of ethyl cellulose, polylactic acid, polycaprolactone, polyhydroxy butyrate and polyethylene oxide, and any mixtures thereof, a plasticizer selected from the group consisting of poloxamer, polyethylene oxide, cetostearyl alcohol, castor oil and dibutyl sebacate and any mixtures thereof, a filler, which is titanium dioxide, and an outer coat selected from the group consisting of task masking coats, coats with aqueous moisture barriers and/or oxidative barriers, cosmetic coats, and any mixtures thereof.

In a very preferred embodiment the pharmaceutical composition comprises morphine sulphate as the active drug, a mixture of polyethylene oxide 200,000 and polyethylene oxide 300,000 as polyglycol, poloxamer as plasticizer, mannitol as stabilizer, a mixture of carrageenan and hydroxypropylmethylcellulose as gelling agent, butylated hydroxytoluene as antioxidant and a mixture of polylactic acid and polyethylene oxide as the coating.

In another very preferred embodiment the pharmaceutical composition comprises morphine sulphate as the active drug, polyethylene oxide 300,000 as polyglycol, poloxamer as plasticizer, a mixture of mannitol and butylated hydroxytoluene as stabilizer and a mixture of ethylcellulose, cetostearyl alcohol and titanium dioxide as the coating.

In another very preferred embodiment the pharmaceutical composition comprises morphine sulphate as the active drug, polyethylene oxide 200,000 as polyglycol, a mixture of mannitol and Vitamin E Polyethylene Glycol Succinate as stabilizer and a mixture of ethylcellulose, cetostearyl alcohol and titanium dioxide as the coating.

## Administration

The pharmaceutical composition according to the invention is preferably designed for oral administration. More preferably, oral intake is by swallowing one or more intact units of the pharmaceutical composition. In one embodiment the pharmaceutical composition is prepared in dosage units, such that a daily dosage of the active drug substance is comprised within one unit. The pharmaceutical composition may in a preferred embodiment be in the form of tablets and thus even more preferably each tablet comprises one daily dosage of the active drug substance.

Furthermore, the pharmaceutical composition according to the invention is preferably prepared for continuous administration once daily. Interestingly, the pharmaceutical compositions according to the invention are effective for at least 24 hours after intake. In particular, in embodiments of the invention, wherein the pharmaceutical composition are for treatment of pain, then the pharmaceutical compositions relieve or ameliorate pain for at least 24 hours after intake.

The pharmaceutical composition according to the invention is preferably prepared for continuous administration, and accordingly, the composition is prepared for repeated administration once daily. More preferably, the continuous administration is administration once daily for several days, preferably at least 3 days, more preferably at least 4 days, even more preferably at least 5 days, yet more preferably at least 6 days, even more preferably at least 7 days, for example at least 9 days, such as at least 11 days, for example for at least 14 days, such as for at least 30 days. Continuous administration is preferably at least administration for a sufficient number of days to arrive at steady state in the individual to whom the pharmaceutical composition of the invention is being administered.

The pharmaceutical composition of the invention is prepared for administration of a given daily dosage. The

daily dosage will be dependent on the individual to whom the pharmaceutical composition of the invention is being administered and the active drug substance.

In general, the daily dosage is in the range of 1 to 1000 mg, such as in the range of 10 to 1000 mg, for example in the range of 30 to 1000 mg, such as in the range of 1 to 750 mg, for example in the range of 1 to 500 mg, such as in the range of 1 to 250 mg, preferably in the range of 15 to 500 mg, more preferably in the range of 15 to 240 mg of said active drug substance.

In particular, when the active drug substance is an opioid, and more particular when the active drug substance is morphine or a pharmaceutically acceptable salt thereof, then the daily dosage is in the range of 1 to 1000 mg, such as in the range of 10 to 1000 mg, for example in the range of 15 to 1000 mg, such as in the range of 1 to 750 mg, for example in the range of 1 to 500 mg, such as in the range of 1 to 250 mg, preferably in the range of 15 to 500 mg, more preferably in the range of 15 to 240 mg, for example in the range of 15 to 200 mg, such as in the range og 30 to 200 mg, for example 15, 30, 45, 60, 75, 90, 100, 120, 140, 160, 180 or 200 mg.

In particular, when the active drug substance is an opioid, and more particular when the active drug substance is oxycodone or a pharmaceutically acceptable salt thereof, then the daily dosage is in the range of 1 to 1000 mg, such as in the range of 10 to 1000 mg, for example in the range of 30 to 1000 mg, such as in the range of 10 to 500 mg, for example in the range of 10 to 250 mg, such as in the range of 10 to 200 mg, for example in the range of 10 to 50, preferably in the range of 10 to 500 mg, more preferably in the range of 10 to 160 mg, even more preferred in the range of 10 to 100 mg, such as in the range of 10 to 80 mg, for example in the range of 20 to 80 mg, such as in the range of 40 to 80 mg, preferably in the range of 30 to 50 mg, such as for example 10, 20, 30, 40, 50, 60, 70 80, 90 or 100 mg.

In particular, when the active drug substance is an opioid, and more particular when the active drug substance is hydrocodone or a pharmaceutically acceptable salt thereof, then the daily dosage is in the range of 1 to 1000 mg, such as in the range of 10 to 1000 mg, for example in the range of 15 to 1000 mg, such as in the range of 1 to 750 mg, for example in the range of 1 to 500 mg, such as in the range of 1 to 250 mg, for example in the range of 1 to 100 mg, such as in the range of 1 to 30 mg, preferably in the range of 10 to 500 mg, more preferably in the range of 10 to 200 mg, such as in the range of 10 to 160 mg, for example in the range of 10 to 30 mg, more preferably in the range of 20 to 160 mg, such as in the range of 20 to 80mg, for example 10, 20, 30, 40, 50, 60, 70, 80, 100, 120, 140 or 160 mg.

In particular, when the active drug substance is an opioid, and more particular when the active drug substance is hydromorphone or a pharmaceutically acceptable salt thereof, then the daily dosage is in the range of 1 to 1000 mg, such as in the range of 1 to 500 mg, for example in the range of 1 to 250 mg, such as in the range of 1 to 100 mg, preferably in the range of 2 to 250 mg, more preferably in the range of 2 to 100 mg, for example in the range of 4 to 100 mg, such as in the range of 4 to 80 mg, preferably in the range of 4 to 64 mg, for example, 2, 4, 6, 8, 10, 12, 14, 16, 18, 20, 24, 28, 32, 40, 48, 56, 64, 72 or 80 mg.

Above-mentioned daily dosages are in particular relevant when the individual in need of treatment is a human being, such as an adult human being.

## Individuals in need of treatment

The pharmaceutical composition of the invention is prepared for administration to an individual in need thereof. Said individual is preferably a mammal, more preferably a human being.

Preferably, the pharmaceutical composition is for continuous treatment of pain and accordingly, the individual in need of treatment is an individual suffering from pain, preferably an individual suffering from pain for a prolonged period of time requiring continuous treatment, wherein continuous treatment is as described in this section above.

In embodiments of the invention, wherein the active drug substance is an opioid, such as morphine or pharmaceutically acceptable salts thereof, then the pharmaceutical compositions are suitable for treatment of moderate to severe pain such as severe pain.

Examples of individuals, who may benefit from treatment with the pharmaceutical compositions according to the invention, includes for example the following:

The individual may be an individual suffering from chronic pain, such as moderate to severe chronic pain.

The individual may be an individual suffering from cancer and the pharmaceutical composition may be useful for continuous treatment of pain or even moderate to severe pain, such as severe pain in an individual suffering from cancer.

The individual may also be an individual who has suffered a moderate to severe injury.

The individual may be an individual suffering from pain associated with surgical conditions, such as a pre-surgical individual (an individual in need of surgery) or a post surgical individual (an individual who has undergone surgery).

The individual may also be an individual suffering from or having suffered from a myocardial infarction, sickle cell crises, kidney stone or severe back pain.

**Steady state**

It is an important feature of the pharmaceutical compositions according to the present invention that they are useful for continuous treatment upon once daily administrations. Accordingly, once steady state has been arrived at, Cmin is sufficiently high to ensure continuous efficacy over the entire administration period. Furthermore, it is a significant advantage of the pharmaceutical compositions of the invention that once steady state has been arrived at, then the ratio between Cmax and Cmin is small.

An individual is in steady state with regard to a particular active drug substance when the plasma concentration level after one dosing is the same within the standard deviation as the plasma concentration level after the following dosing. Thus, for pharmaceutical compositions for once daily administration then at steady state $AUC_{(0-24h)d}=AUC_{(0-24h)d+1}$+/- the standard deviation, and $Cmax_{(0-24h)d}=Cmax_{(0-24h)d+1}$ +/- the standard deviation, where d is day. AUC refers to the "area under the curve" and is a measurement for the plasma concentration over the entire dosing interval.

Unfortunately, studies using single dosages are not useful for determining whether a medicament is useful for continous treatment in steady state individual. The present invention however demonstrates that the pharmaceutical compositions disclosed therein are useful for treatment in steady state individuals and that a useful ratio between Cmax and Cmin can be achieved using these compositions.

Thus, upon continuous administration of the pharmaceutical compositions comprising an active drug substance (preferably an analgesic, such as an opioid, for example morphine) according to the invention, then steady state C24 in respect of the active drug substance is preferably at least 20%, more preferably at least 25%, even more preferably at least 30%, such as at least 40%, for example at least 50% of steady state Cmax in respect of the active drug substance. In specific embodiments steady state C24 in respect of the active drug substance may even be at least 60%, such as at least 70%, for example at least 80%, such as at least 90%, for example at least 95% of steady state Cmax in respect of the active drug substance.Thus steady state C24 in respect of the active drug substance may preferably be in the range 30 to 95%, such as in the range of 30 to 90%, preferably in the range of 30 to 80%, such as in the range of 30 to 70%, preferably in the range of 30 to 60% of steady state Cmax in respect of the active drug substance. Aforementioned percentages are in particular relevant for pharmaceutical compositions according to the invention prepared for once daily administration.

Preferably, C24 and Cmax is determined as an average in at least 10, for example in at least 18 steady state individuals.

It is furthermore preferred that upon continuous administration of the pharmaceutical compositions comprising an active drug substance (preferably an analgesic, such as an opioid, for example morphine) according to the invention, then steady state Cmin is at least 20%, preferably at least 25% of steady state Cmax. Thus steady state Cmin may be in the range of 20 to 75%, such as in the range of 20 to 60%, for example in the range of 20 to 50%, preferably in the range of 25 to 75%, such as in the range of 25 to 60%, for example in the range of 25 to 50% of steady state Cmax. In some embodiments of the invention the difference between steady state Cmin and steady state Cmax may be even smaller and steady state Cmin may thus be at least 30%, such as at least 40%, for example at least 50%, such as at least 60%, for example at least 70%, such as at least 80% of steady state Cmax. Aforementioned percentages are in particular relevant for pharmaceutical compositions according to the invention prepared for once daily administration.

Preferably, Cmin and Cmax is determined as an average in at least 10, for example in at least 18 steady state individuals.

It is also another advantage of the compositions of the invention that the difference between trough and Cmax is relatively small.

Accordingly, upon continuous administration of the pharmaceutical compositions comprising an active drug substance (preferably an analgesic, such as an opioid, for example morphine) according to the invention, then steady state trough is preferably at least 20%, more preferably at least 25%, then steady state trough is at least 20%, more preferably at least 25%, even more preferably at least 30%, such as at least 40%, for at least 50% of steady state Cmax. In specific embodiments steady state trough may even be at least 60%, such as at least 70%, for example at least 80%, such as at least 90%, for example at least 95% of steady state Cmax .Thus steady state trough may preferably be in the range 30 to 95%, such as in the range of 30 to 90%, preferably in the range of 30 to 80%, such as in the range of 30 to 70%, preferably in the range of 30 to 60% of steady state Cmax. Aforementioned percentages are in particular relevant for pharmaceutical compositions according to the invention prepared for once daily administration.

Preferably, trough and Cmax is determined as an average in at least 10, for example in at least 18 steady state individuals.

Cmin is preferably not reached too early, thus preferably Cmin is reached no earlier than half way through a given dosing interval in a steady state individual. Thus, for the pharmaceutical compositions of the invention comprising an active drug substance (preferably an analgesic, such as an opioid, for example morphine), which are prepared for once daily administration, then Cmin is preferably reached no earlier than 10 hours after, preferably no earlier than 12

hours after last administration to a steady state individual. Preferably, the time when Cmin is reached is determined as an average of at least 10, such as at least 18 steady state individuals.

The plasma concentration usually reaches 50% of steady state Cmax twice after each administration. Once at the time when plasma concentration is rising soon after administration (referred to 1st point) and once when plasma concentration is decreasing after the peak concentration has been reached (referred to as 2nd point).

For continuous once daily administration of an active drug substance comprising an active drug substance (preferably an analgesic, such as an opioid, for example morphine), then preferably the 2nd point where the plasma concentration reaches 50% of steady state Cmax should not be reached too fast. Additionally, fast onset may be an advantage and this would be supported by a profile with a short time to the 1st point where the plasma concentration reaches 50% of of steady state Cmax. Theoretically, If the steady state profile becomes really protracted/blunted, the 50% of steady state Cmax may never be reached and another marker i.e. 75% of Cmax could be chosen to define the period for the passing the first and the second time.

Interestingly, the pharmaceutical compositions according to invention are able to both provide a profile with a very high steady state minimum plasma concentration (Cmin) with a long time between the first and second time of passing a fraction of Cmax (i.e. 50 or 75%) and as compared to other controlled release formulations. Thus, the 2nd point where a concentration of 50% of steady state Cmax is reached is preferably no earlier than 3.5 hours, more preferably no earlier than 4 hours, no earlier than 4.5 hours, no earlier than 5 hours, more preferably no earlier than 6 hours, such as no earlier than 6.5 hours after last administration of the pharmaceutical compositions according to the invention to a steady state individual. For example the 2nd point where a concentration of 50% of steady state Cmax is reached is preferably in the range of 3.5 to 24 hours, more preferably in the range of 4 to 24 hours, such as in the range of 4.5 to 24 hours, such as in the range of 5 to 24 hours, more preferably in the range of 6 to 24 hours, such as in the range of 6.5 to 24 hours, for example in the range of 4 to 20 hours, such as in the range of 4 to 16 hours, for example in the range of 4 to 13.5 hours after last administration of the pharmaceutical compositions according to the invention to a steady state individual. Preferably, the time to 50% of Cmax is determined as an average of at least 10, such as at least 18 steady state individuals.

For continuous once daily administration of an active drug substance (preferably an analgesic, such as an opioid, for example morphine), then preferably the 1st point where the plasma concentration reaches 50% of steady state Cmax is not later than 4 hours, for example no later than 2 hour, for example in the range of 0.25 to 3 hours after last administration of the pharmaceutical compositions according to the invention to a steady state individual. Preferably, the time to 50% of Cmax is determined as an average of at least 10, such as at least 18 steady state individuals.

Therefore, the larger time window between the two 50% of steady state Cmax the better if a consistent plasma concentration is the goal. This time frame should preferably not be less than 6 h for example not less than 10 h, for example in the range of 8-24h. Similarly, for 75% of steady state Cmax the window between the two points should be not less than 1 h for example not less than 2 hours, for example in the range of 1-24h such as in the range of 4-16h.

Tmax of the pharmaceutical compositions according to the invention comprising an active drug substance (preferably an analgesic, such as an opioid, for example morphine) is preferably in the range of 2 to 5 hours, for example in the range of 3 to 4 hours after last administration to a steady state individual. Preferably, Tmax is determined as an average of at least 10, such as at least 18 steady state individuals.

It is furthermore preferred that upon once daily administration of the pharmaceutical compositions comprising 30 mg of an active drug substance (preferably an analgesic, such as an opioid, for example morphine) according to the invention, then steady state $AUC_{0-24h}$ in respect of the active drug substance is preferably at least 200 nmol*h/L, more preferably at least 300 nmol*h/L, for example at least 350 nmol*h/L, such as in the range of 200 to 1000 nmol*h/L, for example in the range of 300 to 1000 nmol*h/L, such as in the range of 300 to 500 nmol*h/L, for example in the range of 300 to 400 nmol*h/L.

It is also preferred that upon once daily administration of the pharmaceutical compositions comprising 100 mg of an active drug substance (preferably an analgesic, such as an opioid, for example morphine) according to the invention, then steady state $AUC_{0-24h}$ in respect of the active drug substance is preferably at least 400 nmol*h/L, more preferably at least 600 nmol*h/L, even more preferably at least 800 nmol*h/L, yet more preferably at least 1000 nmol*h/L, for example at least 1200 nmol*h/L, such as at least 1400 nmol*h/L, for example in the range of 1000 to 3000 nmol*h/L, such as in the range of 1000 to 2000 nmol*h/L, for example in the range of 1200 to 2000 nmol*h/L, such as in the range of 1200 to 1600 nmol*h/L, for example in the range of 1400 to 1600 nmol*h/L.

Preferably, $AUC_{0-24h}$ is determined as an average in at least 10, for example in at least 18 steady state individuals.

It is another important feature of the present invention that the Protraction index lies as closely to 1 as possible, because such value of the index denotes that the pharmacological profile is very flat. In such cases the plasma concentration is substantially constant throughout the 24 hour dosing interval, i.e. throughout the period between two consecutive administrations. Hence, the Protraction index is preferably at least 0.2, such as at least 0.25, more preferred at least 0.30, for example at least 0.35, such as at least 0.40, for example at least 0.45, for example at least 0.50, such as at least 0.55, for example at least 0.60, such as at least 0.70, for example at least 0.80.

**Clinical efficacy**

It is of great importance that controlled release formulations of active drug substances, releases the active ingredient in a manner that the desired clinical efficacy is achieved. For treatment of pain, it is important, that the pain is relieved continuously throughout the treatment period. Thus, pharmaceutical compositions, which are administered only once daily should be capable of relieving pain for at least 24 hours.

Unfortunately, the efficacy of a particular active drug substance frequently can not be predicted from in vitro studies. Even if studies regarding in vivo serum concentrations are available, the efficacy can often not be predicted - in particular efficacy in relation to treatment of pain. In particular for opioids MEAC is unknown (see more details in the "Background" section herein above) and may also differ from person to person and accordingly the minimal efficacious Cmin can not be predicted.

However, it is an important feature of the pharmaceutical compositions according to the invention, that they are efficacious in a clinical setting. Thus, the pharmaceutical compositions according to the invention comprising analgesics are efficient in relieving pain for at least 24 hours after last administration, even upon once daily continuous administration.

Because the perception of pain may vary amongst individuals, efficacy in treatment of pain should be determined as an average in a number of individuals, preferably as an average in at least 30 individuals, such as an average of in the range of 30 to 1000 individuals.

Thus, preferably the average pain intensity determined in at least 30 steady state individuals determined approximately 24 hours, preferably 24 hours after last administration immediately prior to next administration is at the most 4, preferably at the most 3 on a scale from 0 to 10, where 0 is equivalent to "no pain" and 10 is equivalent to "pain as bad as you can imagine", wherein said steady state individuals are continuously treated once daily with a pharmaceutical composition comprising an analgesic (preferably an opioid such as morphine or pharmaceutically acceptable salts thereof) according to the invention. In this context "approximately" preferably means 23.5 to 24 hours.

It is also preferred that the average pain intensity determined in at least 30 steady state individuals from approximately 12 hours to approximately 24 hours, preferably from 12 hours to 24 hours after last administration is at the most 4, preferably at the most 3, on a scale from 0 to 10, where 0 is equivalent to "no pain" and 10 is equivalent to "pain as bad as you can imagine", wherein said steady state individuals are continuously treated once daily with a pharmaceutical composition comprising an analgesic (preferably an opioid such as morphine or pharmaceutically acceptable salts thereof) according to the invention. In this context "approximately" preferably means 11.5 to 12 and 23.5 to 24 hours, respectively.

Said steady state individuals are preferably individuals, who would have experienced pain in the absence of the treatment, for example patients suffering from cancer. Pain intensity is preferably determined based on an evaluation of the steady state individuals. It is preferably performed as described herein below in Example 1.

Break Through Pain (BTP) is pain, which is not alleviated by a patients normal pain suppression management. Frequently, Break Through pain comes on suddenly and for a short period of time. It is common in cancer patients who commonly have a background level of pain controlled by administration of analgesics, but the pain periodically "breaks through" the medication.

Upon once daily continuous administration of the pharmaceutical compositions according to the invention the number of Break Through Pain episodes is very low. Thus, the average number of daily Break Through Pain episodes determined in at least 30 steady state individuals is preferably at the most 2, preferably at the most 1. Preferably, the average number of daily Break Through Pain episodes are determined over a number days, for example over in the range of 3 to 30 days, such as over in the range of 5 to 20 days, for example for in the range of 7 to 14 days.

**Drug abuse**

Abuse of active drug substances and in particular opioids constitutes a problem. The pharmaceutical compositions according to the present invention have a reduced risk for drug abuse and/or alcohol induced dose dumping.

In order to ensure that the use of a composition mitigates alcohol induced dose dumping, the ratio (R50) between t50% w/w (40% w/w ethanol in medium 1) and t50% w/w (medium 1) is 1 or more. t50% w/w (medium 1) denotes the time it takes to release 50% w/w of the active drug substance from the pharmaceutical composition in an in vitro dissolution test according to USP 30, NF 25, (711), Apparatus 2, paddle employing water optionally buffered to a specific pH as dissolution medium (medium 1), and t50% w/w(40% w/w ethanol in medium 1) denotes the time it takes to release 50% w/w of the active drug substance from the pharmaceutical composition in an in vitro dissolution test according to USP 30, NF 25, (711), Apparatus 2, paddle employing 40% w/w ethanol in medium 1 as dissolution medium.

In a specific embodiment, the ratio R50 is at the most 5 such as at the most 4, at the most 3 or at the most 2. Notably, the ratio R50 is from 1 to 1.5 such as, e.g., from 1 to 1.4, from 1 to 1.3, from 1 to 1.2, from 1 to 1.1, from 1 to 1.05, or about 1.

The same may also apply for ratios determined e.g. when 25%, 30%, 40%, 60%, 70%, 80%, 90% and/or 95% w/w has been released, the conditions being as described above.

The likelihood of a composition being subject to drug abuse may for example be tested by the below four different tests:

1. Crushing test
2. Melting test
3. Extraction/dissolving
4. Injection test

In the crushing test, the composition is subjected to crushing using a hammer, electronic tools (e.g. coffee mill) or an apparatus designed to measure the hardness of an oral dosage form. A suitable apparatus is specified in Ph. Eur. If the composition disintegrates into particles, then it may be possible to dissolve or suspend these particles and use them for abuse purposes. Moreover, if it is possible to disintegrate (crunch) the composition, then it is possible to use the powder for snorting or sniffing and in this way abuse the composition, however, if it is not possible to crush the composition in this test, then there will be no particles to use for such abuse purposes. Thus, preferably, the compositions of the invention can not be crushed into particles.

In the melting test, the composition is subjected to heating e.g. on a spoon or by exposure to microwave induced heating. If the composition is suitable for abuse purposes, the composition should become so liquid that it is possible to inject it without being too hot. However, if this is not the case, the composition is not suitable for abuse purposes. Accordingly, the compositions of the invention preferably do not become so liquid that it is possible to inject them upon heating.

In the extraction test it is tested whether it is possible to extract the active drug substance from the composition by means of normally available organic solvents. If it is possible to dissolve the composition then if may be possible to misuse the drug e.g. by injection. On the contrary, if it is not possible, then it is likely that the composition cannot be misused. Thus, preferably, it is not possible to dissolve the pharmaceutical compositions of the invention faster than in a dissolution medium which may be either ethanol or phosphate buffer pH 6.8 or hydrochloride pH 1.2.

In the Injection test, the composition is dissolved in 2 ml water possibly after extensive heating. The preparation is put into a syringe and the time of passage through a fitted 0.5 mm needle is measured upon a weight applied to the syringe of 3 kg. The time of passage of the pharmaceutical compositions according to the invention is preferably at least 10 sec. more preferably at least 15 sec. yet more preferably at least 20 sec.

The pharmaceutical compositions of the invention are preferably of such nature that it is basically impossible to abuse either by chewing, crushing, melting, extraction, dissolving or similar. Furthermore, the pharmaceutical composition exhibits decreased (or essentially the same) release rate in alcohol containing media as compared to a purely aqueous media. The release rate from the pharmaceutical composition will depend on several parameters such as in an unlimited list: solubility of the polyglycol, active drug substance and the excipients, the wetability of the composition, the diffusion of water into the composition, the enthalpy of melting and enthalpy of solubilization, and the disentanglement rate of the polyglycol during dissolution. Controlled release dosage forms are used to extend the release from the dosage form for an extended period of time. In the present context the term "controlled release" is used to designate a release a desired rate during a predetermined release period.

## Examples

The invention is further illustrated in the following non-limiting examples.

Egalet® morphine Formulation A, B1 and B2 are designed to provide pain relief for up to 24 hours and requires dosing only once or twice per day, in general only once per day. The advantages of this formulation include better patient compliance, and smaller fluctuations in plasma concentrations, possibly resulting in attenuation of morphine-related AEs. In addition, the formulation is designed to be tamper-resistant and not subject to alcohol-induced dose-dumping; two problems with misuse of opioids intended for treatment of chronic pain which are currently gaining a lot of focus. Egalet® morphine Formulation A, B1 and B2 are, therefore, a relevant and important new formulation of morphine for oral use.

| Components % w/w | Morphine Formulation | | | Reference |
|---|---|---|---|---|
| | Matrix | | | |
| Formulation | A | B1 | B2 | |
| Morphine sulphate pentahydrate | 16 | 51.5 | 36.0 | *USP/NF* |
| Polyethylene Oxide 200k | 71.4 | | 22.7 | USP/NF |
| Polyethylene Oxide 300k | | 32.0 | 16.0 | USP/NF |
| Poloxamer 188 | | 13.4 | 12.2 | USP/NF |
| Poloxamer 407 | | | | USP/NF |
| Eudragit L100-55 | | | | USP/NF |
| Vitamin E polyethylene glycol succinate (TPGS) | 2.6 | | | |
| Mannitol | 10.0 | 3.0 | 3.0 | USP/NF |
| Carrageenan | | | 5.0 | USP/NF |
| Hydropropylmethylcellulose 100k | | | 5.0 | USP/NF |
| Butylated Hydroxytoluene | | 0.1 | 0.1 | USP/NF |
| | Coating | | | |
| Polylactic acid | | | 86 | DMF21817/12983 |
| PEO 200k | | | 14 | USP/NF |
| Ethyl cellulose | 87.0 | 87.0 | | USP/NF |
| Cetostearyl alcohol | 12.0 | 12.0 | | USP/NF |
| Titanium dioxide | 1.0 | 1.0 | | USP/NF |

Composition of Egalet® morphine 30 mg, Formulation A

| **Components** | **Amount per tablet** (% w/w) | **Amount per 30 mg tablet** (mg) | **Reference** |
|---|---|---|---|
| ***Matrix composition*** | **100** | **188 mg** | |
| Morphine sulphate pentahydrate | 16.0 | 30 | Ph.Eur. |
| Polyethylene oxide 200.000 NF | 71.4 | 134.2 | USP/NF |
| Vitamin E Polyethylene Glycol Succinate (TPGS) | 2.6 | 4.9 | USP/NF |
| Mannitol | 10.0 | 18.8 | Ph.Eur.; USP |
| ***Coating*** | **100** | **139 mg** | |
| Ethyl cellulose | 87.0 | 120.9 | Ph.Eur. |
| Cetostearyl alcohol | 12.0 | 16.7 | Ph.Eur. |
| Titanium dioxide | 0.74 | 1.4 | Ph.Eur.; USP/NF |
| **Total** | | **327 mg** | |

**Example 1A**

**A randomized, double-blind, two-way cross-over efficacy and safety study of once daily dosing of Egalet® morphine compared to twice daily dosing of MST Continus in the treatment of cancer pain.**

The study (herein also referred to as MP-EG-002) included a run-in phase of up to 3 weeks duration, a treatment phase of 4 weeks duration (2 weeks on each treatment), and a follow-up period of up to 1 week duration.

The study was conducted at 8 sites in Poland and Lithuania. Each site received Ethics Committee approval before recruiting patients for the study, and all patients gave their written informed consent to participate before any study related procedures were performed.

MST Continus 15 mg tablets were used for dose finding and stabilization during the run-in phase. Throughout the study patients received immediate release morphine sulfate (Actiskenan 5, 10 or 20 mg capsules, Bristol-Myers

Squibb, France) for use as needed for treatment of Break Through Pain (BTP) episodes.

The study medication, Egalet® morphine Formulation A 30 mg tablets once daily or MST Continus 15 mg tablets twice daily (Napp Pharmaceuticals, UK), was blinded by over-encapsulating with standard, caramel brown gelatin capsules. To maintain the blind with the different dosing regimens, patients received placebo capsules (capsules with filler only) for the evening dose during the Egalet® morphine Formulation A treatment period. During both treatment periods the patients received the number of capsules required to reach the individual total daily dose up to a maximum TDD of 240 mg/day corresponding to 8 capsules morning and evening.

Adult patients with a stable strong opioid use equipotent to 30-240 mg oral morphine sulfate daily for a minimum of 2 weeks prior to entering the run-in phase were eligible for the study. The patient should have opioid-sensitive pain caused by active cancer, be able to comprehend and communicate effectively with the Investigator and staff, and to comply with all of the trial requirements.

Patients were excluded from the study if they had a life expectancy less than 2 months, if they had received chemotherapy or radiotherapy less than 4 weeks prior to entering the run-in phase, or if there was planned radiotherapy or chemotherapy or other non-pharmacological treatments with potential analgesic effect during the study. Patients were also excluded from the study if they had any concurrent condition or required concomitant medication that could interfere with the study assessments or might represent a safety hazard to the patient.

It was planned to randomize up to 60 cancer patients in order for 36 patients to complete both treatment periods, and enrolment was stopped when the target of 36 completed patients was reached.

Upon screening eligible patients started a run-in period during which each patient was individually titrated to a dose of MST Continus providing an acceptable level of pain intensity and number of BTP episodes (≤ 4 per day). If patients prior to the study were taking a strong opioid other than morphine sulfate, the appropriate dose of MST Continus was calculated from an equivalency table provided in the study protocol. The total daily dose of MST Continus during run-in was evenly distributed between morning and evening doses and the dose found to be appropriate during run-in served as the fixed dose of study medication during both treatment periods.

Treatment of BTP episodes with rescue medication, immediate release morphine, was initiated during the run-in period according to Table 1. If BTP episodes were not satisfactory treated with the rescue dose strength in the table, the dose could be increased based on Investigator's discretion and two (or more) rescue doses could be taken simultaneously per BTP episode. If the number of BTP episodes exceeded 4 per day, the patient's basal dose of MST Continus was increased and the run-in period continued until the patient was stable on the new level of CR morphine sulfate. The minimum duration of the run-in period was 3 days. If patients were not stabilized after 3 weeks of run-in they were discontinued from the study.

When patients were stable they were randomized (in blocks of 4) to a treatment sequence (Egalet® morphine Formulation A followed by MST Continus or MST Continus followed by Egalet® morphine Formulation A).

The duration of each treatment period was 2 weeks, and as only data from the last week of each treatment period was used for analysis a washout period between the two treatments was not deemed necessary. A study visit was performed at the last day of each treatment period. During this visit a blood sample was taken before the scheduled morning dose of study medication for analysis of morphine and metabolites, patients rated their impression of the treatment received during the past treatment period, and level of sedation was rated hourly from approximately 8:00 (before morning dose of study medication) until approximately 22:00 (2 hours after evening dose of study medication). At the study visit after the last treatment period, global preference was also rated by the patients.

Within one week of completing study treatment a follow-up visit was performed for final safety evaluations and return of any remaining medication and diaries used.

Patients were provided with paper diaries for the run-in and treatment periods which were completed on a daily basis during the study. Diaries were provided in the local languages, and all translations were verified by a back-translation. The diaries captured information about intake of run-in or blinded study medication, rescue medication, ratings of pain intensity, interference of pain with sleep and daily level of sedation. In addition, new or changing doses of concomitant medication and adverse experiences were entered in the diaries. Patients were instructed to take the study medication daily at approximately 8:00 and 20:00 with 12 hours in between morning and evening doses, wherein Egalet® morphine Formulation A was taken at 8:00 in the morning and placebo at 20:00 in the evening, and to perform the diary ratings just before each scheduled morning and evening dose of study medication. For the hourly sedation ratings and ratings of impression of treatment and global preference, which were performed at the end of each treatment period, separate visit diaries were used.

Blood samples for analysis of morphine and metabolites were collected before morning dose of study medication on the last day of each treatment period. After collection, samples were centrifuged and plasma separated and stored at -20 degrees Celsius until analysis. Plasma concentrations of morphine, M-3-G and M-6-G were measured using a validated LC-MS/MS analysis

**Efficacy**

One endpoint of the study was the average daily number of rescue medication doses used the last 7 days of each treatment period (exclusive the visit day) as recorded by the patients in the diaries.
Another endpoint was the number of BTP episodes and use of rescue medication in mg/day and in percent of TD were derived from the diary data for number of rescue medication doses.
The current pain intensity and the average, least and worst pain intensity for the previous 12 hours was rated on an 11-point Numeric Rating Scale (NRS) (0=no pain to 10=pain as bad as you can imagine) in the patient diaries every morning and evening immediately prior to intake of next dosage.
Pain interfering with sleep was rated every morning on a 5-point Verbal Rating Scale (VRS) (0=not affecting sleep, 1=little effect on sleep, 2=moderate effect on sleep, 3=much effect on sleep, 4= very much affect on sleep).
End of treatment drug rating was performed by the patients at the end of each treatment period. Patients recorded their overall impression of the study medication taken during the past 2 weeks on a verbal rating scale (1=poor, 2=fair, 3=good, 4=very good, 5=excellent).
At the end of the study the patient gave their global assessment of the study treatment by indicating which treatment period they preferred (preference for period 1, preference for period 2 or no preference).
In addition, at the end of each treatment period a blood sample was collected before the morning dose of study medication for analysis of trough levels of morphine, morphine-3-glucuronide (M-3-G), and morphine-6-glucuronide (M-6-G).

**Safety**

Every evening the patients rated the average daily level of sedation on an 11-point NRS (0=completely alert to 10=impossible to stay awake). In addition, on the last day of each treatment period, the patients rated the level of sedation on an 11-point NRS every hour from just before morning dose of study medication until 2 hours after evening dose.
Adverse experiences, ECGs, physical examinations, vital signs as well as hematology, biochemistry, coagulation and urine analyses were performed to assess safety of the study medications.

**Data analysis**

The primary method of analysis for the efficacy variables was analysis of covariance (ANCOVA) for cross-over design. The ANCOVA model included effects for site, sequence, treatment, period and the random effects of patients within sequences. The baseline value (last 3 days of run-in period) was incorporated into the model as a covariate, if available. All effects were tested and model-based 95% Confidence Intervals (CIs) were calculated for the mean difference between treatments.
When the distributional assumptions required for the ANCOVA model were not met, a non-parametric approach was used. The Mann-Whitney test was applied for the analysis of sequence, treatment and period effects, and in addition, the Lehmann-Hodges non-parametric 95% CI was calculated for the median difference between treatments.
Diary data from the last 7 days of each treatment period (exclusive the visit day on the last day of the treatment period) were used for the analyses of rescue use, BTP episodes, pain intensity and interference of pain with sleep.
For the analysis of use of rescue medication, one dose was defined according to table 1. If a patient's dose of rescue medication was different from that in the table, the number of doses taken was calculated according to the table; for example if a patient with a total daily dose of 60 mg morphine sulfate had a 5 mg recue dose replaced with a 10 mg dose (whether as a 10 mg capsule or two 5 mg capsules) the 10 mg dose was handled as two doses.
For analysis of BTP episodes, the number of BTP episodes was calculated as the number of times at least one capsule of rescue medication was taken. If an additional dose of rescue medication was taken within two hours of the first dose, it was considered as one episode of BTP.
End-of-dose concentrations of morphine, M-3-G and M-6-G was analyzed using ANOVA model for log-transformed data. The ratio of means and 95% CI was estimated for each analyte. As the total daily dose varied between patients, concentration values dose-normalized to a total daily dose of 100 mg/day were also calculated.
All safety data was presented as descriptive statistics only, with the exemption of the sedation ratings which were analyzed as described above for the efficacy endpoints. As the study was explorative the sample size of 36 patients was not based on a power calculation. The sample size was, however, deemed to be sufficient to obtain adequate characterization of the efficacy parameters based on similar sample sizes used in published cross-over studies of cancer pain treatment with different controlled release opioids.

**Results**

*Patient disposition*

41 patients were randomized. Three patients discontinued the study before the end of the first treatment period and without contributing any efficacy data; two withdraw due to Adverse Events (AEs) and one at patient's own request. The Full Analysis Set (FAS) therefore included 38 patients: 19 in each treatment sequence group. Of these, two patients discontinued the study after completing the first treatment period due to progression of the underlying cancer disease. Patients with major protocol deviations, i.e. deviations that could potentially impact any of the efficacy outcomes of the study, were excluded form the Per Protocol (PP) analysis set for the study period in which the deviation occurred. Five patients had major protocol deviations during the study, and hence the PP set contained data from 34 patients. The final assessment of patients included in the PP analysis set was made before the blind was broken. Thirty patients had a blood sample collected at the end of each treatment period for the analysis of morphine and metabolites.

*Study medication*

The daily dose levels ranged from 30 to 210 mg/day. No patients received the maximum dose level of 240 mg. Based on individual drug accountability of study medication all patients were deemed fully compliant with use of study medication. Diary completion during the study was close to 100%. Compliance with use of rescue medication was assessed based on a cross-check between diary entries and accountability of rescue medication. One patient had uncertain compliance (>20% discrepancy between accountability and diary) and was excluded from the PP set for this reason. All other patients were deemed to be compliant with use of rescue medication.

*Demographics*

More than half of the patients were male (63.2%), and the mean age ranged from 42 to 81 years. All patients were Caucasian. The most common type of cancer causing pain was lung cancer (23.7%), followed by breast (15.8%) and rectal (10.5%) cancer. All patients were taking concomitant medications. The most common concomitant medications were natural opium alkaloids (34 [89.5%] patients) followed by proton pump inhibitors (14 [36.8%] patients), propionic acid derivatives (13 [34.2%] patients) and benzodiazepine derivatives (13 [34.2%] patients).

*Efficacy*

For the primary efficacy variable, average number of doses of rescue medication per day no difference between treatments was found. The median number rescue doses per day was 1.0 (range 0.0 - 4.6) during the Egalet® morphine Formulation A treatment period and 0.7 (range 0 - 6.9) during the MST Continus treatment period. The estimated difference between medians (Egalet® morphine Formulation A- MST Continus) was 0.07 doses per day (95% CI -0.21 ; 0.29) and was not statistically significant (p=0.76).

The median number of BTP episodes, as identified by use of rescue medication, was 0.7 episodes/day in both treatment periods and no difference between the two treatment periods was found (Table 1). The median amount of rescue medication as a percentage of the TDD per day was slightly lower in the Egalet® morphine Formulation A treatment period than in the MST Continus treatment period, while the median amount of rescue medication in mg/day was slightly higher during Egalet® morphine Formulation A treatment than during MST Continus treatment (Table 1). The estimated median difference between treatments in the amount of rescue medication as a percentage of the TDD at 4-hourly intervals was zero at every time interval except for 0 - 4 hours post morning dose where the estimated median difference (Egalet® morphine Formulation A- MST Continus) was -0.04% (95% CI -1.19 ; 0.60). The estimated median difference between treatments in the amount of rescue medication in mg/day at 4-hourly intervals was zero at every time interval. During treatment with Egalet® morphine Formulation A the number of patients experiencing BTP requiring rescue medication during the final hours of the 24-hour treatment period was small, and similar to the number of patients experiencing BTP during the same hours while taking MST Continus twice daily. During the final 4-hour interval (20 hours post morning dose until next morning dose), nine subjects in each treatment group experienced BTP requiring rescue medication on at least one occasion during the 7 days of observation. The corresponding numbers for the previous 4-hour interval (16 to 20 hours post morning dose) was 8 patients in the Egalet® morphine Formulation A group and 11 patients in the MST Continus group, and hence there was no trend to increasing numbers of patients with BTP in the final interval compared to the previous interval.

No differences were found for any of the pain intensity scores (Table 2). All average pain intensity scores were in the range of 1.3 to 4.4 for Egalet® morphine Formulation A, and 1.3 to 4.3 for MST Continus (for minimum and maximum pain intensity, respectively). All of the differences between the treatments were small and statistically non-significant.

Mean or median (as appropriate) differences between the treatments ranged from 0.00 to 0.18 (Egalet® morphine Formulation A - MST Continus) with a maximum width of the 95% CI of approximately 0.65. The current pain intensity at the morning evaluation, 24 hours after the most recent exposure to Egalet® morphine Formulation A *versus* 12 hours after the most recent exposure to MST Continus, showed similar low median values (2.3 and 2.0, respectively) with an estimated median difference of zero (CI -0.36 ; 0.29). This indicates that both Egalet® morphine Formulation A and MST Continus provided effective pain control at the end of their respective dosing intervals.

The median interference of pain with sleep was 1.0 (little effect on sleep) in both treatment periods. During treatment with Egalet® morphine Formulation A the range was 0.0 - 3.4 and during MST Continus treatment the range was 0.0 - 2.3. The estimated median difference between the treatments (Eaglet® morphine - MST Continus) was 0.07 (95% CI -0.07; 0.21) and was not statistically significant (p=0.36).

Median assessment of the drugs by patients was 3 (good) for both treatments. The ranges were 1 (poor) to 4 (very good) for the Egalet® morphine Formulation A treatment and 1 (poor) to 5 (excellent) for the MST Continus treatment (Table 3). The estimated median treatment difference (Egalet® morphine Formulation A- MST Continus) was 0.00 (95% CI -0.50 ; 0.50, p=1.0).

Neither treatment was clearly preferred. Thirteen (37.1 %) patients expressed a preference for Egalet® morphine Formulation A, 14 (40.0%) patients expressed a preference for MST Continus, 8 (22.9%) patients had no preference, and 1 value was missing. A binominal test performed among patients who preferred either Egalet® morphine Formulation A or MST Continus showed no difference when the proportion of patients preferring Egalet® morphine Formulation A was compared to 50% preference for Egalet®morphine Formulation A.

Trough morphine, M-3-G and M-6-G concentrations were measured from 30 patients who had a blood sample collected in the morning of the last day in each treatment period (Table 4). There were no differences between the treatments in the geometric mean concentrations of morphine and its metabolites at trough plasma levels 24 hours after the last dose of Egalet® morphine Formulation A and 12 hours after the last dose of MST Continus. For the trough concentrations dose normalized to a TDD of 100 mg/day and for the sub-set of patients not taking any rescue medication within 4 hours prior to blood sampling the results were comparable.

*Safety*

There was no evidence to indicate a difference in the incidence, nature or severity of AEs between the treatments groups of MST Continus and Egalet® morphine Formulation A. The pattern of the overall and treatment related AEs did not differ between treatments, and was what a clinician would reasonably expect in a population with advanced malignancy and chronic use of opioids. Importantly, there were no deaths or other severe adverse effects during this study.

**Conclusion**

One challenge for a once daily product as Egalet® morphine Formulation A is to provide pain relief for the entire 24-hour period. End-of-dose failure would result in reduced efficacy in the hours preceding the next scheduled dose of medication, and a number of measurements were employed in this study in order to investigate the pharmacological efficacy of Egalet® morphine Formulation A during and no end-of-dose failure was detected at the end of the 24-hour dosage interval for Egalet® morphine Formulation A. Less frequent dosing normally results in better patient compliance with opioid analgesics. In addition, Egalet® morphine Formulation A is designed to be resistant to alcohol-induced dose-dumping and tampering. The study demonstrated that the efficacy of Egalet® morphine Formulation A dosed once daily is comparable to another commonly used CR morphine product, MST Continus, dosed twice daily in cancer patients with chronic pain. Based on this Egalet® morphine Formulation A is considered a highly relevant new formulation of morphine sulfate.

Dosing with Egalet® morphine Formulation A at intervals of 24 hours was therapeutically equivalent to MST Continus dosed at intervals of 12 hours as shown by similar use of rescue medication, pain intensity and number of BTP episodes during the two treatment periods, and supported by substantially identical steady state trough concentration of morphine for the two treatments.

**Tables:**

**Table 1**

Use of rescue medication and number of BTP episodes (n=37)

| Endpoint | Egalet® morphine Formulation A | MST Continus® | Difference (95% CI) |
|---|---|---|---|
| ***Use of rescue medication*** | | | |
| Average daily number of rescue medication doses[1] Median (min-max) | 1.0 (0.0-4.6) | 0.7 (0.0-6.9) | 0.07 (-0.21 ; 0.29) p=0.76 |
| Average daily amount of rescue medication as % of TDD[2] Median (min-max) | 8.3 (0.0-52.4) | 9.5 (0.0-57.1) | 0.57 (-2.38 ; 3.17) p=0.74 |
| Average daily amount of rescue medication in mg Median (min-max) | 9.3 (0.0-45.7) | 7.9 (0.0-68.6) | 0.00 (-2.86 ; 2.14) p=0.99 |
| ***BTP episodes*** | | | |
| Average daily number of BTP episodes[3] Median (min-max) | 0.7 (0.0-4.4) | 0.7 (0.0-3.4) | 0.00 (-0.21 ; 0.21) p=0.90 |

' One dose of rescue medication was approximately 10% of TDD

[2] TDD: Total Daily Dose. The individual dose of ER morphine which was established during run-in period of the study and remained fixed for both treatment periods

[3] BTP episodes: Break Through Pain episodes. A BTP episode was defined of number of times a rescue dose was taken. Two or more rescue doses within 2 hours were considered as one BTP episode.

**Table 2**

Pain intensity ratings[1] (n=37)

| | Egalet® morphine Formulation A | MST Continus® | Difference (95% CI) |
|---|---|---|---|
| *Pain intensity rated in the morning* | | | |
| Average pain intensity during past 12 hours Mean (SD) | 2.5 (1.5) | 2.4 (1.5) | 0.10 (-0.13; 0.32) p=0.39 |
| Minimum pain intensity during past 12 hours Median (min-max) | 1.3 (0.0-3.9) | 1.3 (0.0-3.7) | 0.07 (-0.07 ; 0.21) p=0.50 |
| Maximum pain intensity during past 12 hours Mean (SD) | 4.1 (2.3) | 4.0 (2.0) | 0.15 (-0.18 ; 0.47) p=0.37 |
| Current pain intensity Median (min-max) | 2.3 (0.0-6.7) | 2.0 (0.0-5.9) | 0.00 (-0.36 ; 0.29) p=0.96 |
| *Pain intensity rated in the evening* | | | |
| Average pain intensity during past 12 hours Mean (SD) | 2.6 (1.5) | 2.6 (1.5) | 0.04 (-0.17 ; 0.25) p=0.68 |
| Minimum pain intensity during past 12 hours Mean (SD) | 1.6 (1.2) | 1.5 (1.1) | 0.06 (-0.15 ; 0.27) p=0.55 |
| Maximum pain intensity during past 12 hours Mean (SD) | 4.4 (2.2) | 4.3 (2.2) | 0.18 (-0.15 ; 0.50) p=0.27 |

(continued)

| | | | |
|---|---|---|---|
| *Pain intensity rated in the evening* | | | |
| Current pain intensity | | | 0.12 (-0.10 ; 0.35) |
| Mean (SD) | 2.5 (1.8) | 2.4 (1.7) | p= 0.27 |

Pain intensity was rated on an 11-point Numeric Rating Scale (0=no pain to 10=pain as bad as you can imagine) every morning and evening. Results are averages over last 7 days of each treatment period (exclusive the visit day).

**Table 3**

Patients' overall impression of treatment[1] (n=37)

| | Egalet® morphine Formulation A | MST Continus® |
|---|---|---|
| Poor | 4 | 3 |
| Fair | 12 | 12 |
| Good | 15 | 17 |
| Very good | 6 | 4 |
| Excellent | 0 | 1 |

Ratings of patient's overall impression of treatment were made on the last day of each treatment period p=1.0, Hodges-Lehmann estimate for difference between medians

**Table 4**

Morphine, morphine-3-glucuronide, and morphine-6-glucoronide concentrations at the end of the 24-hour dosing interval (n=30)

| Analyte | **Egalet® morphine** Formulation A | **MST Continus** | **Egalet® morphine** Formulation A / **MST Continus** |
|---|---|---|---|
| | Geometric mean[1] (range) (nmol/L) | | Ratio of means[2] (95% CI) |
| Morphine | 37.4 (<0.75 ; 219.6) | 37.1 (<0.75 ; 257.2) | 0.99 (0.74 ; 1.33) |
| M-3-G | 1120.8 (<5 ; 9838.0) | 1061.6 (<5 ; 6488.0) | 1.04 (0.83 ; 1.30) |
| M-6-G | 159.8 (<1 ; 1489.0) | 148.3 (<1 ; 1077.0) | 1.06 (0.82 ; 1.37) |

[1] In the calculation of geometric mean, values that were below the quantification limit were replaced with half of the limit. The quantification limit is 0.75 nmol/L for morphine, 5 nmol/L for M-3-G and 1 nmol/L for M-6-G.
[2] Ratio of means is based least square mean difference estimated from 2x2 ANOVA model for log-transformed data.

**Example 1 B**

**Pharmacokinetic sampling addendum to study MP-EG-002**

**Objectives:**

The objectives of this sub-study were to evaluate the correlation between the intensity of hourly sedation as reported by the patients (Example 1A) and the plasma concentration of morphine and its metabolites, and to assess the steady-state pharmacokinetic (PK) parameters for Egalet® morphine Formulation A compared with MST Continus.

**Methodology:** Patients at selected centers who participated in study MP-EG-002 (see Example 1A) were invited to participate in this sub-study. Patients who were enrolled in the main protocol MP-EG-002, and who gave separate informed consent for the sub-study, had blood samples taken for analysis of morphine and the morphine metabolites morphine-3-glucuronide (M-3-G) and morphine-6-glucuronide (M-6-G) at Visit 3 and Visit 4. These blood samples were additional to all of the procedures in study MP-EG-002.

Patients were instructed to fast from 22.00 of the evening before the visits. On the visit day the patients arrived at the clinic at approximately 07.00, before the scheduled morning dose of study medication. Patients then had a Venflon intravenous cannula inserted. Before 07.45, the patients were given a standardized breakfast, which was served and eaten at the clinic, the patients completed the morning ratings in the diary for the treatment phase, and the pre-dose

ratings in the visit diary.

The morning dose of the study medication was taken at approximately 08.00, and 7 mL blood samples for analysis of plasma levels of morphine and its metabolites were drawn at hours 0 (immediately pre-dose), 1, 2, 3, 5, 8, 12, 13, 14, 15 and 24.

Water was allowed ad libitum from 2 hours after dosing but caffeine-containing drinks were disallowed throughout the sampling period. Patients were given a standardized meal at 12.00, a non-standardized evening meal at 18.00, and snacks at 15.00 and 21.00.

**Number of patients**

A total of 12 patients were included in the steady state PK sub-study and in the steady state PK full analysis set. Two patients (1 patient in each treatment sequence group) discontinued from the study after completing the first treatment period. One other patient was excluded from the PK population because of a protocol violation in dosing.

**Efficacy**

The relationships between plasma concentrations of morphine, M-3-G and M-6-G, and sedation were examined by estimating the linear regression coefficient using three different covariates: concentration, change per hour, and two biggest adjacent changes in concentration. No statistically significant relationship was found in any of these analyses, i.e., the regression coefficients and CIs were approximately zero in all cases. Overall, there was a statistically significant relationship between the absorption rate of morphine and sedation. The slope was 0.008 (95%CI 0.001 ; 0.015), i.e., the steeper the increase in concentration, the greater the increase in sedation. For the metabolites the relationship was also positive, but was statistically non-significant. No differences between treatments were found.

**Steady state PK Results**

Plasma morphine PK parameters were similar after the Egalet® morphine Formulation A once daily administration compared with MST Continus (Table 5). AUCO-24, and Cmax were slightly lower after Egalet® morphine Formulation A than after MST Continus, whereas Cmin was practically the same after both treatments. However, the ratios of means all lay within 0.90 and 1.25, demonstrating similar exposure after Egalet® morphine Formulation A dosed once daily and MST Continus dosed twice daily. Tmax occurred approximately 1 hour later after Egalet® morphine Formulation A compared with MST Continus. Fluctuation and swing were almost identical after both treatments. When AUCO-24, Cmax and Cmin were dose normalized, the ratios of means Egalet® morphine Formulation A/MST Continus were only slightly lower than non dose-normalized values. Meaning that the dose normalized $C_{max}$ of MST Continus is about twice the $C_{max}$ of Egalet® morphine.

**Table 5**
**Summary of Steady state Morphine Pharmacokinetic Parameters PK Population**

| Pharmacokinetic parameter | **Egalet® morphine Formulation A** (n=10)[1] | **MST Continus** (n=11)[1] | **Ratio of means[2]** (Egalet® morphine Formulation A-MST Continus) (95% CI) |
|---|---|---|---|
| | Geometric mean (range) | Geometric mean (range) | |
| $AUC_{0-24}$ (nmol*h/L) | 1282.3 | 1354.3 | 0.90 |
| $C_{max}$ (nmol/L) | 98.7 | 102.5 | 0.90 |
| $C_{min}$ [3] (nmol/L) | 26.1 | 26.6 | 0.96 |
| $C_{24}$ (nmol/L) | 41,76 | -- | -- |
| $T_{max}$[4] (h) (mdian (range)) | 4 | 3 | 1.17 |
| $t_{1/2}$ (h) (n=6) | 23.0 | not calculated | not calculated |
| $k_e$ (1/h) (n=6); | 0.03 | not calculated | not calculated |
| Fluctuation | 1.33 | 1.33 | 0.98 |
| Swing | 2.72 | 2.82 | 1.02 |
| *Dose normalized parameters* | | | |
| $AUC_{0-24}$ (nmol*h/L) | 1402.5 | 1700.7 | 0.86 |
| $C_{max}$ (nmol/L) | 108.0 | 128.7 | 0.86 |

(continued)

| | | | |
|---|---|---|---|
| *Dose normalized parameters* | | | |
| $C_{min}$ (nmol/L) | 28.5 | 33.4 | 0.90 |
| $C_{24}$ (nmol/L) | 45,7 | -- | -- |
| [1] Blood samples at hours 15 and 24 only collected from 4 patients<br>[2] ANCOVA - log transformation applied<br>[3] $C_{min}$ = minimum concentration during the 0-24 h interval<br>[4] for MST Continus $t_{max}$ derived based on the 0-12 h interval | | | |

Plasma concentrations of M-3-G and M-6-G were higher over the first 14 hours after Egalet® morphine Formulation A compared with after MST Continus, and the maximum value was reached slightly later than after the morning dose of MST Continus. However, plasma concentrations of M-3-G and M-6-G were similar after both formulations at the end of the 24-hour treatment period. There were no meaningful differences between treatments in the steady state PK parameters for M-3-G and M-6-G.

**Conclusions:**

The steady state PK parameters $AUC_{0\text{-}24h}$, Cmax, Cmin, fluctuation and swing for morphine,M-3-G and M-6-G were similar after Egalet® morphine Formulation A dosed once daily and MST Continus dosed twice daily. This means that with half the number of doses Egalet® morphine was able to keep the same range of plasma concentrations as MST Continus

Tmax for morphine, M-3-G and M-6-G occurred between zero and two hours later after Egalet® morphine Formulation A compared with MST Continus.

There was no statistically significant relationship between plasma concentrations of morphine, M-3-G and M-6-G, and sedation.

Overall, there was a statistically significant positive correlation between the absorption rate of morphine (but not M-3-G and M-6-G) and sedation.

**Example 2**

**A single-period, multiple-dose, single-centre, phase I trial evaluating the steady-state pharmacokinetic profile of Egalet® morphine Formulation A 30 mg controlled extended release dosage unit in healthy volunteers using naltrexone**

**blockade**

This study is also referred to as MP-EG-003 herein.

One objective was to evaluate the steady-state pharmacokinetic profile of Egalet® morphine Formulation A 30 mg controlled release dosage unit administered once daily for 10 consecutive days under fasting conditions.

Another objective was to evaluate the safety and tolerability of multiple doses of Egalet® morphine Formulation A 30 mg extended release dosage units in healthy subjects.

This was a single-centre, non-comparative, multiple-dose, phase I trial, performed under fasting conditions. Subjects were confined to the Clinical Research Facility from at least 14 hours before the first study drug administration (evening of Day -1, when the first administration of co-medication [naltrexone] was given) and were discharged from the clinic on Day 11, after the 36.0-hour post-dose blood draw. Subjects came back for all subsequent blood draws on Days 12, 13, 14, and 15. Naltrexone is an opioid receptor antagonist.

Number of subjects enrolled, randomised and completed the study was: 18 (8 females and 10 males).

Subjects had to be healthy, adult non-smokers, aged ≥ 18 and ≤ 55 years; body mass indices ≥ 18.0 and < 30.0 kg/m2. All subjects had to be in compliance with the inclusion and exclusion criteria described in the protocol and were judged eligible for enrolment in this study based on medical and medication histories, demographic data (including sex, age, race, body weight [kg], height [cm], and BMI [kg/m2]), vital signs measurements (including pulse oxymetry), a 12-lead ECG, a physical examination, a urine drug screen, an alcohol breath test, a pregnancy test, and clinical laboratory tests (hematology, biochemistry, urinalysis, HIV, hepatitis C [HCV] antibodies, and hepatitis B surface antigen [HBSAg]).

**Table 6**

| Treatment | | |
|---|---|---|
| | Study Drug | Co-medication |
| Name | Egalet® morphine Formulation A | Naltrexone hydrochloride (Revia®) |
| Unit dose | 30 mg | 50 mg |
| Regimen | single dose of 1 x 30 mg controlled release dosage unit by oral administration for 10 consecutive days (Days 1 to 10) | single dose of 1 x 50 mg film coated tablet by oral administration on the following days: Day -1, 12 hours before the first morphine administration; Days 1 through 10: 1 hour before each morphine administration; Day 11: approximately 24 hours after the last morphine administration (immediately prior to next dose) |

The following pharmacokinetic parameters were calculated for morphine: AUC $_{0\text{-}24h}$, $T_{max}$, steady state $C_{max}$, steady state $C_{min}$, PTF, $AUC_{0\text{-}inf}$, $T_{½\ el}$, and $K_{el}$.

The pharmacokinetic parameters listed above were also calculated for morphine-3-glucuronide and morphine-6-glucuronide.

Additional pharmacokinetic parameters were MRT, HVD and $T_{75\%Cmax}$ (for morphine only)

Also the Protraction index was calculated for each individual with regard to the morphine concentration profile.

Safety: Adverse events, vitals signs (including pulse oxymetry) and ECG measurements, and standard laboratory evaluations.

A single arm, non-comparative study, formal statistical analyses were not performed for the PK endpoints. Endpoints are summarized and represented by N, arithmetic and geometric mean, median, standard deviation, minimum and maximum.

The attainment of steady state was assessed based on log-transformed pre-dose plasma concentrations of morphine recorded on Days 4 to 10. In a repeated measures model with subject and day as factors, Day 10 concentration was compared to Days 4 to 9, respectively. The first day with a non-significant difference to Day 10 is considered steady state. Mean and individual curves of untransformed pre-dose plasma concentrations versus time (Days 4 to 11) were produced. The steady state analysis was repeated exploratively including time since physical activity and time since last bowel movement as covariates in the model.

Although it was not planned to include the Day 11 24h plasma concentrations in the analyses, an exploratory analysis (as above) was planned to be performed to check if this analysis would add any information to the steady-state data.

**Results**

Figure 2 shows the mean steady state morphine plasma concentration versus time curve (0-24h).

Steady state was obtained already after 4 days of administration of the Egalet® morphine Formulation A 30mg extended release dosage unit. 4 days was the earliest investigated time point and thus steady state may possibly have been reached even earlier. Both the mean and individual concentration vs. time profiles seem to demonstrate that the Egalet® morphine Formulation A dosage unit offers at least a twice daily and preferably also a once daily treatment for most subjects, by providing steady morphine concentration throughout the 24 hours for most subjects. For some subjects, however, the morphine concentration decreases and reaches a relatively low level at the 24h time point. The co-administration of naltrexone may have marginally influenced the PK-profiles and some of the PK endpoints. No severe, significant, or serious adverse events were reported during the study.

**TABLE 7**
**Pharmacokinetics - morphine**

| | |
|---|---|
| Enrolled subjects | 18 |
| $AUC_{0\text{-}24h}$(nmol*h/L) | |
| Geom. mean (CV) | 353 (43%) |
| Min, Max | 176 - 795 |

(continued)

**Pharmacokinetics - morphine**

| | |
|---|---|
| TMAX(h) | |
| Geom. mean (CV) | 1.54 (77%) |
| Min, Max | 0.50 - 5.05 |
| CMAX (SS)(nmol/L) | |
| Geom. mean (CV) | 31.6 (47%) |
| Min, Max | 14.1 - 59.3 |
| CMIN (SS)(nmol/L) | |
| Geom. mean (CV) | 6.9 (64%) |
| Min, Max | 1.6 - 23.4 |
| C24 (SS)(nmol/L) | |
| Geom. mean (CV) | 12,52 (62%) |
| Min, Max | 2,19-27,3 |
| SS denotes steady state | |

Also the Protraction index was determined, and the data below in Table 8 are derived from the steady state profiles obtained in the individuals, which participated in this study.

**TABLE 8**

**Protraction index**

| | $(AUC_{0-24h} / 24h) / Cmax$ |
|---|---|
| | 0.36 |
| | 0.34 |
| | 0.39 |
| | 0.36 |
| | 0.39 |
| | 0.34 |
| | 0.30 |
| | 0.35 |
| | 0.35 |
| | 0.36 |
| | 0.39 |
| | 0.35 |
| | 0.40 |
| | 0.29 |
| | 0.42 |
| | 0.39 |
| | 0.47 |
| Mean | 0.37 |
| Min | 0.29 |
| Max | 0.47 |

**Example 3**

**A SINGLE-CENTRE, SINGLE-DOSE, RANDOMISED, OPEN-LABEL, 5-WAY CROSSOVER, DOSE-LINEARITY STUDY OF EGALET® MORPHINE 30, 60, 100 AND 200 mg CONTROLLED-RELEASE DOSAGE UNITS IN HEALTHY VOLUNTEERS USING NALTREXONE BLOCKADE UNDER FASTING CONDITIONS**

This study is also referred to as MP-EG-005 herein.

**Objectives**

The primary objective of this study was to evaluate dose-linearity of the four strengths of Egalet® Morphine controlled-release dosage units of Formulation B1.

**Rationale**

1) Optimisation of the dosage regimen for patients suffering from moderate-to-severe pain by offering a controlled-release formulation for dosing only once a day that can be developed in high strengths.
2) Demonstration of dose proportionality between 4 different geometries of the Egalet® morphine corresponding to 30, 60, 100 and 200mg morphine sulfate.

**Design**

This was a single centre, open-label, single-dose, randomised, 5-way crossover, comparative bioavailability study, performed under fasting conditions to evaluate dose-linearity of the four strengths of Egalet® Morphine of Formulation B1.
Evaluation of safety and tolerability to controlled-release dosage units included adverse events (i.e., seriousness, severity, and relationship), vital signs and clinical laboratory parameters.

**Sample collection**

Measurements of morphine plasma concentrations and secondary analysis with morphine-3-glucuronide and morphine-6-glucuronide plasma concentrations were performed at the following timepoints: pre-dose and 0.333, 0.667, 1.00, 2.00, 3.00, 4.00, 5.00, 6.00, 7.00, 8.00, 10.0, 12.0, 15.0, 18.0, 21.0, 24.0, 30.0, 36.0, and 48.0 hour post-dose.

**Compositions**

Three different formulations with different compositions were tested. The compositions were designated formulation A, B1 and B2. The content of the formulations is described in Table 9 herein below. The compositions were prepared by two component injection molding. All formulations showed the same dissolution properties as tested in an USP 2 apparatus at 50 rpm and pH 6.8 (see figure 3). This indicates that the three compositions most likely will show similar release profiles in-vivo. Two of the formulations were tested in two different tablet shapes: round (formulation A) and elliptical (formulation B1). It was found that the dose was released proportionally to the release area, such that each composition released the complete dose (100%) at the same timepoint.

**Table 9**

| Components | Amount per tablet (% w/w) | | | Function |
|---|---|---|---|---|
| | Form. A | Form. B1 | Form. B2 | |
| Matrix | 100 | 100 | 100 | |
| Morphine sulfate pentahydrate | 16.0 | 51.5 | 36.0 | Active ingredient |
| Polyethylene oxide 200 000 | 71.4 | - | 22.7 | Carrier, release modifier |
| Polyethylene oxide 300 000 | - | 32.0 | 16.0 | Carrier, release modifier |
| Poloxamer 188 | - | 13.4 | 12.2 | Co-carrier, Plasticizer |
| Mannitol | 10.0 | 3.0 | 3.0 | Release modifier and stabilizer |
| Carrageenan | - | - | 5.0 | Gelling agent |

(continued)

| Components | Amount per tablet (% w/w) | | | Function |
|---|---|---|---|---|
| | Form. A | Form. B1 | Form. B2 | |
| Matrix | 100 | 100 | 100 | |
| Hydroxymethylcellulose 100k | - | - | 5.0 | Gelling agent |
| Butylated hydroxytoluene (BHT) | - | 0.1 | 0.1 | Antioxidant, Stabilizer |
| Vitamin E Polyethylene Glycol Succinate (TPGS) | 2.6 | - | - | Stabilizer |
| Coating | 100 | 100 | 100 | |
| Polylactic acid | - | - | 86 | Coat material |
| PEO 200k | - | - | 14 | Plasticizer |
| Ethylcellulose | 87.0 | 87.0 | - | Coat material |
| Cetostearyl alcohol | 12.0 | 12.0 | - | Plasticizer |
| Titanium dioxide | 1.0 | 1.0 | - | Coloring agent, UV stabiliser |

**Treatments**

In each treatment period, subjects were administered a single oral dose of either Egalet® Morphine of Formulation B1 (dosage unit of 30, 60, 100, or 200 mg) or Formulation A (two tablets of 30 mg) controlled-release dosage units on Day 1, in accordance with the subjects' randomization sequence. The treatment periods were separated by a washout of 7 days.

- Treatment A: 1 x 30 mg Egalet® Morphine controlled-release dosage unit of Formulation B1 (08-0140-066).
- Treatment B: 1 x 60 mg Egalet® Morphine controlled-release dosage unit of Formulation B1 (08-0138-066).
- Treatment C: 1 x 100 mg Egalet® Morphine controlled-release dosage unit of Formulation B1 (08-0137-066).
- Treatment D: 1 x 200 mg Egalet® Morphine controlled-release dosage unit of Formulation B1 (08-0139-066).
- Treatment E: 2 x 30 mg Egalet® Morphine controlled-release dosage units of Formulation A (08-0141-066).

To alleviate or avoid opioid side effects that are expected in opioid-naive subjects, naltrexone was administered as a 1 x 50 mg tablet with approximately 120 mL of water approximately 12 hours before morphine administration (Day -1), approximately 1 hour before morphine administration (Day 1), and approximately 24 hours post- morphine administration (Day 2).

**Methodology**

A total of 39 healthy, adult non-smokers signed the study-specific informed consent form and were confined for Period 1; of these subjects, 35 (18 males and 17 females) were enrolled and dosed in the study; 31 of these enrolled subjects completed the study. Prior to entering the trial, subjects completed all screening procedures. Upon arrival at the clinical facility for the confinement (Day -1) and once eligibility had been confirmed, subjects were sequentially allocated a two-digit subject number that corresponded to the randomisation scheme.

All subjects received standardised meals throughout during their confinements, not less than 4 hours post-dose, approximately 9 hours post-dose, and an evening snack approximately 13 hours post-dose. With the exception of the volume administered at the time of the administration of morphine, fluids were not permitted from 1 hour before dosing to 1 hours post-morphine dose, but water was permitted ad libitum at all other times.

A urine drug screen and an alcohol breath test were performed for all subjects upon admission to the clinical unit for each period.

Female subjects of childbearing potential and who had sexual intercourse with a non-sterile male partner were required to use a method of contraception from 14 days prior to study drug administration until 7 days following the last drug administration.

Data were evaluated descriptively only, as defined in the statistical analysis plan (SAP).

**Pharmacokinetic Parameters**

The following PK parameters were calculated and summarised by standard non-compartmental methods for morphine plasma concentrations, morphine-3-glucuronide plasma concentrations, and morphine-6-glucuronide plasma

concentrations. The morphine-3-glucuronide plasma concentrations and morphine-6-glucuronide plasma concentrations were included for supportive information.

1) $AUC_{0-t}$: area under the concentration-time curve from time zero to the last non-zero concentration
2) $AUC_{0-inf}$: area under the concentration-time curve from time zero to infinity (extrapolated)
3) $C_{max}$: maximum observed concentration
4) Residual area: calculated as 100*(1-$AUC_{0-t}$ / $AUC_{0-inf}$).
5) $T_{max}$: time of observed $C_{max}$
6) $T_{½el}$: elimination half-life
7) $K_{el}$: elimination rate constant
8) MRT: mean residence time
9) Proportion of AUC before $T_{max}$

**Pharmacokinetic Methods**

The PK endpoints were calculated individually for each subject and dose based on the plasma concentrations obtained on Days 1-3 (0 - 48h) within each period.

**$AUC_{0-t}$**

The area under the concentration-time-curve from time 0h until the last concentration sample at time 48h, $AUC_{0-t}$., were calculated by the linear trapezoidal method, using the actual sampling time points. If the last blood sample was taken less than 48 hours after drug administration, the 48h values were extrapolated using the terminal elimination rate constant, $K_{el}$ as described below. If the last sample was taken after 48 hours, a 48h value was estimated by interpolation. Intermediate missing values remained missing (equivalent to interpolating between neighbouring points when calculating AUC). Intermediate values below the limit of quantification (LOQ) were assigned a value of LOQ/2, while trailing values below LOQ were assigned a value of zero.

**$AUC_{0-inf}$**

The area under the concentration-time-curve from time 0h until infinity was determined for profiles that did not return to zero within 48 hours. $AUC_{0-inf}$, was calculated as the sum of $AUC_{0-t}$ and $C_t/K_{el}$ where Ct was the last sample above LOQ.

**$T_{max}$ and $C_{max}$**

$T_{max}$ and $C_{max}$ were derived from the samples 0 - 48h after drug administration. Actual sampling time points were used for $T_{max}$.

**Residual area:**

Calculated as 100 * (1-$AUC_{0-t}$ / $AUC_{0-inf}$)

**$T_{1/2\ el}$:**

The elimination half-life $T_{½}$ was found by Ln(2)/$K_{el}$,(for calculation of $K_{el}$ refer to the below)

**$K_{el}$:**

The elimination rate constant, $K_{el}$ was the slope of the terminal part of the log-concentration-time-curve and was found using log-linear regression. The final four plasma concentrations above LOQ were included in the calculation as a minimum. However, the log-linear plots of plasma concentration were inspected and a different selection of data points could have been chosen to ensure that the time period represented the terminal elimination phase. Actual time values were used.

**MRT:**

The mean residence time was calculated as

$$MRT_{0\text{-}inf} = AUMC_{0\text{-}inf} / AUC_{0\text{-}inf},$$

where

$$AUMC_{0\text{-}inf} = AUMC_{0\text{-}t} + t^*C_t/K_{el} + C_t/(K_{el})^2,$$

and where $AUMC_{0\text{-}t}$ was the area under the first moment curve from time 0 until the last valid measurement at the time point t. $C_t$ was the last valid plasma concentration found at this time point, t.

**%$AUC_{0\text{-}Tmax}$**

The proportion of AUC before $T_{max}$ was found by 100 * ($AUC_{0\text{-}Tmax}$/ $AUC_{0\text{-}inf}$)

**Pharmacokinetic Results**

As displayed in Figure 4, below, there was a clear increase in the concentration of morphine with the increase in dosage. The curves of 1 x 60 mg Egalet® Morphine Formulation B1 and 2 x 30 mg Egalet® Morphine Formulation A were very close together, however during the first 8 hours, the plasma concentration of 1 x 60 mg Egalet® Morphine Formulation B1 was slightly higher than that of the 2 x 30 mg Egalet® Morphine Formulation A.

There was a very small bump in the mean profiles at 24 hours. However, this was more pronounced in some of the individual plots and could be a result of a hepatic recirculation or a naltrexone-derived increase in morphine absorption.

Also the metabolites morphine-3-glucuronide and morphine-6-glucuronide concentrations were proportional between strengths.

Individual plasma concentration profiles for each subject showed consistency across profiles for morphine, morphine-3-glucuronide, and morphine-6-glucuronide concentrations within each subject.

For morphine, these relationships are also presented in Table 11, displaying a slightly greater than two-fold increase of the $AUC_{0\text{-}48}$ when the dose was doubled. It was also shown that the $AUC_{0\text{-}48}$ for the 60 mg Egalet® Morphine Formulation B1 was higher than the $AUC_{0\text{-}48}$ for the 2 x 30 mg Egalet® Morphine Formulation A. The results for $C_{max}$ displayed the same pattern as the results for $AUC_{0\text{-}48}$ and the results for $AUC_{0\text{-}48}$ and $C_{max}$ was confirming the patterns displayed by Figure 4. The relationship between dosage and $AUC_{0\text{-}inf}$ was the same as for $AUC_{0\text{-}48}$.

**Table 11 Endpoints for Morphine**

| Treatment | 30 mg Form B1 | 60 mg Form B1 | 100 mg Form B1 | 200 mg Form B1 | 2x30 mg Form A |
|---|---|---|---|---|---|
| AUC(0-48h)(nmol*h/L): | | | | | |
| Mean | 300 | 681 | 1175 | 2437 | 618 |
| Min - Max | 110-535 | 364 - 1127 | 756 - 2189 | 1371-4176 | 203 - 1008 |
| Cmax (nmol): | | | | | |
| Mean | 19 | 43 | 73 | 168 | 35 |
| Min-Max | 8-40 | 23-69 | 38-138 | 71-277 | 16-72 |
| AUC(0-inf)(nmol*h/L): | | | | | |
| Mean | 381 | 823 | 1355 | 2702 | 728 |
| Min - Max | 117 - 1668 | 414 - 2582 | 784 - 2795 | 1483 - 4528 | 209 - 1324 |

(continued)

| | | | | | |
|---|---|---|---|---|---|
| Residual area (Pct.) | | | | | |
| Mean | 13 | 13 | 11 | 9 | 13 |
| Min-Max | 0-74 | 1 - 75 | 2-44 | 0-20 | 1 - 43 |
| Tmax (h): | | | | | |
| Mean | 3 | 3 | 3 | 4 | 4 |
| Min-Max | 1-6 | 1-5 | 1 -10 | 1 -10 | 0-24 |
| T(1/2)(h): | | | | | |
| Mean | 17 | 17 | 14 | 13 | 14 |
| Min-Max | 4-129 | 5-134 | 7-47 | 5-20 | 6-31 |
| Elimination rate (1/h): | | | | | |
| Mean | 0.06 | 0.06 | 0.06 | 0.06 | 0.06 |
| Min - Max | 0.01 - 0.17 | 0.01 - 0.13 | 0.01 - 0.10 | 0.03-0.14 | 0.02-0.12 |
| MRT (h): | | | | | |
| Mean | 27 | 29 | 24 | 21 | 25 |
| Min-Max | 9-178 | 14-186 | 13-61 | 12-29 | 9-49 |
| Proportion AUC(0-Tmax) (Pct.): | | | | | |
| Mean | 12 | 9 | 11 | 15 | 12 |
| Min - Max | 1 - 36 | 1 - 20 | 1 - 28 | 2-33 | 1 - 54 |

For morphine-3-glucuronide and morphine-6-glucuronide plasma concentrations, the relationship between dosage and $AUC_{0-48}$, $C_{max}$, and $AUC_{0-inf}$ was the same as for the morphine plasma concentrations. The pattern of the residual area and the elimination rate for morphine-3-glucuronide and morphine-6-glucuronide concentrations was also similar as to that of morphine. For both morphine-3-glucuronide and morphine-6-glucuronide concentrations, the mean $T_{max}$ was 4 hours.

**Primary PK Analysis (Dose-linearity)**

From the descriptive summaries of $AUC_{0-48}$ and $C_{max}$ in Table 12, it was clear that a dose response relationship was present for $AUC_{0-48}$ and $C_{max}$.

**Table 12** Primary Analysis of Morphine (Dose-Linearity)

| | Coefficient for log-dose | | | 90% Confidence Interval | |
|---|---|---|---|---|---|
| | | Estimate | Std.Err. | Lower | Upper |
| Full PK Data Set: | | | | | |
| AUC(0-48h) (nmol*h/L) | B | 1.1171 | 0.02281 | 1.0792 | 1.1550 |
| AUC(0-inf) (nmol*h/L) | B | 1.0806 | 0,03317 | 1.0225 | 1.1358 |
| Cmax | B | 1.1365 | 0.02297 | 1.0983 | 1.1747 |
| Completers Only: | | | | | |
| AUC(0-48h) (nmol*h/L) | B | 1.1185 | 0.02310 | 1.0801 | 1.1569 |
| AUC(0-inf) (nmol*h/L) | B | 1.0826 | 0.03376 | 1.0265 | 1.1387 |
| Cmax | B | 1.1349 | 0.02310 | 1.0965 | 1.1733 |

The coefficient (beta) for log-dose was estimated in a mixed linear model including period as a fixed effect and subject as a random effect.

The analyses for completers only are regarded as exploratory.

Table 12 presents the analysis of dose-linearity for morphine concentration for $AUC_{0-48}$ and $C_{max}$.

The table showed that dose-linearity could be assumed as the 90% confidence interval for β was fully contained within the interval 0.80- 1.25 for $AUC_{0-48}$, $AUC_{0-inf}$ as well as for $C_{max}$, both for the full PK analysis set and for completers only. The estimates of coefficient for the log-dose, β, for the three parameters ranged from 1.08 to 1.14. This indicated that the bio-availability increased slightly more than proportionally with dose. However, since the confidence intervals were within the regulatory acceptance limits, this slight deviation was not considered clinically important.

The analysis of morphine-3-glucuronide and morphine-6-glucuronide concentrations confirmed the results for the morphine plasma concentration, as all 90% confidence intervals were contained within the interval 0.80-1.25 and all estimates of β were slightly larger than 1.

**Bioequivalence of 1x60 mg Formulation B1 versus 2x30 mg Formulation A**

From Table 11, it was apparent that the mean values for AUC and $C_{max}$ in the 60 mg Egalet® Morphine Formulation B1 treatment group and 2 x 30 mg Egalet® Morphine Formulation A treatment group were similar, but with slightly higher values for the 60 mg Egalet® Morphine Formulation B1 treatment group.

The results of the secondary analysis of morphine are presented in Table 13 and the estimated ratios of means for $AUC_{0-48h}$ and $AUC_{0-inf}$ were 110.2 and 111.6, respectively. The estimated ratio for $C_{max}$ was 121.7. The 90% confidence intervals for $AUC_{0-48h}$ and $AUC_{0-inf}$ lay within the boundaries of 0.80 and 1.25; however the upper limit of the 90% confidence intervals for $C_{max}$ exceeded the 1.25 boundary value. Hence, bioequivalence was not demonstrated. Both $AUC_{0-48h}$ and $C_{max}$ were statistically significantly different from 100 on a 5% level as a minimum. The results were confirmed by the analyses of the completers only and the analysis of subjects with a residual area less than 20%. Moreover, the ratio was statistically significantly different from 100 on a 5% level.

The estimated ratios and associated 90% confidence intervals reflected the results of morphine concentration. However, for the morphine-3-glucuronide concentration, $AUC_{0-inf}$ and $C_{max}$ were statistically significantly different from 100 on a 5% level and the 90% confidence interval for analysis of subjects with a residual area less than 20% was contained within 0.80 - 1.25. It should be noted that the upper boundary of the 90% confidence interval for $C_{max}$ was below the 133% limit, which was the upper limit of a widened acceptance interval of 75-133%, as mentioned in guidelines. The estimated ratios and associated 90% confidence intervals for morphine-6-glucuronide concentration reflected the results of the morphine concentration. However, in this analysis, the ratio between Egalet® Morphine Formulations A and B1 for all endpoints except $AUC_{0-48h}$ were statistically significantly different from 100.

**Table 13** Secondary Analysis of Morphine (Bioequivalence)

| | Means | | Form B1 / Form A | | |
|---|---|---|---|---|---|
| | Form. B1 (1x60 mg) | Form. A (2x30mg) | Ratio | 90 % CI | p-value |
| Full PK data set: | | | | | |
| AUC(0-48h) (nmol*h/L) | 642.3 | 583.0 | 110.2 | (102.7, 118.2) | 0.0235 |
| AUC(0-inf) (nmol*h/L) | 755.2 | 676.8 | 111.6 | (100.9, 123.5) | 0.0749 |
| Cmax (nmol/L) | 40.5 | 33.3 | 121.7 | (113.0, 131.2) | <.0001 |
| Completers only: | | | | | |
| AUC(0-48h) (nmol*h/L) | 654.7 | 591.3 | 110.7 | (103.0, 119.1) | 0.0218 |
| AUC(0-inf) (nmol*h/L) | 772.2 | 693.6 | 111.3 | (100.2, 123.7) | 0.0945 |
| Cmax (nmol/L) | 41.1 | 33.4 | 122.9 | (113.8, 132.8) | <.0001 |
| PK set-tail less 20%: | | | | | |
| AUC(0-inf) (nmol*h/L) | 714.4 | 624.9 | 114.3 | (104.6, 125.0) | 0.0141 |

Endpoints are log-transformed before analysis, and results are transformed back and presented as ratios. The model includes period and treatment as fixed effects and subject as a random effect.

Estimates and comparisons are based on the full model with all treatments included.

The mean is the geometric mean estimated from the model.

**Exploratory Secondary Analysis of Bioequivalence of 1x30 mg Formulation B1 versus 1x30 mg Formulation A**

The results in Table 14 showed that for all endpoints based on morphine plasma concentrations, the 90% confidence for the estimated ratio of means lay within the boundaries of 0.80 to 1.25 and none of the ratios were statistically significantly different from 100. Hence, bioequivalence could be assumed to have been demonstrated.

**Table 14** Exploratory Secondary Analysis of Morphine (Bioequivalence) - 1x30 mg Formulation B1 versus 1x30 mg Formulation A

| | Means | | Form B1 / Form A | | |
|---|---|---|---|---|---|
| | Form. B1 (1x30 mg) | Form. A (1x30mg) | Ratio | 90 % CI | p-value |
| <u>Full PK data set:</u> | | | | | |
| AUC(0-48h) (nmol*h/L) | 277.8 | 291.5 | 95.3 | (88.9, 102.2) | 0.2551 |
| AUC(0-inf) (nmol*h/L) | 326.5 | 338.4 | 96.5 | (87.3, 106.7) | 0.5569 |
| Cmax (nmol/L) | 18.0 | 16.6 | 108.2 | (100.5, 116.6) | 0.0811 |
| <u>Completers only:</u> | | | | | |
| AUC(0-48h) (nmol*h/L) | 282.2 | 295.7 | 95.5 | (88.8, 102.6) | 0.2899 |
| AUC(0-inf) (nmol*h/L) | 332.6 | 346.8 | 95.9 | (86.3, 106.6) | 0.5114 |
| Cmax (nmol/L) | 18.3 | 16.7 | 109.4 | (101.3, 118.2) | 0.0547 |
| <u>PK set-tail less 20%:</u> | | | | | |
| AUC(0-inf) (nmol*h/L) | 296.8 | 312.4 | 95.0 | (86.5, 104.2) | 0.3604 |

Formulation A (1*30mg) is derived by dividing AUC and Cmax by 2 - since two tablets were administered.

Endpoints are log-transformed before analysis, and results are transformed back and presented as ratios. The model includes period and treatment as fixed effects and subject as a random effect.

Estimates and comparisons are based on the full model with all treatments included. The mean is the geometric mean estimated from the model.

Yet another explorative analysis was comparing the 24 hour plasma concentrations of morphine from formulation B1 to formulation A. The ratio between 60 mg Egalet® Morphine Formulation B1 and 2x30 mg Egalet® Morphine Formulation A at hour 24 was 116.0% (Cl: 98.5% - 136.7%), p= 0.1351.

**Safety Results**

A total of 105 treatment emergent adverse experiences (TEAEs) were reported by 17 of the 24 subjects who received at least one dose of the study medication (safety population). No adverse events were severe, significant, or serious.

No safety issues were observed with respect to clinical laboratory results and vital signs results.

No relevant differences were observed among the treatment groups with respect to mean values and changes from baseline for vital signs and clinical laboratory results.

**Discussion**

The PK profiles of single doses of four different strengths of Egalet® Morphine Formulation B1 have been

evaluated in 35 subjects in this 5-period cross over study to assess whether dose-proportionality of Egalet® Morphine Formulation B1 could be demonstrated. PK profiles of a single dose of 1x 60 mg Egalet®Morphine Formulation B1 and 2 x 30 mg Egalet®Morphine Formulation A have been evaluated to assess bioequivalence between Egalet® Morphine Formulations B1 and A. In addition PK profiles of a single dose of 1 x 30 mg Egalet®Morphine Formulation B1 and 1 x 30 mg Egalet® Morphine Formulation A (in the form of dividing PK parameter of 2x30 mg with 2) have been evaluated.

As the 90% confidence intervals for the regression coefficient of the log-dose for $AUC_{0-48h}$ and $C_{max}$ were contained within the interval 0.8-1.25 for morphine, dose-linearity has been demonstrated. Since the estimated coefficient of the log-dose for $AUC_{0-48h}$ as well as $C_{max}$ were larger than 1 and the lower limit of the 90% confidence interval was larger than 1, there was some statistical evidence of "over-proportionality" relative to dose. Combining these two observations, some deviation from dose proportionality was present, but in the light of the protocol defined limits and the fact that this "over-proportionality" was shown across the doses, therefore in fact providing proportionality (just not with the theoretically expected slope of 1), this deviation was concluded not clinically relevant. Evaluating the slight deviation from proportionality between the dose levels, table 15 gives the ratios between geometric means after adjusting for dose. It was observed that the main part of the deviation was caused by the 30 mg tablet having a lower bioavailability than the other three doses.

**Table 15**

| Ratio of Geometric Means | 60mg/30mg | 100mg/60mg | 200mg/100mg |
|---|---|---|---|
| $AUC_{0-48}$ | 1,16 | 1,03 | 1,04 |
| $AUC_{0-inf}$ | 1,15 | 1,00 | 1,01 |
| $C_{max}$ | 1,14 | 1,01 | 1,16 |

The Egalet® morphine 30 mg formulation differed in some ways from the other strengths (this was later adjusted) and the $C_{max}$ ratio of this was slightly higher than the 125 guidance limit obtained in the bioequivalence range. Therefore, a second analysis using the 60 mg strength was generated

Evaluating morphine plasma concentration the 90% confidence intervals for the ratio of means between 1 x 60 mg Egalet® Morphine Formulation B1 and 2 x 30 mg Egalet® Morphine Formulation A for $AUC_{0-48h}$ and $AUC_{0-inf}$ were contained within the interval 80-125. However, as the upper limit of the 90% confidence intervals for the ratio of means for $C_{max}$ exceeded 125 bioequivalence was not demonstrated.

As the dose-linearity analysis showed some evidence of over-proportionality and the analysis of bioequivalence compared 1 x 60 mg Egalet® Morphine Formulation B1 and 2 x 30 mg Egalet® Morphine Formulation A, an analysis comparing AUC and $C_{max}$ of 1 x 30 mg Egalet® Morphine Formulation B1 to half AUC and half $C_{max}$ of 2 x 30 mg Egalet® Morphine Formulation A was performed. All 90% confidence intervals for this analysis were contained within the interval 0.80-1.25 for morphine. This means that when assuming the two Egalet® Morphine 30 mg Formulation A tablets result in a doubling of the PK response, then bioequivalence has been demonstrated between Egalet® Morphine Formulations A and B1.

The minor peak in PK profiles at 24 hours could be an influence of naltrexone as seen in earlier studies and/or as a result of hepatic recirculation.

A total of 105 TEAEs were reported by 83% (n=29) of the 35 subjects who received at least one dose of the study medication (safety population). No trend was observed with respect to overall adverse event frequencies or types of adverse events experienced with respect to dose level or treatment. No other adverse events derived from abnormal clinical laboratory results or vital signs measurements were recorded for more than one subject in any given treatment group. No notable differences were observed with respect to mean values and changes from baseline for clinical laboratory and vital signs measurements.

### Conclusion

The primary objective of evaluating dose-linearity of four different strengths of Egalet® Morphine Formulation B1 resulted in a demonstration of dose-linearity.

No severe, significant, or serious adverse events were reported during the study. The frequency of adverse event observations was not related to dose level or treatment. The most frequently occurring adverse events were expected or procedure-related and were mild or moderate in intensity. No safety issues were observed with respect to the clinical laboratory tests and vital signs. The evaluation of safety and tolerability of Egalet® Morphine showed no notable differences between 1 x 60 mg of Formulation B1 (Treatment B) and 2 x 30 mg of Formulation A (Treatment E), with respect to the safety parameters collected (adverse events and vital signs).

## Claims

1. A pharmaceutical composition comprising

a) a matrix composition comprising

i) an active drug substance; and
ii) at least one polyglycol

said matrix composition having a cylindrical shape optionally with tapered end(s), said matrix being surrounded by
b) a coating having at least one opening exposing one surface of said matrix, said coating being substantially impermeable to an aqueous medium;

for continuous treatment of pain, wherein the medicament is prepared for administration once daily for at least 3 days, such as at least 7 days, for example at least 14 days.

2. The composition according to any one of the preceding claims, wherein the daily dosage is in the range of 15 to 500 mg of said active drug substance.

3. The composition according to anyone of the preceding claims, wherein the 2$^{nd}$ point where a concentration of 50% of steady state Cmax is reached is in the range of 4 to 13.5 hours) after last administration to a steady state individual.

4. The composition according to anyone of the preceding claims, wherein the 1$^{st}$ point where a concentration of 50% of steady state Cmax(1 $^{st}$ time) is reached is no earlier than 0.25 hour, for example in the range of 0.25 to 2.5 hours, such as for example 0.25 to 3 hours after last administration to a steady state individual.

5. The composition according to anyone of the preceding claims, wherein Tmax is in the range of 3 to 4 hours after last administration to a steady state individual.

6. The composition according to anyone of the preceding claims, wherein Cmin is reached no earlier than 12 hours after last administration to a steady state individual.

7. The composition according to anyone of claims 1 to 6, wherein the drug substance is an opioid, such as for example morphine or a pharmaceutically acceptable salt thereof.

8. The composition according to anyone of the preceding claims, wherein at least one polyglycol is a copolymer.

9. The composition according to anyone of the preceding claims, wherein the total concentration of polyglycols in the matrix composition is from 5 % to 99% w/w such as from 15 % to 95% w/w, for example from 30 % to 90% w/w, such as from 30 % to 85% w/w, for example from 30 % to 80% w/w, such as from 40 % to 80% w/w, for example from 45 % to 75% w/w, such as from 40 % to 50% w/w, for example from 45 % to 50% w/w, such as from 60 % to 85% w/w, for example from 70 % to 80% w/w, for example from 70 % to 75% w/w.

10. The composition according to anyone of the preceding claims, wherein at least one polyglycol is a polyethylene glycol and/or a polyethylene oxide.

11. The composition according to claim 10, wherein the polyethylene glycol and/or polyethylene oxide has a molecular weight of in the range of 20,000 to 700,000 daltons, such as in the range of 20,000 to 600,000 daltons, for example in the range of 35,000 to 500,000 daltons, such as in the range of 35,000 to 400,000 daltons, for example in the range off 35,000 to 300,000 daltons, such as in the range of 50,000 to 300,000 daltons, for example about 200,000 daltons, such as about 300,000 daltons.

12. The composition according to anyone of the preceding claims 8 to 11, wherein the copolymer is a poloxamer that has an average molecular weight in the range of 2,000 to 30,000 dalton, such as in the range of 2,000 daltons to 20,000 daltons, for example in the range of 4,000 daltons to 18,000 daltons, such as in the range of 6,000 daltons to 10,000 daltons.

13. The composition according to any of the claims 1 to 6, wherein the composition comprises morphine sulphate as

the active drug, a mixture of polyethylene oxide 200,000 and polyethylene oxide 300,000 as polyglycol, poloxamer as plasticizer, mannitol as stabilizer, a mixture of carrageenan and hydroxypropylmethylcellulose as gelling agent, butylated hydroxytoluene as antioxidant and a mixture of polactic acid and polyethyleneoxide as the coating.

**14.** The composition according to anyone of the preceding claims, wherein the composition is designed for oral administration, such as for example in the form of tablets.

**15.** The composition according to anyone of the preceding claims, wherein said pain is chronic pain.

**Figure 1**

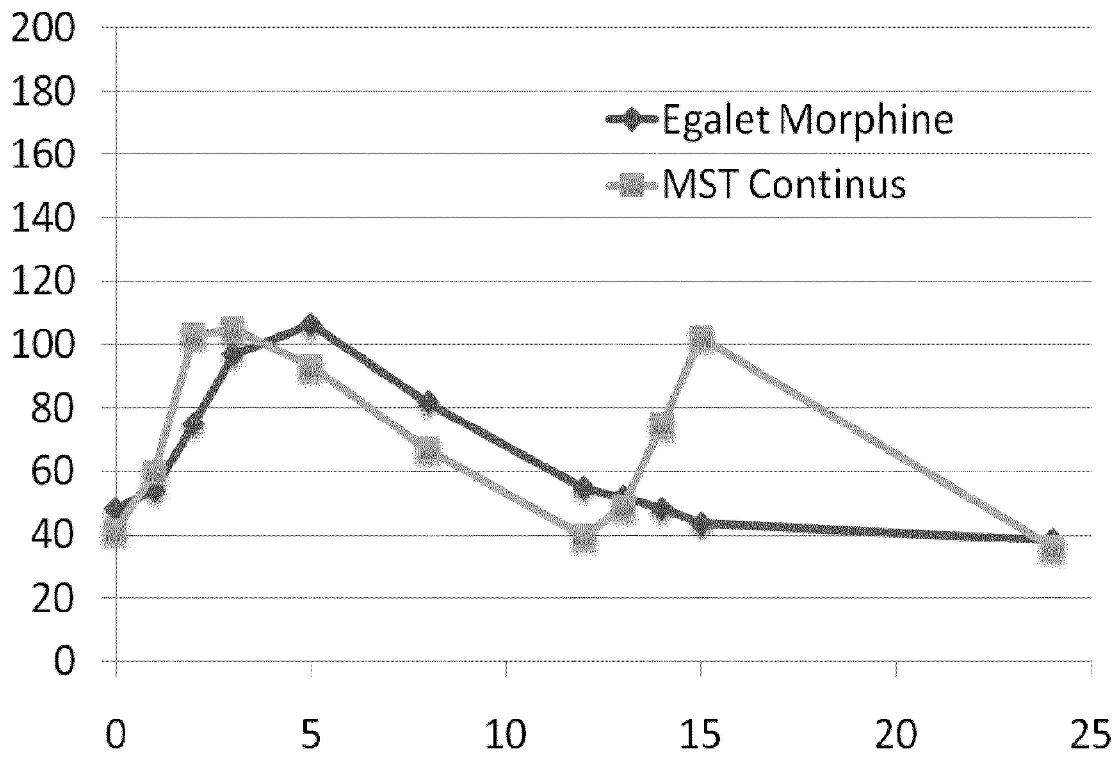
200
180
160
140
120
100
80
60
40
20
0
0
5
10
15
20
25
Egalet Morphine
MST Continus

**Figure 2**

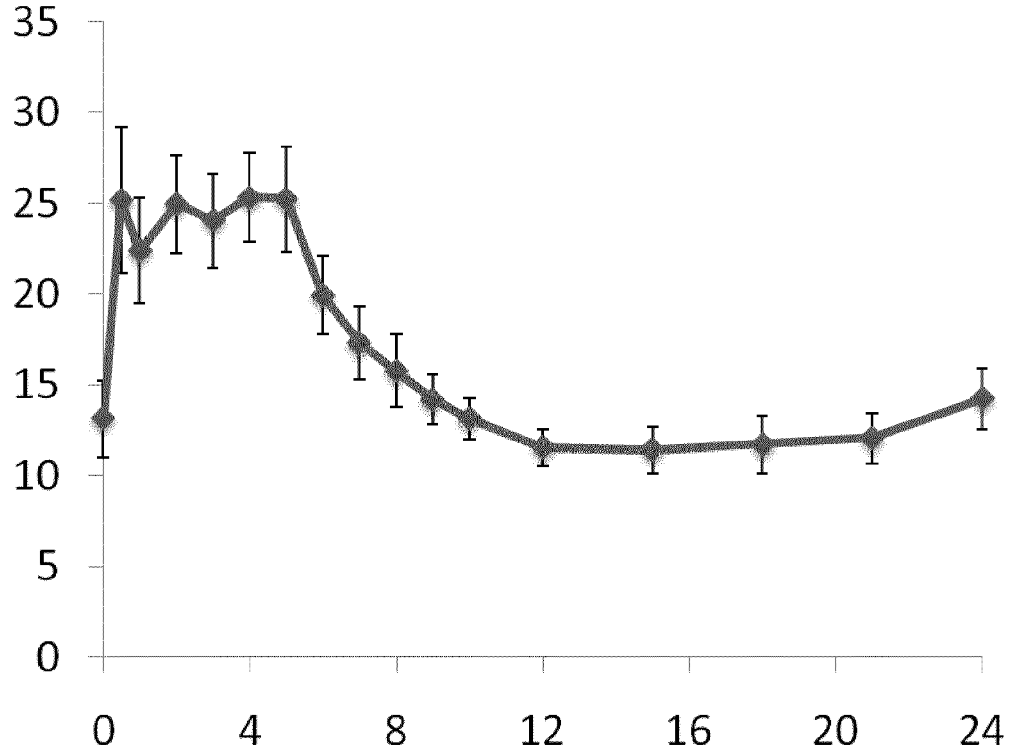

Figure 3

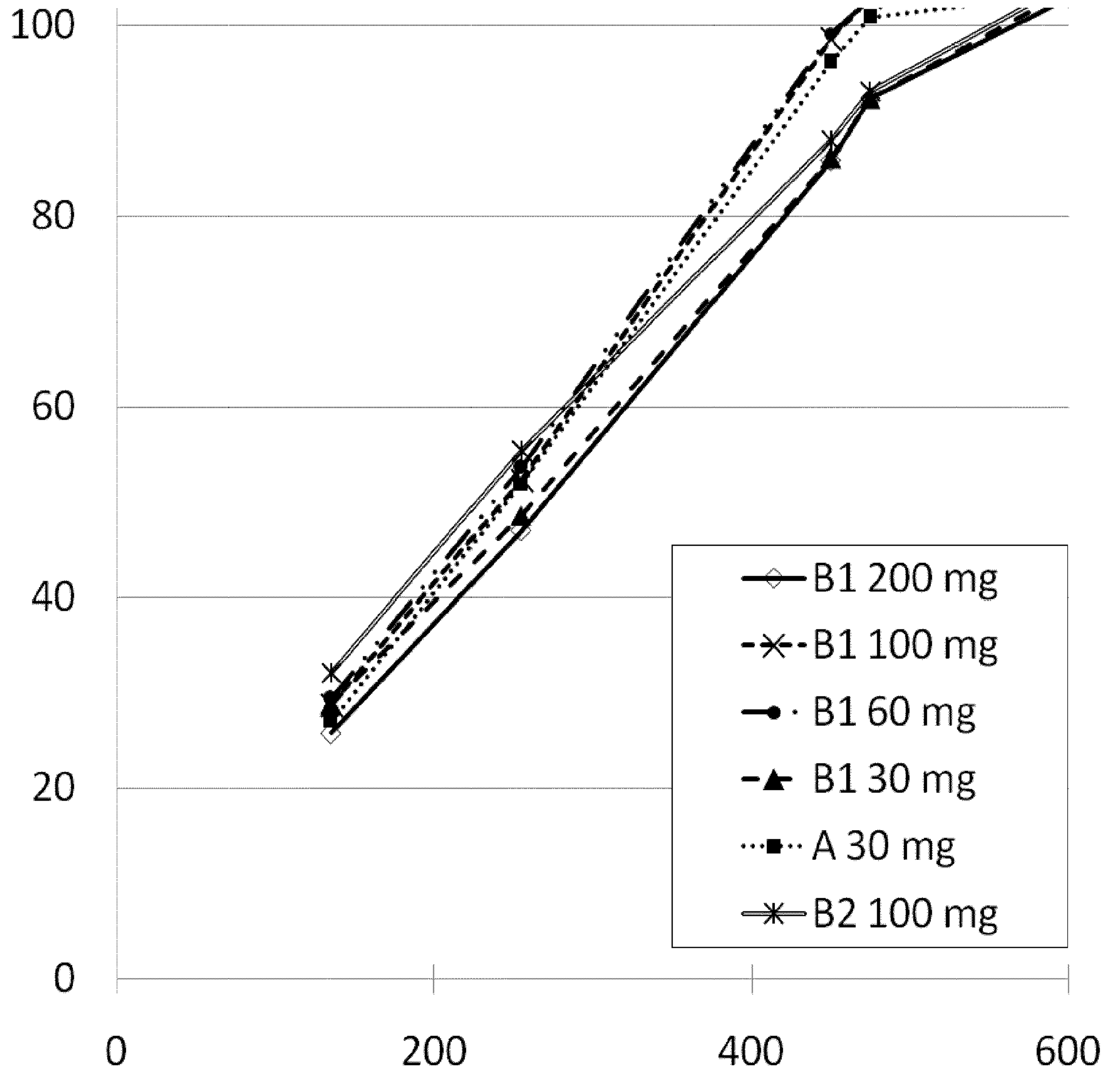
100
80
60
40
20
0
0
200
400
600
B1 200 mg
B1 100 mg
B1 60 mg
B1 30 mg
A 30 mg
B2 100 mg

Figure 4

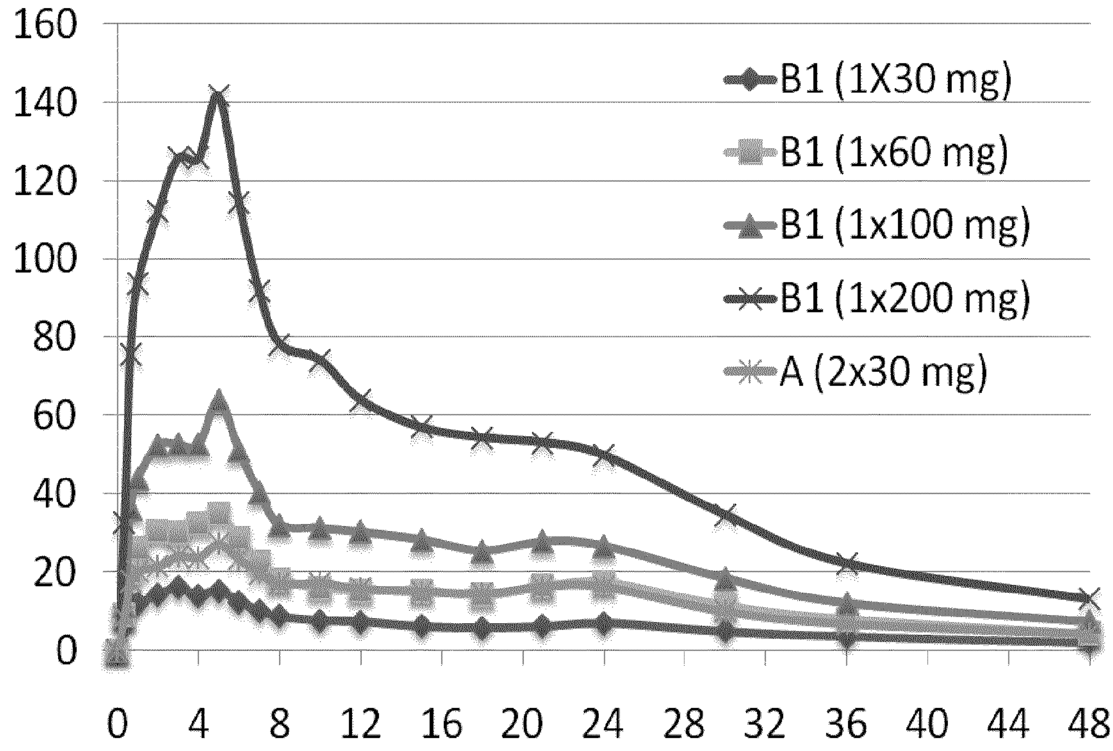
160
140
120
100
80
60
40
20
0
0 4 8 12 16 20 24 28 32 36 40 44 48
B1 (1X30 mg)
B1 (1x60 mg)
B1 (1x100 mg)
B1 (1x200 mg)
A (2x30 mg)

Europäisches Patentamt
European Patent Office
Office européen des brevets

# EUROPEAN SEARCH REPORT

Application Number
EP 13 19 1878

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/024430 A (EGALET AS [DK]; FISCHER GINA [DK]; BAR-SHALOM DANIEL [DK]; SLOT LILLIA) 27 March 2003 (2003-03-27)<br>* page 27, line 14 - page 28, line 2 *<br>* claims *<br>----- | 1-15 | INV.<br>A61K9/22<br>A61K9/36<br>A61K9/20<br>A61K9/28<br>A61K31/485 |
| X,D | WO 2004/084868 A1 (EGALET AS [DK]; FISCHER GINA [DK]; BAR-SHALOM DANIEL [DK]; SLOT LILLIA) 7 October 2004 (2004-10-07)<br>* page 3, line 18 - line 37 *<br>* page 4, line 11 - line 16 *<br>* page 14, line 5 - line 21 *<br>* page 41, line 15 - line 24 *<br>* examples *<br>* claims *<br>* figures *<br>----- | 1-15 | |
| | | | TECHNICAL FIELDS SEARCHED (IPC) |
| | | | A61K |

The present search report has been drawn up for all claims

1

| Place of search | Date of completion of the search | Examiner |
|---|---|---|
| Munich | 20 January 2014 | S. von Eggelkraut-G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 19 1878

This annex lists the patent family members relating to the patent documents cited in the above-mentioned European search report.
The members are as contained in the European Patent Office EDP file on
The European Patent Office is in no way liable for these particulars which are merely given for the purpose of information.

20-01-2014

| Patent document cited in search report | Publication date | Patent family member(s) | Publication date |
|---|---|---|---|
| WO 03024430 A | 27-03-2003 | EP 1429744 A1 | 23-06-2004 |
| | | US 2004253310 A1 | 16-12-2004 |
| | | US 2008254123 A1 | 16-10-2008 |
| | | WO 03024430 A1 | 27-03-2003 |
| WO 2004084868 A1 | 07-10-2004 | AT 495732 T | 15-02-2011 |
| | | EP 1610767 A1 | 04-01-2006 |
| | | EP 2301526 A2 | 30-03-2011 |
| | | ES 2360102 T3 | 31-05-2011 |
| | | US 2007003617 A1 | 04-01-2007 |
| | | WO 2004084868 A1 | 07-10-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003024430 A
- WO 2004084868 A


### Non-patent literature cited in the description


- **RAEHHAL ; BOHN.** *Mu Opioid Receptor Regulation and Opiate Responsiveness The AAPS Journal,* 2005, vol. 7 (3), 60
- **CAMU ; VANLERSBERGHE.** Pharmacology of systemic analgesics. *Best Pract Res Clin Anaesthesiol,* 2002, vol. 16 (4), 475-88
- **DAHLSTRÖM et al.** Patient-controlled analgesic therapy, part IV: Pharmacokinetics and analgesic plasma concentrations of morphine. *Clin Pharmacokinet;,* 1982, vol. 7, 266-79
- **GRAVES et al.** Relationship between plasma morphine concentrations and pharmacologic effects in postoperative patients using patient-controlled analgesia. *Clin Pharm,* 1985, vol. 4, 41-7